# EUROPÄISCHE PATENTSCHRIFT

(11) **EP 2 004 670 B1**
(45) Veröffentlichungstag und Bekanntmachung des Hinweises auf die Patenterteilung: **27.01.2010**
(21) Anmeldenummer: 07724251.9
(22) Anmeldetag: 13.04.2007
(51) Int. Cl.: C07K 1/02, C07K 5/06, C07K 5/08, C07K 7/64

(54) **VERFAHREN ZUM HERSTELLEN VON LYSOBACTIN-DERIVATEN**
PROCESS FOR THE MANUFACTURE OF LYSOBACTIN DERIVATIVES
PROCÉDÉ DE PRODUCTION DE DÉRIVÉS DE LYSOBACTINE

(30) Priorität: 13.04.2006 DE 102006018250
(43) Veröffentlichungstag der Anmeldung: 24.12.2008
(73) Patentinhaber: AiCuris GmbH & Co. KG, 42117 Wuppertal (DE)
(72) Erfinder: NUSSBAUM, Franz, 40219 Düsseldorf (DE); ANLAUF, Sonja, 42115 Wuppertal (DE); KOEBBERLING, Johannes, 41516 Grevenbroich (DE); TELSER, Joachim, 42115 Wuppertal (DE); HAEBICH, Dieter, 45115 Wuppertal (DE)
(74) Vertreter: Scheuermann, Erik
(86) Internationale Anmeldenummer: PCT/EP2007/003313
(87) Internationale Veröffentlichungsnummer: WO 2007/118693

(56) Entgegenhaltungen:
- DE-A1-102004 053 410
- EGNER B J ET AL: "Monitoring the solid phase synthesis of analogues of lysobactin and the katanosins using in situ MALDI-TOF MS" TETRAHEDRON, ELSEVIER SCIENCE PUBLISHERS, AMSTERDAM, NL, Bd. 53, Nr. 41, 13. Oktober 1997 (1997-10-13), Seiten 14021-14030, XP004618631 ISSN: 0040-4020

## Beschreibung

Die vorliegende Erfindung betrifft ein Verfahren zum Herstellen von cyclischen Depsipeptiden der folgenden Formel (I) 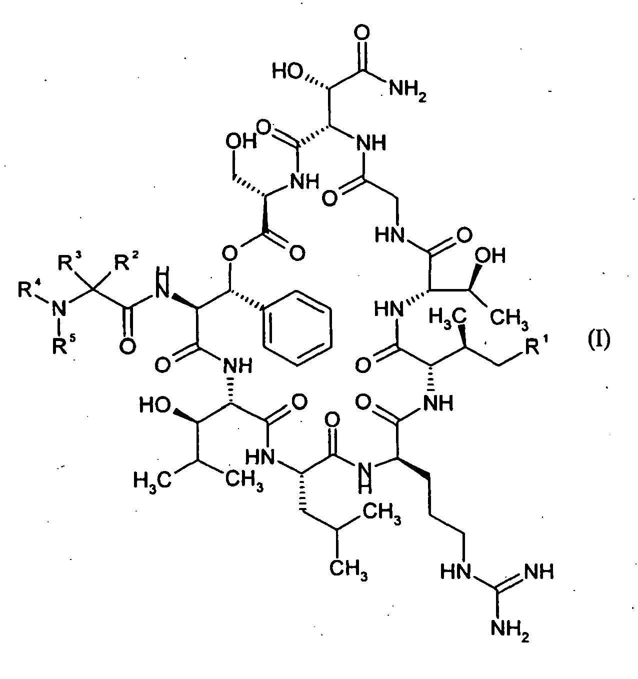
in der R¹ = H oder CH₃ ist,
in der R² = Wasserstoff, C₃-C₆-Cycloalkyl, C₅-C₆-Cycloalkenyl, C₃-C₆ Cycloalkylmethyl, 5- bis 7-gliedriges Heterocyclylmethyl, Methyl, Ethyl, n-Propyl, iso-Propyl, 1-Methylprop-1-yl, 2-Methylprop-1-yl, 2,2-Dimethylprop-1-yl, 1,1-Dimethylprop-1-yl, 1-Ethyl-prop-1-yl, 1-Ethyl-1-methylprop-1-yl, n-Butyl, 2-Methylbut-1-yl, 3-Methylbut-1-yl, 1-Ethylbut-1-yl, *tert*-Butyl, 4-Methylpent-1-yl, n-Hexyl, Alkenyl oder Aryl ist,
wobei R² substituiert sein kann mit 0, 1, 2 oder 3 Substituenten unabhängig voneinander ausgewählt aus der Gruppe bestehend aus Halogen, Hydroxy, Amino, Cyano, Trimethylsilyl, Alkyl, Alkoxy, Benzyloxy, C₃-C₆-Cycloalkyl, Aryl, 5- bis 10-gliedriges Heteroaryl, Alkylamino, Arylamino, Alkylcarbonylamino, Arylcarbonylamino, Alkylcarbonyl, Alkoxycarbonyl, Arylcarbonyl und Benzyloxycarbonylamino,
worin Aryl und Heteroaryl ihrerseits substituiert sein können mit 0, 1, 2 oder 3 Substituenten unabhängig voneinander ausgewählt aus der Gruppe bestehend aus Halogen, Hydroxy, Amino, Cyano, Nitro, Alkyl, Alkoxy und Phenyl,
in der R³ = Wasserstoff oder C₁-C₄-Alkyl ist,
oder
in der R² und R³ zusammen mit dem Kohlenstoffatom, an das sie gebunden sind, einen C₃-C₆-Cycloalkyl-Ring oder einen 5- bis 7-gliedrigen Heterocyclyl-Ring bilden, wobei der Cycloalkyl-Ring und der Heterocyclyl-Ring substituiert sein können mit 0, 1, 2 oder 3 Substituenten unabhängig voneinander ausgewählt aus der Gruppe bestehend aus Trifluormethyl, Alkyl, Alkoxy und Alkylcarbonyl,
in der R⁴ = Alkyl, C₃-C₆-Cycloalkyl, 5- bis 7-gliedriges Heterocyclyl, Aryl, 5- oder 6-gliedriges Heteroaryl, Alkylcarbonyl, Alkoxycarbonyl, C₃-C₆-Cycloalkylcarbonyl, 5-bis 7-gliedriges Heterocyclylcarbonyl, Arylcarbonyl, 5- oder 6-gliedriges Heteroarylcarbonyl oder Alkylaminocarbonyl ist,
wobei Alkyl, Cycloalkyl, Heterocyclyl, Aryl, Heteroaryl, Alkoxycarbonyl, Cycloalkylcarbonyl, Heterocyclylcarbonyl, Arylcarbonyl, Heteroarylcarbonyl und Alkylaminocarbonyl substituiert sein können mit 0, 1, 2 oder 3 Substituenten unabhängig voneinander ausgewählt aus der Gruppe bestehend aus Halogen, Hydroxy, Amino, Alkylamino und Phenyl,
und
wobei Alkylcarbonyl substituiert ist mit einem Substituenten Amino oder Alkylamino,
und
wobei Alkylcarbonyl substituiert sein kann mit weiteren 0, 1 oder 2 Substituenten unabhängig voneinander ausgewählt aus der Gruppe bestehend aus Halogen, Hydroxy, Trimethylsilyl, Alkoxy, Alkylthio, Benzyloxy, C₃-C₆-Cycloalkyl, Phenyl, Naphthyl, 5- bis 10-gliedriges Heteroaryl, Alkylcarbonylamino, Alkoxycarbonylamino, Arylcarbonylamino, Arylcarbonyloxy, Benzyloxycarbonyl und Benzyloxycarbonylamino,
wobei Phenyl und Heteroaryl ihrerseits substituiert sein können mit 0, 1, 2 oder 3 Substituenten unabhängig voneinander ausgewählt aus der Gruppe bestehend aus Halogen, Hydroxy, Nitro, Alkyl, Alkoxy und Phenyl,
oder zwei Substituenten an demselben Kohlenstoffatom im Alkylcarbonyl zusammen mit dem Kohlenstoffatom, an das sie gebunden sind, einen C₃-C₆-Cycloalkyl-Ring oder einen 5- bis 7-gliedrigen Heterocyclyl-Ring bilden,
wobei der Cycloalkyl-Ring und der Heterocyclyl-Ring substituiert sein können mit 0, 1, 2 oder 3 Substituenten unabhängig voneinander ausgewählt aus der Gruppe bestehend aus Trifluormethyl, Alkyl und Alkoxy,
oder
wobei der Cycloalkyl-Ring Benzo-anneliert sein kann,
in der R⁵ = Wasserstoff, C₁-C₄-Alkyl, Cyclopropyl oder Cyclopropylmethyl ist,
oder
in der R⁴ und R⁵ zusammen mit dem Stickstoffatom, an das sie gebunden sind, einen 5- bis 7-gliedrigen Heterocyclyl-Ring bilden, wobei der Heterocyclyl-Ring substituiert sein kann mit 0, 1, 2 oder 3 Substituenten unabhängig voneinander ausgewählt aus der Gruppe bestehend aus Halogen, Hydroxy, Amino, Cyano, Alkyl, Alkoxy und Alkylamino,
sowie Verbindungen, die in diesem Verfahren nützlich und in den Ansprüchen beschrieben sind.

Die oben dargestellten cyclischen Depsipeptide umfassen unter Anderem die beiden nachstehend dargestellten Naturstoffe, die als Lysobactin und Katanosin A bezeichnet werden. Diese Substanzen sind Inhibitoren der Zellwandbiosynthese und somit antibakteriell wirksam.

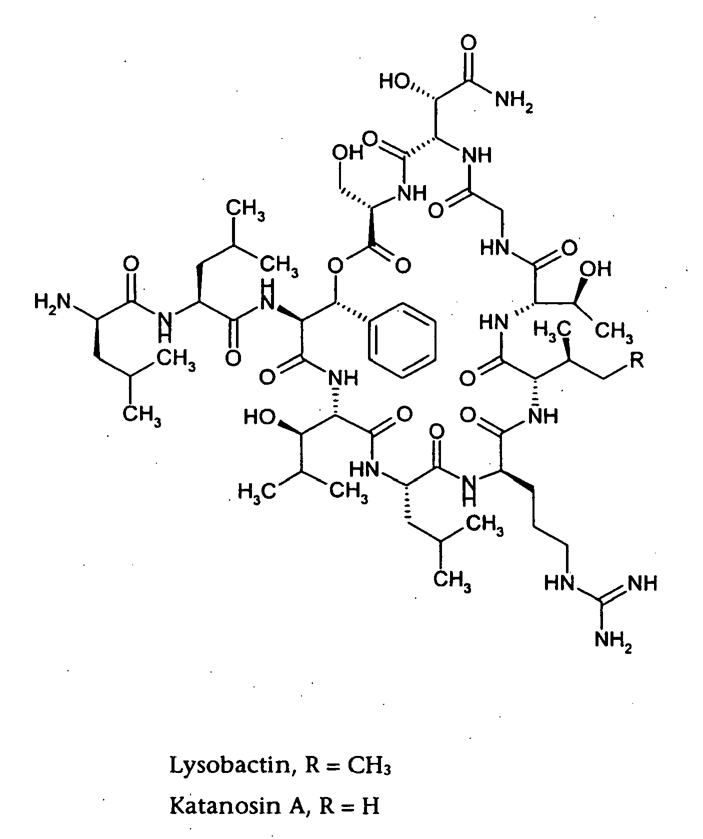

Die bakterielle Zellwand wird durch eine Reihe von Enzymen synthetisiert (Zellwandbiosynthese) und ist essentiell für das Überleben bzw. die Vermehrung von Mikroorganismen. Die Struktur dieses Makromoleküls ebenso wie die an ihrer Synthese beteiligten Proteine und Biosyntheseintermediate ("Precursor") sind innerhalb der Bakterien stark konserviert. Aufgrund ihrer essentiellen Natur und Einheitlichkeit ist die Zellwandbiosynthese ein idealer Angriffspunkt für neue Antibiotika.

Vancomycin und Penicillin sind Inhibitoren der bakteriellen Zellwandbiosynthese und stellen erfolgreiche Beispiele für die antibiotische Potenz dieses Wirkprinzips dar. Sie werden seit mehreren Jahrzehnten in der Klinik zur Behandlung von bakteriellen Infektionen vor allem mit Gram-positiven Erregern eingesetzt. Durch das wachsende Auftreten von resistenten Keimen, zum Beispiel Methicillin-resistenten Staphylokokken, Penicillin-resistenten Pneumokokken und Vancomycin-resistenten Enterokokken sowie jüngst auch erstmals Vancomycin-resistenten Staphylokokken, verlieren diese Substanzen zunehmend ihre therapeutische Wirksamkeit.

Lysobactin wurde bisher durch Fermentation unter Verwendung zum Beispiel von Lysobacter sp. SC 14067 gewonnen. Es ist ferner aus der WO 2004/099239 A1 bekannt, die beiden Leucin-Einheiten, die das lineare Segment bilden, zu entfernen und durch andere Gruppen zu ersetzen. Ein Weg zur vollsynthetischen Herstellung von Lysobactin, Katanosin A oder Derivaten davon, die im linearen Segment Variationen tragen, ist bisher noch nicht bekannt.

Egner und Bradley, Tetrahedron, 1997, 53(41), 14021, stellen eine Festphasensynthese von Lysobactin-Analogen vor.

Es ist somit Aufgabe der Erfindung, ein Verfahren zum Herstellen von cyclischen Depsipeptiden der oben genannten Formel (I) zu beschreiben.

Diese Aufgabe wird durch ein Verfahren zum Herstellen von cyclischen Depsipeptiden der Formel (I) durch intramolekulare Cyclisierung einer Verbindung der folgenden Formel (II) 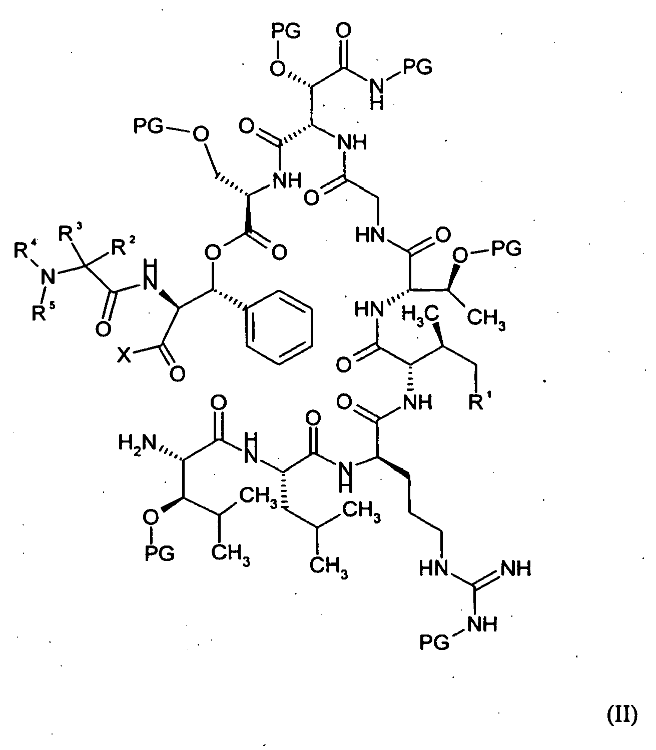
in der R¹ bis R⁵ wie zuvor definiert sind,
in der X = OH, ein Aktivester, ein Pseudohalogen (z.B. ein Azid) oder ein Halogen ist, und
in der PG = H oder eine geeignete Schutzgruppe ist,
und anschließendes Entschützen des cyclischen Zwischenprodukts unter Bildung des cyclischen Depsipeptids der Formel (I) gelöst.

Dieses Verfahren ist **dadurch gekennzeichnet, dass** die an *O*³-veresterte β-Hydroxy-αaminosäure (hier β-Phenylserin = β-Hydroxy-phenylalanin) im Cyclisierungsschritt chemisch aktiviert wird und dann als Acyldonor auftritt. Die Cyclisierung findet über eine Amidverknüpfung (Lactambildung) und nicht über eine Veresterungsreaktion (Lactonbildung) statt.

Das Verfahren ist ferner **dadurch gekennzeichnet, dass** das cyclische Segment der depsipeptidischen Lasso-Struktur durch eine Cyclisierung an der Brückenkopfaminosäure (hier β-Hydroxy-phenylalanin) dargestellt wird.

Im Rahmen der vorliegenden Erfindung haben Substituenten, soweit nicht anders spezifiziert, die folgende Bedeutung:

Alkyl per se und "Alk" und "Alkyl" in Alkoxy. Alkylamino, Alkylcarbonyl, Alkoxycarbonyl, Alkylaminocarbonyl und Alkylcarbonylamino steht für einen linearen oder verzweigten Alkylrest mit in der Regel 1 bis 6, vorzugsweise 1 bis 4, besonders bevorzugt 1 bis 3 Kohlenstoffatomen, beispielhaft und vorzugsweise für Methyl, Ethyl, n-Propyl, Isopropyl, *tert*-Butyl, n-Pentyl und n-Hexyl.

Alkoxy steht beispielhaft und vorzugsweise für Methoxy, Ethoxy, n-Propoxy, Isopropoxy, *tert*-Butoxy, n-Pentoxy und n-Hexoxy.

Alkenyl steht für einen geradkettigen oder verzweigten Alkenylrest mit 2 bis 6 Kohlenstoffatomen. Bevorzugt ist ein geradkettiger oder verzweigter Alkenylrest mit 2 bis 4, besonders bevorzugt mit 2 bis 3 Kohlenstoffatomen. Beispielsweise und vorzugsweise seien genannt: Vinyl, Allyl, n-Prop-1-en-1-yl, n-But-2-en-1-yl, 2-Methyl-prop-1-en-1-yl und 2-Methylprop-2-en-1-yl.

Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, Isopropylamino, *tert*-Butylamino, n-Pentylamino, n-Hexylamino, *N,N-*Dimethylamino, *N,N*-Diethylamino, *N*-Ethyl-*N*-methylamino, *N-*Methyl-*N*-n-propylamino, *N*-Isopropyl-*N*-n-propylamino, *N-tert*-Butyl-*N*-methylamino, *N*-Ethyl-*N*-n-pentylamino und *N*-n-Hexyl-*N*-methylamino.

Arylamino steht für einen Arylaminorest mit einem Arylsubstituenten und gegebenenfalls einem weiteren Substituenten, wie z.B. Aryl oder Alkyl, beispielhaft und vorzugsweise für Phenylamino, Naphthylamino, Phenylmethylamino oder Diphenylamino.

Alkylcarbonyl steht für einen Alkylcarbonylrest mit einem Alkylsubstituenten, beispielhaft und vorzugsweise für Methylcarbonyl, Ethylcarbonyl, n-Propylcarbonyl, Isopropylcarbonyl, *tert*-Butylcarbonyl, n-Pentylcarbonyl und n-Hexylcarbonyl.

Alkoxycarbonyl steht für einen Alkoxycarbonylrest mit einem Alkoxysubstituenten, beispielhaft und vorzugsweise für Methoxycarbonyl, Ethoxycarbonyl, n-Propoxycarbonyl, Isopropoxycarbonyl, *tert*-Butoxycarbonyl, n-Pentoxycarbonyl und n-Hexoxycarbonyl.

Cycloalkylcarbonyl steht für einen Cycloalkylcarbonylrest mit einem Cycloalkylsubstituenten, beispielhaft und vorzugsweise für Cyclopropylcarbonyl, Cyclobutylcarbonyl, Cyclopentylcarbonyl und Cyclohexylcarbonyl.

Heterocyclylcarbonyl steht für einen Heterocyclylcarbonylrest mit einem Heterocyclylsubstituenten, beispielhaft und vorzugsweise für Tetrahydrofuran-2-ylcarbonyl, Pyrrolidin-2-ylcarbonyl, Pyrrolidin-3-ylcarbonyl, Pyrrolinylcarbonyl, Piperidinylcarbonyl, Morpholinylcarbonyl und Perhydroazepinylcarbonyl.

Arylcarbonyl steht für einen Arylcarbonylrest mit einem Arylsubstituenten, beispielhaft und vorzugsweise für Phenylcarbonyl, Naphthylcarbonyl und Phenanthrenylcarbonyl.

Heteroarylcarbonyl steht für einen Heteroarylcarbonylrest mit einem Heteroarylsubstituenten, beispielhaft und vorzugsweise für Thienylcarbonyl, Furylcarbonyl, Pyrrolylcarbonyl, Thiazolylcarbonyl, Oxazolylcarbonyl, Imidazolylcarbonyl, Pyridylcarbonyl, Pyrimidylcarbonyl, Pyridazinylcarbonyl, Indolylcarbonyl, Indazolyl-carbonyl, Benzofuranylcarbonyl, Benzothiophenylcarbonyl, Chinolinylcarbonyl und Isochinolinylcarbonyl.

Alkylcarbonylamino steht für einen Alkylcarbonylaminorest mit einem Alkylsubstituenten, beispielhaft und vorzugsweise für Methylcarbonylamino, Ethylcarbonylamino, n-Propylcarbonylamino, Isopropylcarbonylamino, *tert*-Butylcarbonylamino, n-Pentylcarbonylamino und n-Hexylcarbonylamino.

Arylcarbonylamino steht für einen Arylcarbonylaminorest mit einem Arylsubstituenten, beispielhaft und vorzugsweise für Phenylcarbonylamino, Naphthylcarbonylamino und Phenanthrenylcarbonylamino.

Alkylaminocarbonyl steht für einen Alkylaminocarbonylrest mit einem oder zwei (unabhängig voneinander gewählten) Alkylsubstituenten, beispielhaft und vorzugsweise für Methylaminocarbonyl, Ethylaminocarbonyl, n-Propylaminocarbonyl, Isopropylaminocarbonyl, *tert*-Butylaminocarbonyl, n-Pentylaminocarbonyl, n-Hexylaminocarbonyl, *N,N*-Dimethylaminocarbonyl, *N,N*-Diethylaminocarbonyl, *N*-Ethyl-*N*-methylaminocarbonyl, *N*-Methyl-*N*-n-propylaminocarbonyl, *N*-Isopropyl-*N*-n-propylaminocarbonyl, *N*-*tert*-Butyl-*N*-methylaminocarbonyl, *N*-Ethyl-*N*-n-pentylaminocarbonyl und *N*-n-Hexyl-*N*-methylaminocarbonyl.

Cycloalkyl steht für eine Cycloalkylgruppe mit in der Regel 3 bis 6 Kohlenstoffatomen, beispielhaft und vorzugsweise für Cyclopropyl, Cyclobutyl, Cyclopentyl und Cyclohexyl.

Cycloalkenyl steht für eine Cycloalkenylgruppe mit in der Regel 5 bis 6 Kohlenstoffatomen und einer oder zwei Doppelbindungen, beispielhaft und vorzugsweise für Cyclopent-1-en-1-yl, Cyclopent-2-en-1-yl, Cyclopent-3-en-1-yl, Cyclohex-1-en-1-yl, Cyclohex-2-en-1-yl und Cyclohex-3-en-1-yl.

Aryl steht für einen mono- bis tricyclischen aromatischen, carbocyclischen Rest mit in der Regel 6 bis 14 Kohlenstoffatomen; beispielhaft und vorzugsweise für Phenyl, Naphthyl und Phenanthrenyl.

Heterocyclyl steht für einen mono- oder polycyclischen, vorzugsweise mono- oder bicyclischen, heterocyclischen Rest mit in der Regel 5 bis 7 Ringatomen und bis zu 3, vorzugsweise bis zu 2 Heteroatomen und/oder Heterogruppen aus der Reihe N, O, S, SO, SO₂. Die Heterocyclyl-Reste können gesättigt oder teilweise ungesättigt sein. Bevorzugt sind 5- bis 7-gliedrige, monocyclische gesättigte Heterocyclylreste mit bis zu zwei Heteroatomen aus der Reihe O, N und S, wie beispielhaft und vorzugsweise Tetrahydrofuran-2-yl, Pyrrolidin-2-yl, Pyrrolidin-3-yl, Pyrrolinyl, Piperidinyl, Morpholinyl und Perhydroazepinyl.

Heteroaryl steht für einen aromatischen, mono- oder bicyclischen Rest mit in der Regel 5 bis 10, vorzugsweise 5 bis 6 Ringatomen und bis zu 5, vorzugsweise bis zu 4 Heteroatomen aus der Reihe S, O und N, beispielhaft und vorzugsweise für Thienyl, Furyl, Pyrrolyl, Thiazolyl, Oxazolyl, Imidazolyl, Pyridyl, Pyrimidyl, Pyridazinyl, Indolyl, Indazolyl, Benzofuranyl, Benzothiophenyl, Chinolinyl und Isochinolinyl.

Carbonylgebundene Aminosäure steht für eine Aminosäure, die über die Carbonylgruppe der Aminosäure-Säurefunktion gebunden ist. Bevorzugt sind dabei α-Aminosäuren in der L- oder in der D-Konfiguration, insbesondere natürlich vorkommende α-Aminosäuren, wie z. B. Glycin, L-Alanin, L-Valin, L-Leucin, L-Isoleucin, L-Prolin, L-Phenylalanin, L-Tryptophan oder natürlich vorkommende, α-Aminosäuren in der unnatürlichen D-Konfiguration, wie z.B. D-Alanin, D-Valin, D-Leucin, D-Isoleucin, D-Prolin, D-Phenylalanin, D-Tryptophan oder unnatürliche Aminosäuren, mit einer an das α-Kohlenstoffatom der Aminosäure gebundenen Seitengruppe, wie z.B. C₃-C₆-Cycloalkylmethyl, C₃-C₆-Cycloalkyl, Ethyl, n-Propyl, 2,2-Dimethylpropyl, *tert*-Butyl, 3-Methylbutyl, n-Hexyl oder Allyl, oder die Seitenkette bildet mit dem α-Kohlenstoffatom der Aminosäure einen Ring, wie z. B. Cyclopropyl (Aminosäure: 1-Amino-1-cyclopropancarbonsäure), Cyclobutyl, Cyclopentyl oder Cyclohexyl, oder β-Aminosäuren (zur Nomenklatur vgl.: D. Seebach, M. Overhand, F. N. M. Kühnle, B. Martinoni, L. Oberer, U. Hommel, H. Widmer, *Helv. Chim. Acta* 1996, *79*, 913-941), wie z. B. β-Alanin, β-Phenylalanin, β-Aib (α-Methylalanin) oder Derivate der 2,3-Diaminopropionsäure (z.B. 2,3-Diamino-3-phenylpropionsäure).

Halogen steht für Fluor, Chlor, Brom und Jod, bevorzugt Fluor und Chlor.

Der Begriff Aktivester umfasst alle dem Fachmann bekannten Aktivester. Beispiele für in der Erfindung bevorzugte Aktivester schließen Folgendes ein, nämlich, Cyanomethylester, *p*-Nitrophenylester, *o*-Nitrophenylester, 2,4-Dinitrophenylester, 2,4,5-Trichlorphenylester, Pentachlorphenylester, Pentafluorphenylester (Pfp), *N*-Hydroxyphtalimid-Ester, N-Hydroxysuccinimid-Ester (O-Su), 1-Hydroxypiperidinester, 5-Chlor-8-hydroxyquinolin-Ester.

Bei der intramolekularen Cyclisierung handelt es sich um die Knüpfung einer Amidbindung, die prinzipiell durch jedes dem Fachmann bekannte Verfahren erreicht werden kann.

Die Cyclisierung erfolgt dabei über den hiernach dargestellten nucleophilen Angriff.

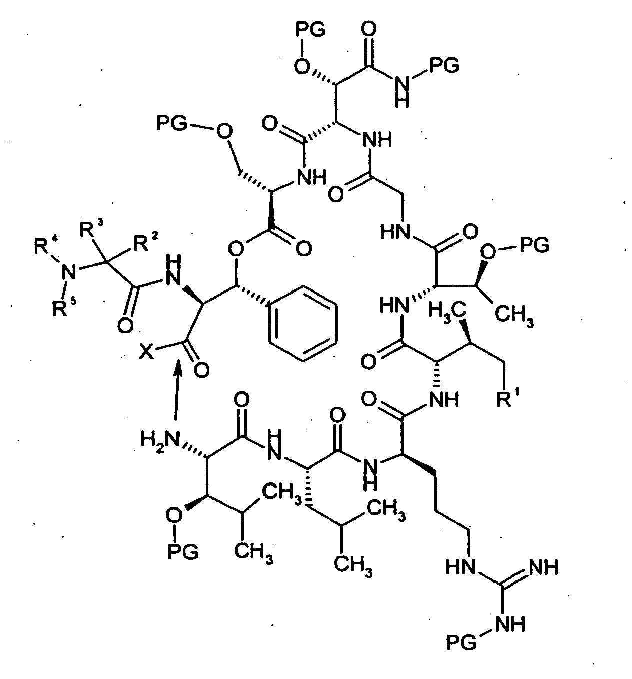

Falls X für einen Aktivester, ein Pseudohalogen oder ein Halogen steht, erfolgt die Umsetzung im Allgemeinen in inerten Lösemitteln ggf. in Gegenwart einer Base bevorzugt in einem Temperaturbereich von -30°C bis 50° bei Normaldruck.

Inerte Lösemittel sind beispielsweise Tetrahydrofuran, Methylenchlorid, Pyridin, Dioxan, Chloroform, Diethylether, *tert*-Butylmethylether, Essigsäureethylester oder Dimethylformamid, wobei Methylenchlorid oder Dimethylformamid bevorzugt sind.

Basen sind beispielsweise Triethylamin, Triisopropylethylamin oder N-Methylmorpholin, bevorzugt ist Triisopropylethylamin.

Falls X für OH steht, erfolgt die Umsetzung im Allgemeinen in inerten Lösungsmitteln in Gegenwart eines Dehydratisierungsreagenzes, ggf. in Gegenwart einer Base, bevorzugt in einem Temperaturbereich von -30°C bis 50°C bei Normaldruck.

Inerte Lösungsmittel sind beispielsweise Halogenwasserstoffe, wie Dichlormethan oder Trichlormethan, Kohlenwasserstoffe wie Benzol, Nitromethan, Dioxan, Dimethylformamid oder Acetonitril. Es ist ferner möglich, ein Gemisch von Lösemitteln einzusetzen. Besonders bevorzugt als Lösemittel sind Dichlormethan und Dimethylformamid.

Als Dehydratisierungsreagenzien eignen sich beispielsweise Carbodiimide, wie zum Beispiel *N,N*'-Diethyl-, *N,N*-Dipropyl-, *N,N*-Diisopropyl-, *N,N*-Dicyclohexylcarbodiimid, *N*-(3-Dimethylaminoisopropyl)-*N'*-ethylcarbodiimid Hydrochlorid (EDC), *N-*Cyclohexylcarbodiimid-*N'*-propyloxymethyl-Polystrol (PS-Carbodiimid) oder Carbonylverbindungen, wie Carbonyldiimidazol oder 1,2-Oxazoliumverbindungen, wie 2-Ethyl-5-phenyl-1,2-oxazolium-3-sulfat oder 2-*tert*-Butyl-5-methyl-isooxazolium-perchlorat oder Acylaminoverbindungen, wie 2-Ethoxy-1-ethoxycarbonyl-1,2-dihydrochinolin oder Propanphosphonsäureanhydrid oder Isobutylchloroformat oder Bis-(2-oxo-3-oxazolidinyl)-phosphorylchlorid oder Benzotriazyloxy-tri(dimethylamino)-phosphoniumhexafluorophosphat oder *O*-(Benzotriazol-1-yl)-,*N,N,N',N'*-tetramethyluronium-hexafluorophosphat (HBTU), 2-(2-Oxo-1-(2-H)-pyridyl)-1,1,3,3-tetramethyluronium-tetrafluoroborat (TPTU) oder *O*-(7-Azabenzotriazol-1-yl)-*N,N,N',N'*-tetramethyluronium-hexafluorophosphat (HATU) oder 1-Hydroxybenzotriazol (HOBt) oder Benzotriazol-1-yl-oxytris(dimethylamino)-phosphoniumhexafluorophosphat (BOP) oder Benzotriazol-1-yloxytris(pyrrolidino)-phosphoniumhexafluorophosphat (PyBOP) oder *N*-Hydroxysuccinimid oder Mischungen aus diesen mit Basen.

Basen sind beispielsweise Alkalicarbonate, wie zum Beispiel Natrium- oder Kaliumcarbonat oder -hydrogencarbonat oder organische Basen, wie Trialkylamine, z.B. Triethylamin, *N*-Methylmorpholin, 4-Methylmorpholin, *N*-Methylpiperidin, 4-Dimethylaminopyridin oder Diisopropylethylamin.

Vorzugsweise wird die Reaktion mit HATU in Gegenwart von 4-Methylmorpholin durchgeführt.

Die Verbindungen der Formel (II) tragen ggf. Schutzgruppen, so dass sich in diesen Fällen der intramolekularen Cyclisierung der Verbindung der Formel (II) eine Abspaltung der Schutzgruppen nach dem Fachmann bekannten Methoden anschließt.

Der Begriff "geeignete Schutzgruppe", wie er hierin verwendet wird, umfasst alle dem Fachmann bekannten Schutzgruppen, die dazu verwendet werden können, eine spezifische Funktion zu maskieren und danach wieder entfernt werden können, ohne weitere Veränderungen im zu entschützenden Molekül auszulösen.

Beispielsweise können primäre oder sekundäre Hydroxygruppen als spaltbare Ether geschützt werden, insbesondere als Methoxymethyl-, Benzyloxymethyl-, *p*-Methoxybenzyloxymethyl-, Benzyl-, *tert*-Butyl-, Tetrahydropyranyl-, Allyl-, *p*-Chlorphenyl-, *p-*Nitrophenyl- oder Triphenylmethylether. Silylether stellen eine weitere Möglichkeit zum Schutz von Hydroxygruppen dar, beispielsweise Trimethylsilyl- (TMS), *tert-*Butyldimethylsilyl (TBDMS), Triisopropylsilyl- (TIPS), *tert*-Butyldiphenylsilyl- (TBDPS) oder Triphenylsilylether. Weiterhin können Hydroxygruppen auch durch Estergruppen geschützt werden, beispielsweise durch Acetyl-, Benzoyl-, Propionyl-, Chloracetyl-, Trichloracetyl-, Trifluoracetyl-, oder Crotylester. Daneben kommen auch Carbonate wie beispielsweise Methylcarbonat, Allylcarbonat, Benzylcarbonat zum Schützen von Alkoholen in Frage. Weiterhin können Ester der Schwefelsäure oder Sulfonsäuren wie beispielsweise Sulfat, Allylsulfonat, *p*-Toluolsulfonat (Tosylat) oder Methylsulfonat als Schutzgruppen für Alkohole verwendet werden.

Bevorzugte Schutzgruppen für Hydroxygruppen sind *tert*-Butylether oder Silylether, insbesondere *tert*-Butyldimethylsilylether.

Für die Guanidinogruppe eignen sich im Prinzip die gleichen Schutzgruppen wie für Hydroxygruppen, bevorzugt ist in diesem Fall die (2,2,5,7,8-Pentamethyl-3,4-dihydro-2*H*-chromen-6-yl)-sulfonylgruppe (PMC-Gruppe).

Carboxylgruppen können in Form ihrer Alkyl-, Silyl-, Arylalkyl- oder Arylester geschützt werden, beispielsweise als Methyl-, Ethyl-, *tert*-Butyl-, Trimethylsilyl-, *tert-*Butyldimethylsilyl-, Benzyl-, Picolyl-, Trichlorethyl-, oder Trimethylsilylester. Carboxylgruppen können auch in Form verschiedener Amide, Anilide oder Hydrazide beispielsweise als *N,N*-Dimethylamid, Pyrrolidinylamid, Piperidinylamid, *o*-Nitro-anilid, *N*-7-Nitroindolylamid oder *N*-Phenylhydrazid geschützt werden. Daneben können sie auch als Orthoester, beispielsweise als Trimethylorthoester geschützt werden. Bevorzugt werden Carbonsäuren in Form ihrer Ester geschützt, insbesondere als Methyl- oder Trimethylsilylethylester.

Zum Schützen von Aminogruppen eignen sich insbesondere Gruppen, die spaltbare Carbamate ergeben, beispielsweise die Methoxycarbonyl-, *tert*-Butoxycarbonyl- (Boc), Benzyloxycarbonyl- (CBz oder Z), Allyloxycarbonyl- (alloc), 9-Fluorenylmethoxycarbonyl- (Fmoc), 2-Trimethylsilylethylcarbonyl-, 1-Adamantylcarbonyl-, *m*-Nitrophenylgruppen. Weiterhin können Aminogruppen auch in Form leicht spaltbarer Amide oder Imide, beispielsweise als Formamid, Chloracetamid, Trichloracetamid, Trifluoracetamid, Benzoylamid, o-Nitrophenylacetamid, Phthalimid, Tetrachlorophthalimid oder Nitrophthalimid geschützt werden. Eine weitere Möglichkeit zum Schutz von Aminogruppen besteht darin, mit bestimmten Alkylgruppen wie beispielsweise der *tert-*Butylgruppe, der Methylgruppe, der Triphenylmethylgruppe der Ferrocenylmethylgruppe oder der Allylgruppe oder mit Arylgruppen wie beispielsweise der 2,4-Dinitrophenylgruppe spaltbare Amine zu bilden.

Bevorzugt werden zum Schützen der Aminogruppe Carbamate, darunter vor allem *tert*-Butoxycarbonyl- (Boc), Benzyloxycarbonyl- (CBz oder Z) oder 9-Fluorenylmethoxycarbonylgruppen (Fmoc) verwendet.

Die hier aufgeführten Schutzgruppen dienen lediglich als Beispiel und stellen keine erschöpfende Aufzählung aller Möglichkeiten dar. Eine weitergehende Behandlung dieses Gebiets findet sich unter anderem in T. W. Greene, P. G. M. Wuts Protective Groups in Organic Synthesis, 3rd Edition, John Wiley & Sons Inc. 1999**.**

Die durch PG dargestellten Gruppen, wie sie hierin verwendet werden, können in einem Molekül die gleichen oder unterschiedliche geeignete Schutzgruppen oder Kombinationen von gleichen oder unterschiedlichen Schutzgruppen mit H oder ausschließlich H sein.

Vorzugsweise ist die Verbindung der Formel (II) eine Verbindung der folgenden Formel (IIa) 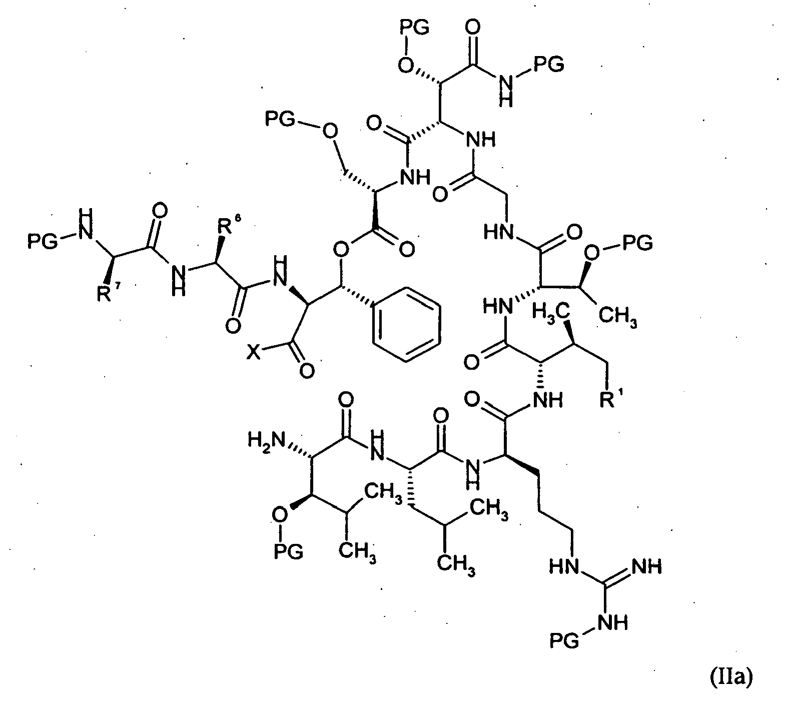
in der X und R¹ wie zuvor definiert sind,
in der R⁶ = Isopropylmethyl, *tert*-Butylmethyl, 2,2-Dimethylbut-1-yl, 2-Ethyl-2-methylbut-1-yl, 2,2-Diethylbut-1-yl, 2,2-Dimethylpent-1-yl, 3-Pyridylmethyl, 4-Trifluormethyl-3-pyridylmethyl, Benzyl oder Trimethylsilylmethyl ist,
in der R⁷ = Isopropylmethyl, *tert*-Butylmethyl, 2,2-Dimethylbut-1-yl, 2-Ethyl-2-methylbut-1-yl, 2,2-Diethylbut-1-yl, 2,2-Dimethylpent-1-yl, Trimethylsilylmethyl oder Benzyl ist, und
in der PG = H oder eine geeignete Schutzgruppe ist.

Vorzugsweise ist R⁶ = Isopropylmethyl, *tert*-Butylmethyl oder 3-Pyridylmethyl und insbesondere ist R⁶ = Isopropylmethyl

Vorzugsweise ist R⁷ = Isopropylmethyl, *tert*-Butylmethyl oder Trimethylsilylmethyl und insbesondere ist R⁷ = Isopropylmethyl

Vorzugsweise ist X = OH.

Vorzugsweise ist R¹ = CH₃.

In einer weiteren Ausführungsform der Erfindung wird die Verbindung (II) durch Kuppeln einer Verbindung der folgenden Formel (III) mit einer Verbindung der folgenden Formel (IV) 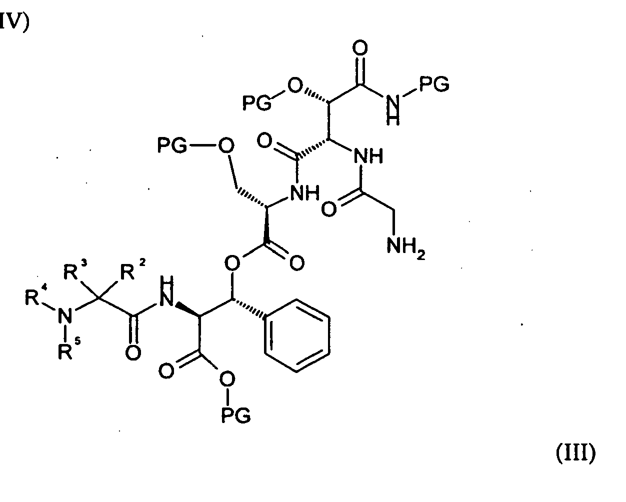 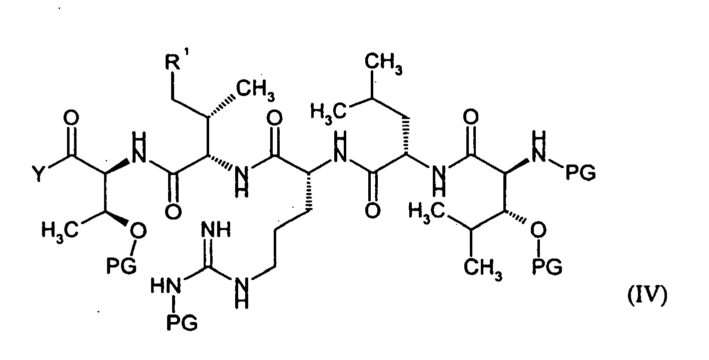
in denen R¹ bis R⁵ wie zuvor definiert sind,
in denen Y = OH, ein Aktivester, ein Pseudohalogen oder ein Halogen ist, und
in denen PG = H oder eine geeignete Schutzgruppe ist,
und ggf. teilweises oder vollständiges Entschützen des Zwischenprodukts sowie ggf. Umwandeln der Carboxylgruppe des 3-Hydroxy-phenylalanins in eine Gruppe der Formel -C(=O)X, in der X wie zuvor definiert ist, hergestellt.

Insbesondere ist die Verbindung der Formel (III) eine Verbindung der folgenden Formel (IIIa). 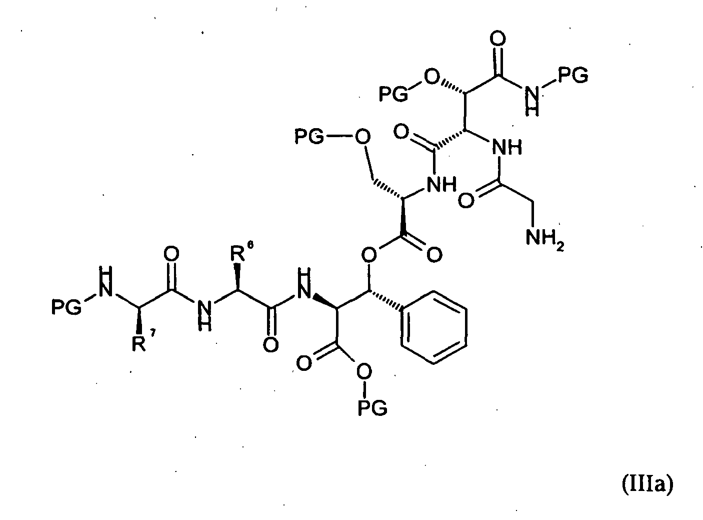
in der R⁶ und R⁷ wie zuvor definiert sind, und
in der PG = H oder eine geeignete Schutzgruppe ist.

Das Kuppeln kann unter den gleichen oder anderen Bedingungen erfolgen, wie zuvor für die intramolekulare Cyclisierung beschrieben. Das Kuppeln erfolgt dabei über den hiernach dargestellten nucleophilen Angriff.

Das Umwandeln der Carboxylgruppe des 3-Hydroxy-phenylalanins in eine Gruppe der Formel -C(=O)X kann durch dem Fachmann bekannte Verfahren erfolgen.

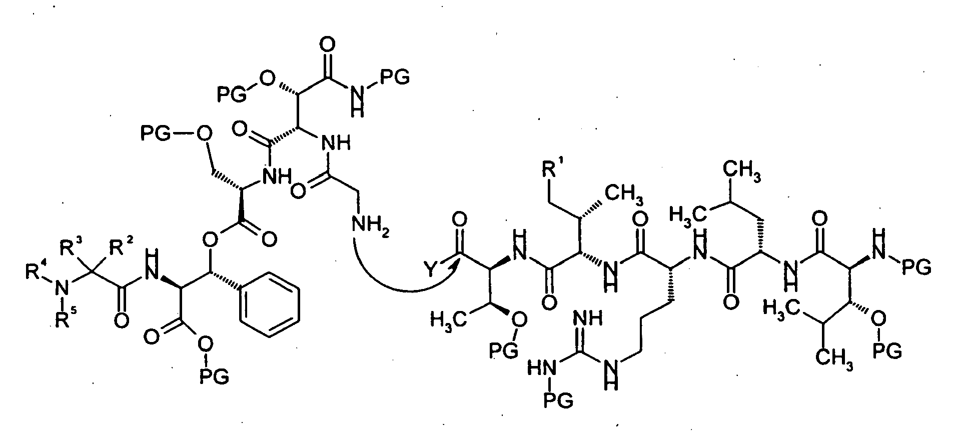

Die in dem Zwischenprodukt vorliegenden Schutzgruppen können vollständig, teilweise oder gar nicht mit denen des gewünschten Produkts übereinstimmen. Diese können ggf. durch Verfahren, die dem Fachmann bekannt sind, entfernt, ersetzt oder angebracht werden.

In einer weiteren Ausführungsform der Erfindung wird eine Verbindung der Formel (III) durch Kuppeln einer Verbindung der folgenden Formel (V) mit einer Verbindung der folgenden Formel (VI) 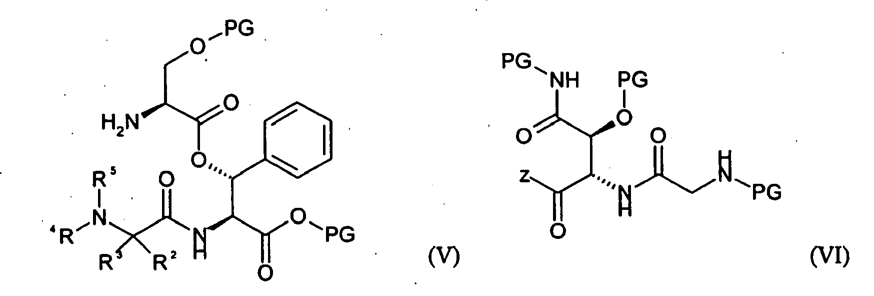
in denen R² bis R⁵ wie zuvor definiert sind,
in denen Z = OH, ein Aktivester, ein Pseudohalogen oder ein Halogen ist, und
in denen PG = H oder eine geeignete Schutzgruppe ist,
und ggf. teilweises oder vollständiges Entschützen des Zwischenprodukts hergestellt.

Insbesondere ist die Verbindung (V) hierbei eine Verbindung der folgenden Formel (Va) 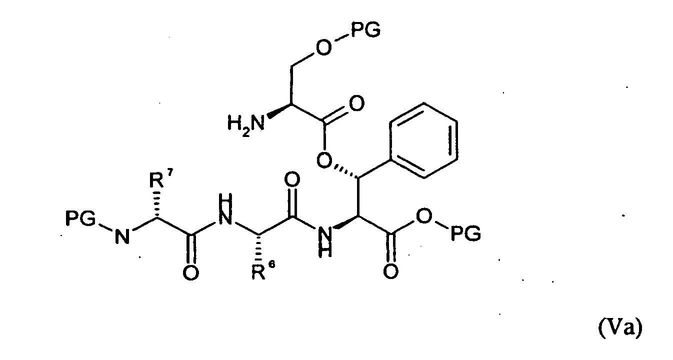
in der R⁶ und R⁷ wie zuvor definiert sind, und
in der PG = H oder eine geeignete Schutzgruppe ist.

Das Kuppeln kann unter den gleichen oder anderen Bedingungen erfolgen, wie zuvor für das zuvor genannte Kuppeln bzw. die intramolekulare Cyclisierung beschrieben. Das Kuppeln erfolgt dabei durch den hiernach dargestellten nucleophilen Angriff. 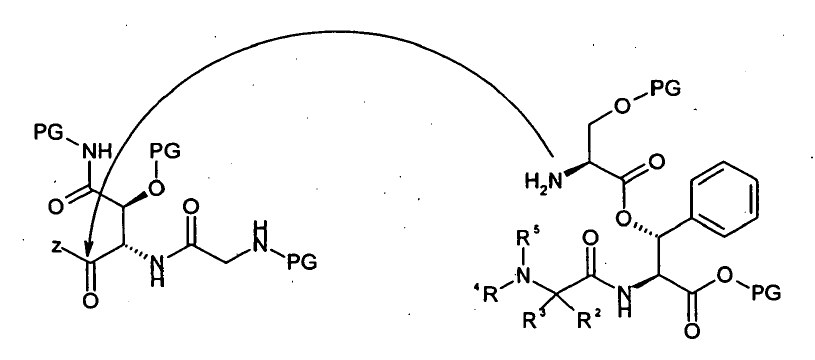 Die in dem Zwischenprodukt vorliegenden Schutzgruppen können teilweise, vollständig oder gar nicht mit denen des gewünschten Produktes übereinstimmen. Diese können ggf. durch Verfahren, die dem Fachmann bekannt sind, angebracht, entfernt oder ersetzt werden.

Die Erfindung betrifft ferner eine Verbindung der folgenden Formel (III) 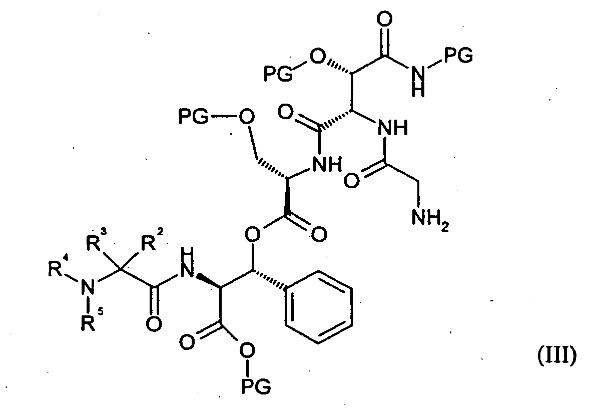
in der R² bis R⁵ wie zuvor definiert sind, und
in der PG = H oder eine geeignete Schutzgruppe ist,
insbesondere eine Verbindung der folgenden Formel (IIIa) 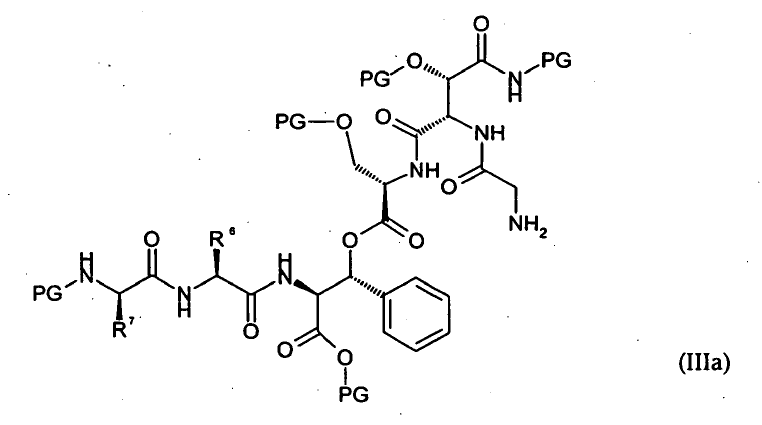
in der R⁶ und R⁷ wie zuvor definiert sind, und
in der PG = H oder eine geeignete Schutzgruppe ist,
und besonders eine Verbindung der folgenden Formel (IIIb).

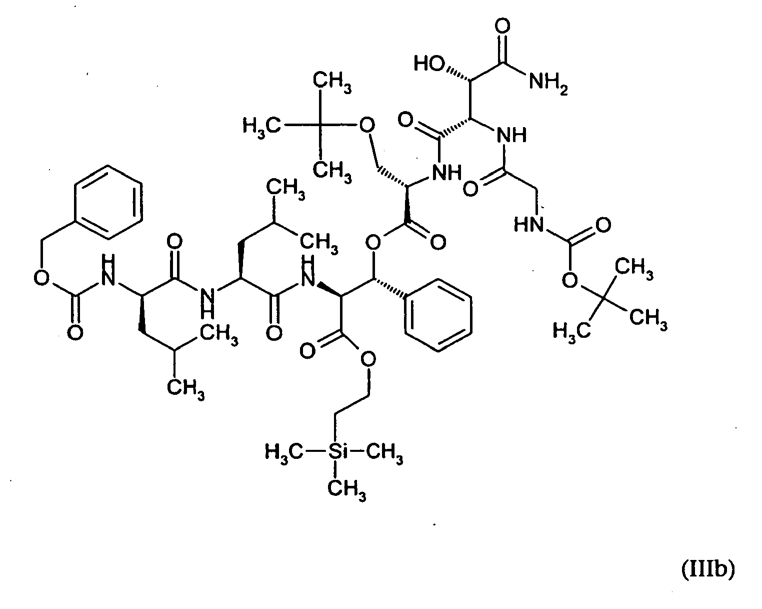

Die Erfindung betrifft ferner ein Verfahren zum Herstellen einer Verbindung der Formel (III) durch Kuppeln einer Verbindung der Formel (V) mit einer Verbindung der Formel (VI) und ggf. teilweises oder vollständiges Entschützen des Zwischenprodukts.

Die Erfindung betrifft ferner eine Verbindung der folgenden Formel (VI) 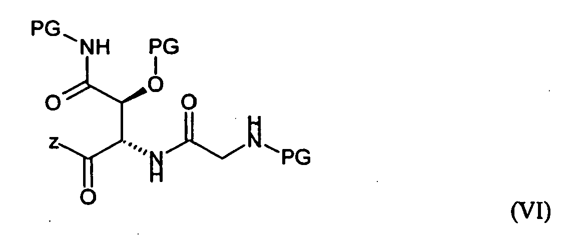
in der Z = OH, ein Aktivester, ein Pseudohalogen oder ein Halogen ist, und
in der PG = H oder eine geeignete Schutzgruppe ist,
insbesondere eine Verbindung der folgenden Formel (VIa).

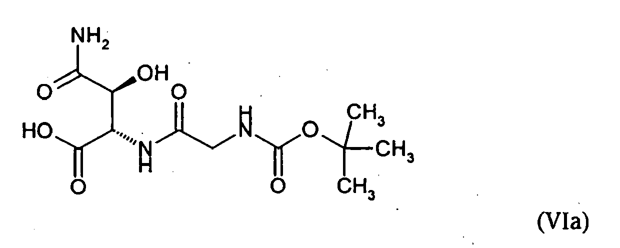

Sowie ein Verfahren zum Herstellen einer Verbindung der Formel (VI) durch Kuppeln einer Verbindung der folgenden Formel (VII) mit einer Verbindung der folgenden Formel (VIII) 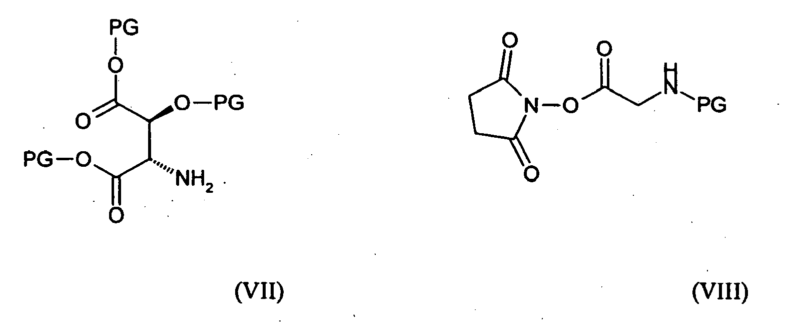 in denen PG = H oder eine geeignete Schutzgruppe ist,
und ggf. vollständiges oder teilweises Entschützen des Zwischenprodukts.

Insbesondere sind die Verbindungen der Formeln (VII) und (VIII) hierbei Verbindungen der folgenden Formeln (VIIa) bzw. (VIIIa).

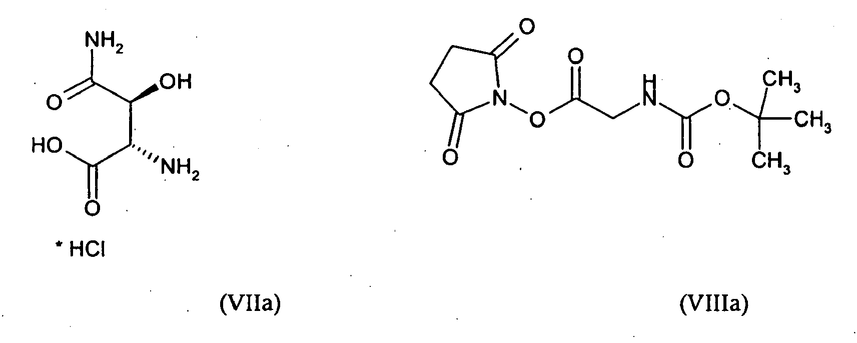

Die Herstellungsverfahren der Erfindung und die Herstellung der erfindungsgemäßen Verbindungen werden durch die folgenden Syntheseschemata an Hand der Synthese von Lysobactin näher erläutert.

Das Verfahren beruht auf einem modulartigen Aufbau von verschiedenen Fragmenten, die dann zu einer Verbindung der Formel (II) zusammengeführt werden. Die Verbindung der Formel (II) wird dann einer intramolekularen Cyclisierung unterworfen und ggf. entschützt, um das gewünschte Endprodukt zu erhalten.

Es hat sich hierbei überraschenderweise gezeigt, dass, beeinflusst von der Wahl der Bausteine und der Reihenfolge, in der diese gekuppelt werden, die offenkettigen Moleküle der Formel (II), die bereits eine Ester-Bindung in ihrer Kette aufweisen, hergestellt werden können. Ferner wurde festgestellt, dass diese offenkettigen Verbindungen in Gegenwart der Ester-Bindung zu den gewünschten cyclischen Depsipeptiden cyclisiert werden können.

Gemäß Syntheseschema 1 wird ein Fragment 1 ausgehend von (2S,3S)-2-Amino-3-hydroxy-4-methoxy-4-oxobuttersäure und Boc-Glycin-*N*-hydroxysuccinimidester synthetisiert.

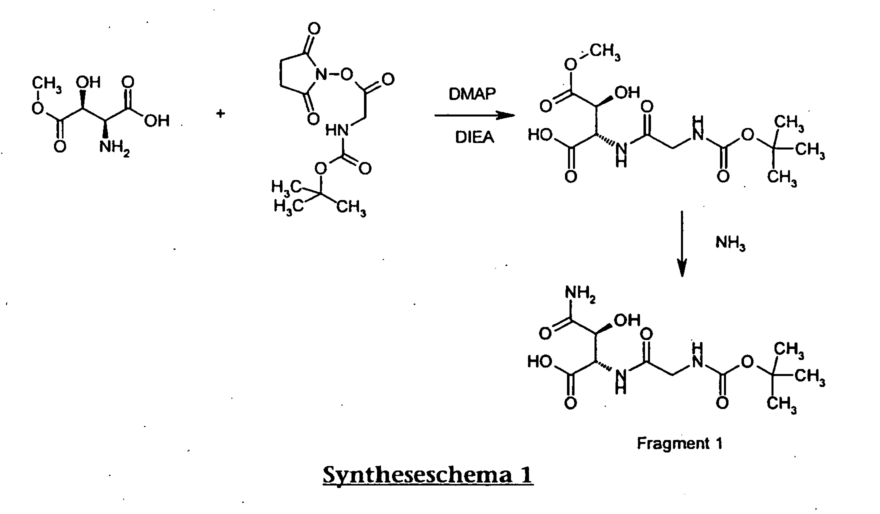

Ein Fragment 2 wird gemäß Syntheseschema 2 ausgehend von 3-Hydroxy-phenylalanin hergestellt.

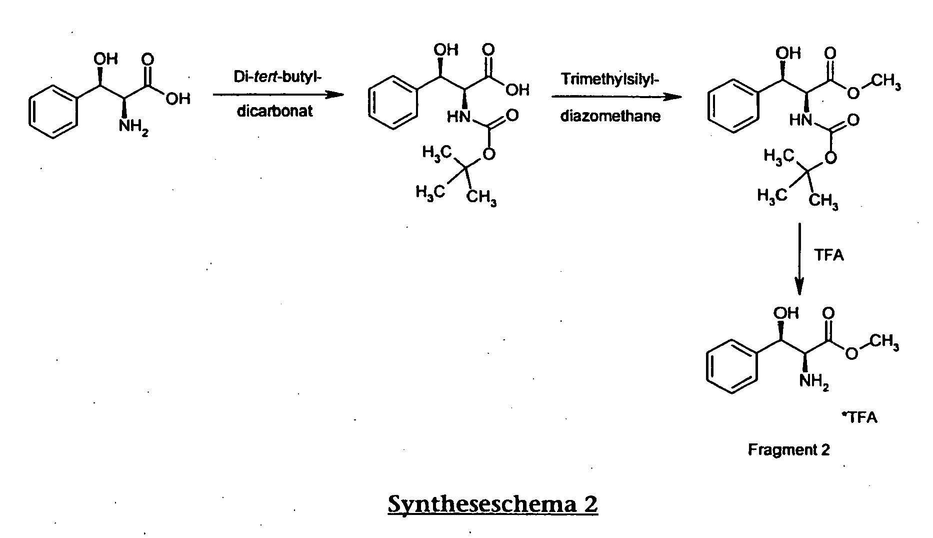

Ein Fragment 3 wird gemäß Syntheseschema 3 ausgehend von *N*²-(Benzyloxycarbonyl)-D-Leucin und Methyl L-Leucinat gemäß Syntheseschema 3 hergestellt. Durch Ersetzen der beiden Leucin-Derivate in diesem Schritt können Verbindungen der Formel (I) mit beliebigen R² bis R⁵-Resten hergestellt werden.

Die Fragmente 2 und 3 werden dann gemäß Syntheseschema 4 gekuppelt, teilweise entschützt und das erhaltene Zwischenprodukt mit *N*²-(*tert*-Butoxycarbonyl)-O³-*tert-*butyl-L-serin umgesetzt, um nach einem weiteren Entschützen ein teilweise entschütztes Fragment 4 zu erhalten.

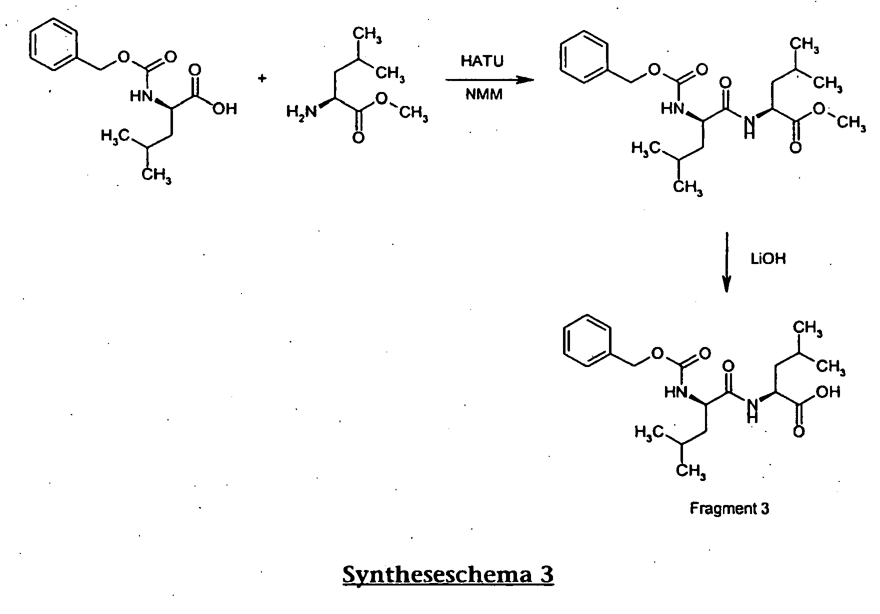

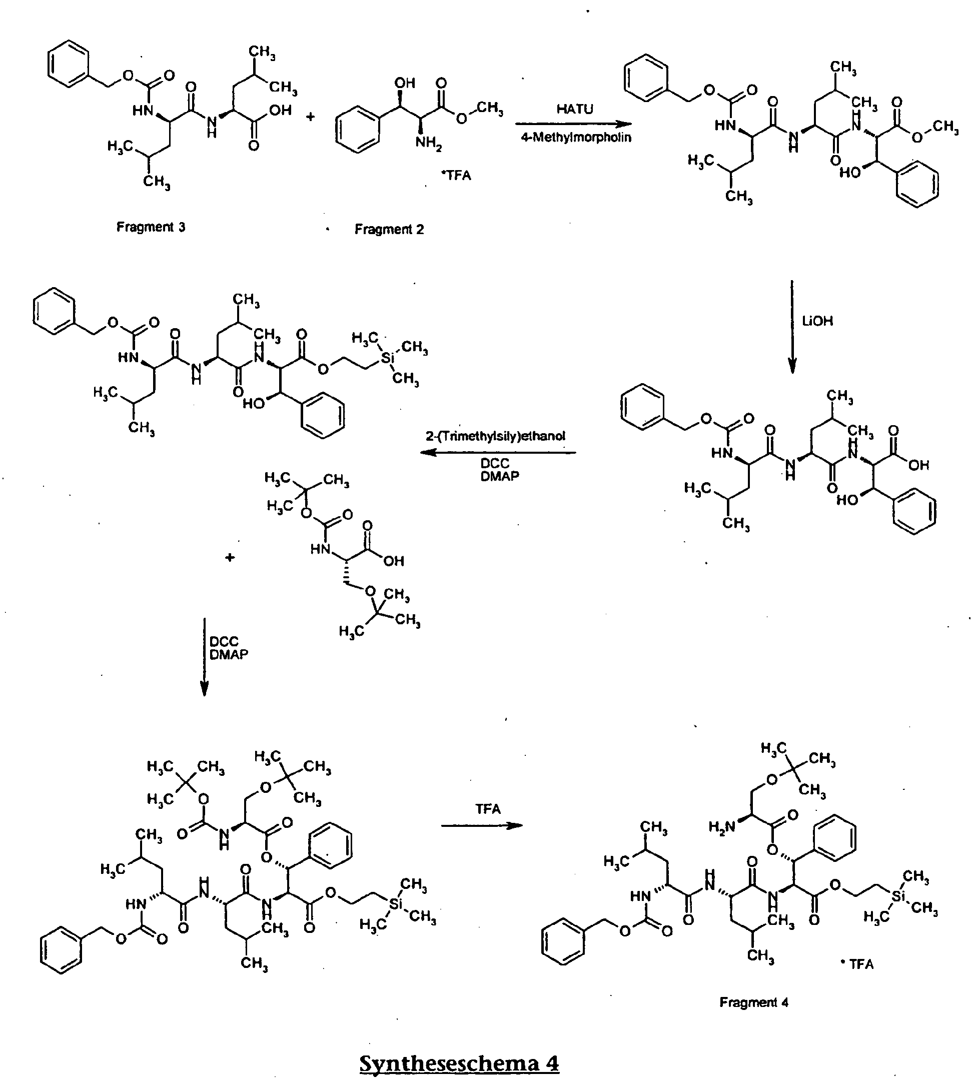

Das so erhaltene Fragment 4 wird gemäß Syntheseschema 5 mit dem Fragment 1 gekuppelt, um nach teilweisem Entschützen ein Fragment 5 zu erhalten.

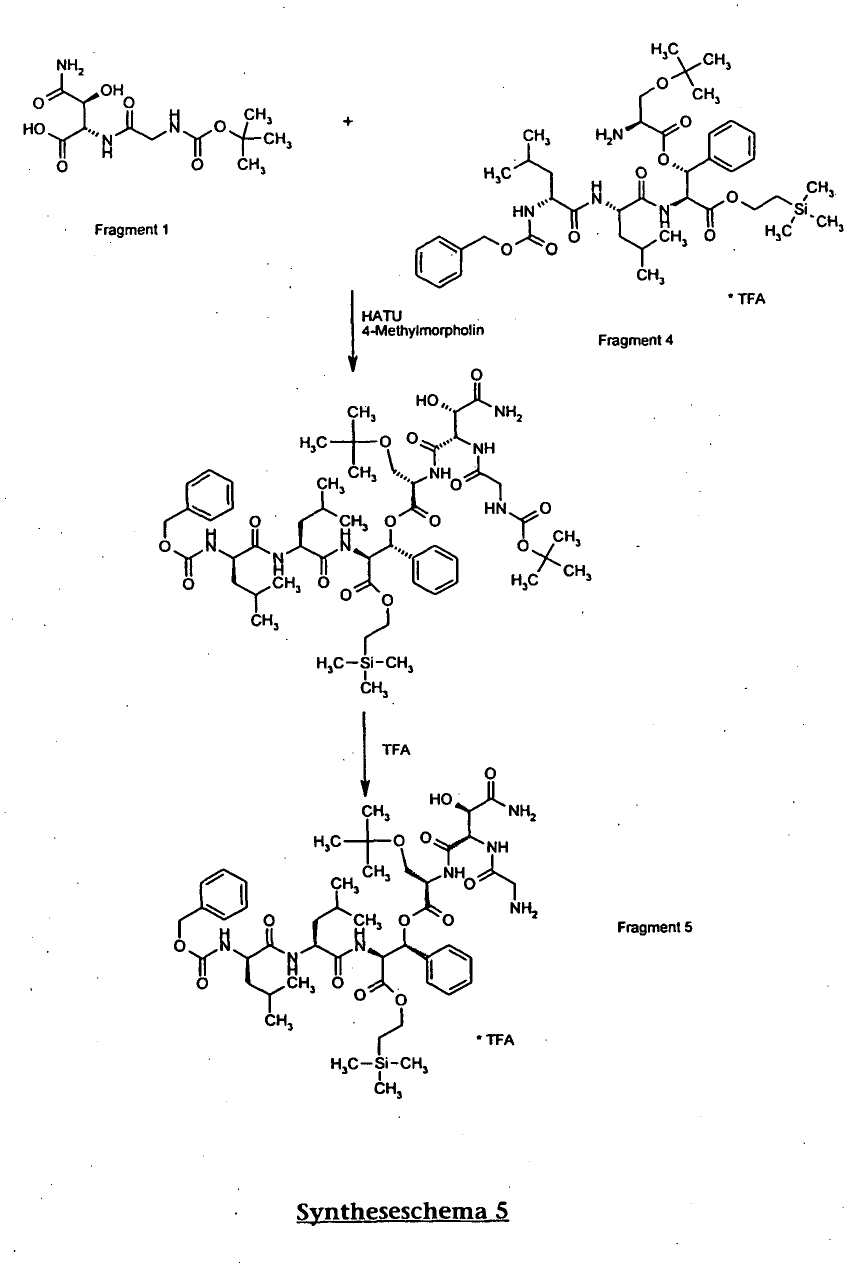

Das Fragment 5 wird dann gemäß Syntheseschema 6 mit einem Fragment 6 gekuppelt. Bei diesem Fragment 6 handelt es sich um ein Pentapeptid, das gemäß bekannter Methoden hergestellt werden kann. Durch Ersetzen des Leucins an Position 2 in Fragment 6 durch ein Valin kann anstatt Lysobactin, Katanosin A hergestellt werden. Nach Entschützen des erhaltenen Zwischenprodukts wird ein Fragment 7 erhalten, welches eine Verbindung der Formel (II) darstellt. Nach einer intramolekularen Cyclisierung und anschließendem Entschützen gemäß Syntheseschema 7 wird das gewünschte cyclische Depsipeptid, in diesem Fall Lysobactin, erhalten.

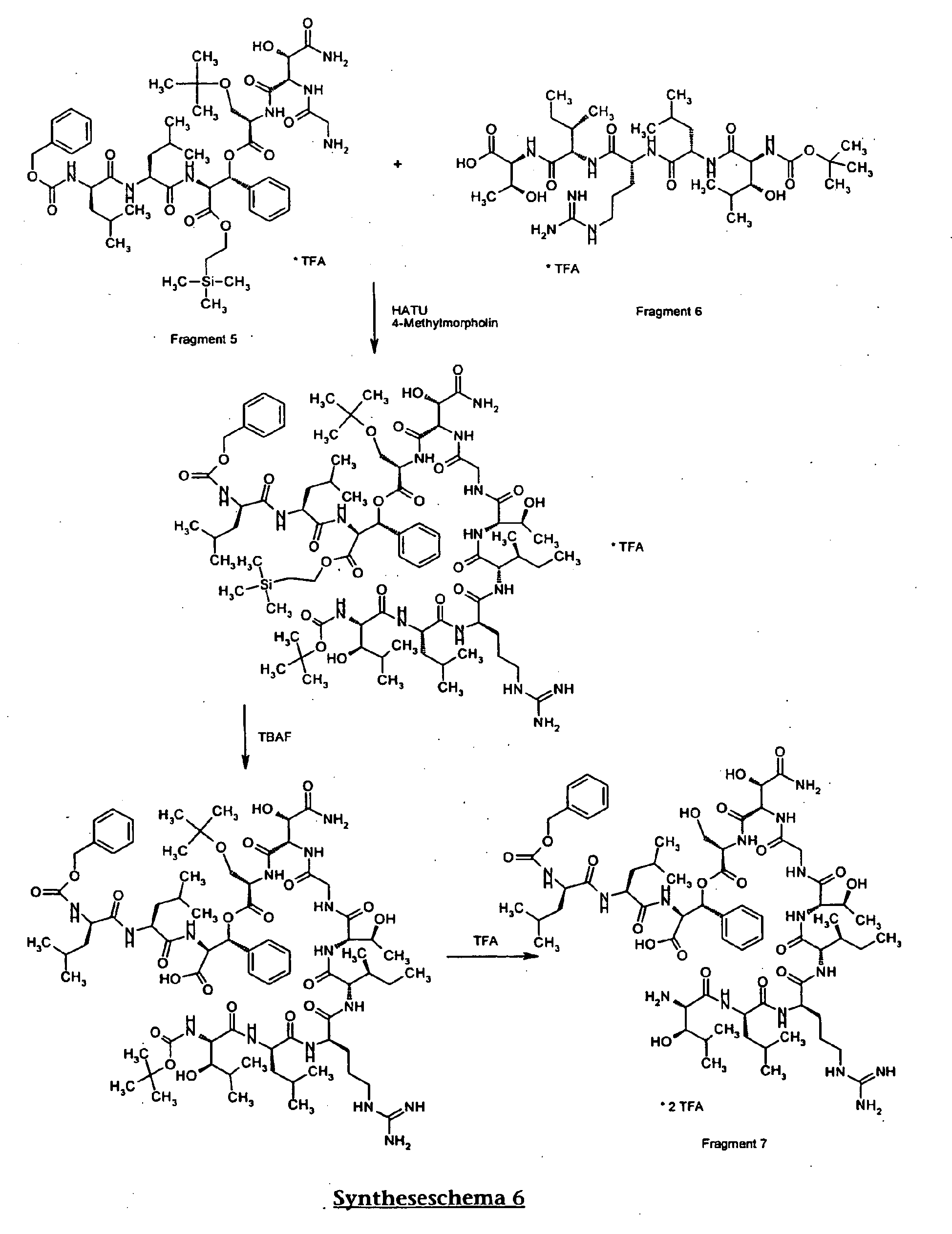

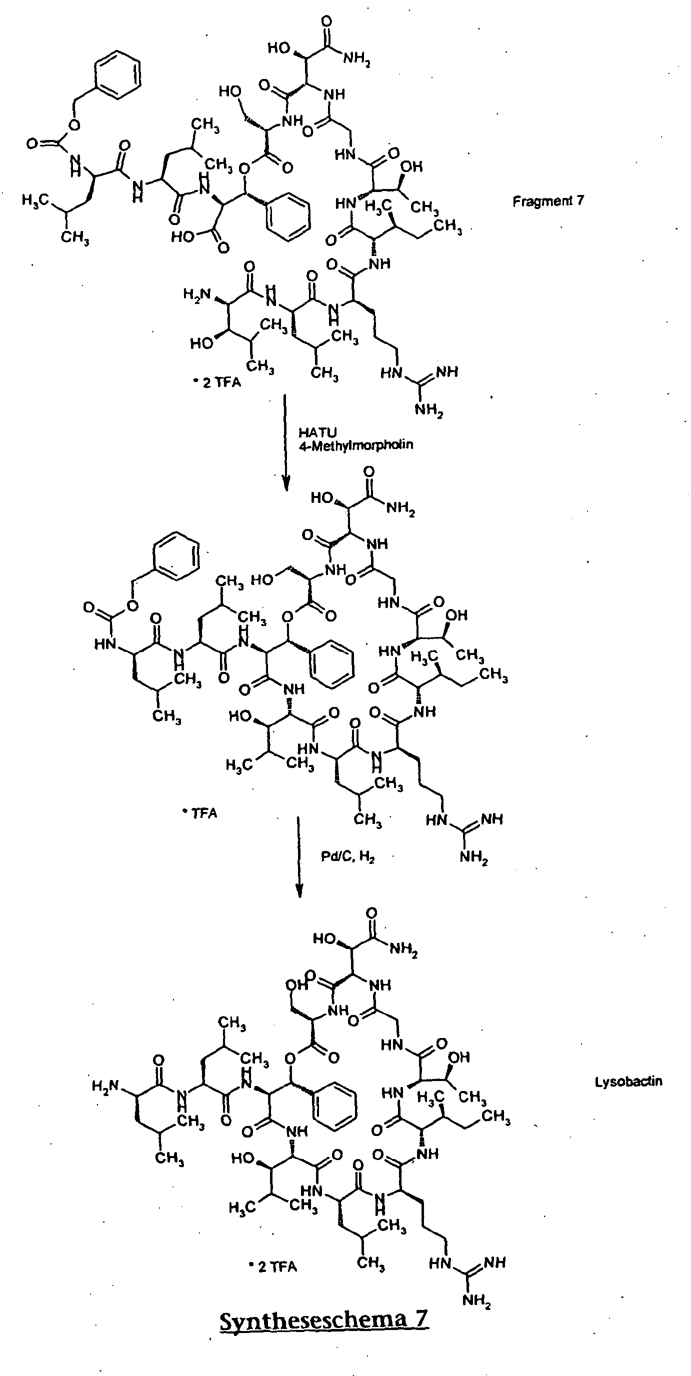

### Beispiel

### De novo Synthese von Lysobactin

### Abkürzungen

- abs.: absolut
- Äq.: Äquivalent(en)
- Boc: *N-tert*-Butoxycarbonyl
- d. Th.: der Theorie
- DCC: Dicyclohexylcarbodiimid
- DIEA: *N,N*-Diisopropylethylamin
- DMAP: 4-Dimethylaminopyridin
- DMF: *N,N*-Dimethylformamid
- EDC: 1-(3-Dimethylaminopropyl)-3-ethylcarbodiimid Hydrochlorid
- EDTA: Ethylendiamintetraessigsäure (Ethylene Diamine Tetraacetic Acid)
- Fa.: Firma
- fmoc: 9-Fluorenylmethoxycarbonyl
- ges.: gesättigt(er)
- h: Stunde(n)
- HATU: *O*-(7-Azabenzotriazol-1-yl)-*N,N,N',N'*-tetramethyluronium Hexafluor- phosphat
- HOBT: 1-Hydroxy-1*H*-benzotriazol Hydrat
- Konz.: Konzentrierte(r)
- LHMDS: Lithiumhexamethyldisilazid
- min: Minute(n)
- MTBE: Methyl-*tert-*butylether
- NMM: *N*-Methylmorpholin
- org.: organische(n)
- RT: Raumtemperatur
- TBAF: Tetrabutylammoniumfluorid
- TBTU: *N*-[(1*H*-1,2,3-Benzotriazol-1-yloxy)(dimethylamino)methylene]-N- methylmethanaminium Tetrafluorborat
- TFA: Trifluoressigsäure
- THF: Tetrahydrofuran
- TMG: *N,N,N,N*-Tetramethylguanidin
- wässr.: wäßrig(e)
- XPHOS: Dicydohexyl(2',4',6'-triisopropylbiphenyl-2-yl)phosphin

### Material und Methoden

### Analytik Methoden - HPLC/UV

### Methode 1

Gerätetyp HPLC: HP 1100 Series; UV DAD; Säule: Phenomenex Synergi 2 µ Hydro-RP Mercury 20 mm x 4 mm; Eluent A: 1 l Wasser + 0.5 ml 50%ige Ameisensäure, Eluent B: 11 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min. 2 ml/min; Ofen: 50°C; UV-Detektion: 210 nm.

### Methode 2

Instrument: HP 1100 mit DAD-Detektion; Säule: Kromasil 100 RP-18, 60mm x 2,1 mm, 3.5µm; Eluent: A=5ml HClO₄ (70%ig) /L H₂O, B= ACN; Gradient: 0 min 2%B, 0.5 min 2%B, 4.5 min 90%B, 9 min 90%B, 9.2 min. 2%B, 10 Min 2%B; Fluß: 0.75 ml/min; SäulenTemp.: 30°C; Detektion: UV 210 nm

### Methode 3

Gerät: Agilent 1100 mit DAD (G1315B), binärer Pumpe (G1312A), Autosampler (G1313A), Lösemittel-Entgaser (G1379A) und Säulenthermostat (G1316A); Säule: Agilent Zorbax Eclipse XDB-C8 4.6 x 150 x 5 mm; Säulentemperatur: 30°C; Eluent A: 0.05% 70% Perchlorsäure in Wasser; Eluent B: Acetonitril; Fluss: 2.00 mL/min; Gradient: 0-1 min 10% B, Rampe, 4-5 min 90% B, Rampe, 5.5 min 10% B.

### Methode 4

Gerätetyp HPLC: HP 1050 Series; UV DAD; Säule: Zorbax 300 mSB-C18 3.5 µ, 4.6 mm x 150 mm; Eluent A: 1 l Wasser + 0.1% TFA, Eluent B: 60 % Acetonitril in Wasser mit 0.1 % TFA; Gradient: 0.0 min 10%B, Rampe, 18.0 min 80%B, 20.0 min 100%B, 25.0 min 100%B. Fluss: 1 ml/min; Ofen: 40°C; UV-Detektion: 210 nm.

### Methode 5

Gerätetyp HPLC: HP 1050 Series; UV DAD; Säule: Zorbax 300 mSB-C18 3.5 µ, 4.6 mm x 150 mm; Eluent A: 11 Wasser + 0.1% TFA, Eluent B: 60 % Acetonitril in Wasser mit 0.1 % TFA; Gradient: 0.0 min 10%B, 2.00 min 10 % B, Rampe, 50.0 min 80%B, 52.0 min 100%B, 55 min 100 % B. Fluss: 0.7 mL/min; Ofen: 40°C; UV-Detektion: 210 nm.

### Methode 6

Agilent 1100 mit DAD (G1315B), Binary Pump (G1312A), Autosampler (G1313A), Entgaser (G1379A) und Säulenthermostat (G1316A); Säule: Phenomenex Gemini 5 µ C-18, 50 x 2 mm; Ofentemperatur: 40 °C; Eluens A: Wasser + 0.1% Ameisensäure; Eluens B: Acetonitril; Fluss: 2.00 mL/min; Gradient: 0-1 min 0% B, Rampe, 0-5 min 100 % B, 5.50 min 100 % B.

### Methode 7

Daicel Chiralpak AD-H 5 µm 250 mm x 2.0 mm, n-Heptan/ Ethanol 95+5, Fluss: 0.2 mL/min, UV-Detektion bei 220 nm.

### Analytik Methoden - HPLC/MS, MALDI, HR-MS

### Methode 8

Gerätetyp MS: Micromass ZQ; Gerätetyp HPLC: Waters Alliance 2795/HP 1100; Säule: Phenomenex Synergi 2 µ Hydro-RP Mercury 20 mm x 4 mm; Eluent A: 1 l Wasser + 0.5 ml 50%ige Ameisensäure, Eluent B: 11 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 mein/4.5 min 2 ml/min; Ofen: 50°C; UV-Detektion: 210 nm.

### Methode 9

Gerätetyp MS: Micromass ZQ; Gerätetyp HPLC: Waters Alliance 2795/HP 1100; Säule: Phenomenex Gemini 3 µ C-18 100 Ä, 30 mm x 3 mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.

### Methode 10

UV-Detektion: 210 nm. Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 1 L Wasser + 0.5 ml 50%ige Ameisensäure, Eluent B: 1 L Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.

### Methode 11

Instrument: Micromass Platform LCZ mit HPLC Agilent Serie 1100; Säule: Thermo Hypersil GOLD 3µ 20 mm x 4 mm; Eluent A: 1 L Wasser + 0,5 ml 50%ige Ameisensäure, Eluent B: 1 L Acetonitril + 0,5 ml 50%ige Ameisensäure; Gradient: 0.0min 100%A → 0.2min 100%A → 2.9min 30%A → 3.1min 10%A → 5.5min 10%A; Ofen: 50°C; Fluss: 0.8 ml/min; UV-Detektion: 210 nm.

### Methode 12

Instrument: Micromass Platform LCZ mit HPLC Agilent Serie 1100; Säule: Thermo HyPURITY Aquastar 3µ 50 mm x 2.1 mm; Eluent A: 1 l Wasser + 0.5 ml 50%ige Ameisensäure, Eluent B: 1 l Acetonitril + 0.5 ml 50%ige Ameisensäure; Gradient: 0.0 min 100%A → 0.2 min 100%A → 2.9 min 30%A → 3.1 min 10%A → 5.5 min 10%A; Ofen: 50°C; Fluss: 0.8 ml/min; UV-Detektion: 210 nm.

### Methode 13

Gerät: Micromass LCT; Ionisation: ESI positiv/negativ; HP1100 mit DAD und Autosampler; Ofen 40°C; Säule: Waters Symmetry C-18, 50 x 2.1 mm, 3.5 µm; Eluent A: 0.1% Ameisensäure/Acetonitril, Eluent B: 0.1% Ameisensäure/Wasser; Fluss: 0.5 ml/min.; Gradient: 0-1 min 0% A, 1-6 min 90% A, 6-8 min 100% A, 8-10 min 100% A, 10-15 0% A.

### Methode 14

*TOF-HR-MS-ESI+* Spektren werden mit einem Micromass LCT Gerät (Kapillarspannung: 3.2 KV, Cone-Spannung: 42 V, Quellentemperatur: 120°C, Desolvations-Temperatur: 280°C) aufgenommen. Hierzu wird eine Spritzenpumpe (Fa. Harvard Apparatus) für die Probenzuführung verwendet. Als Standard dient Leucin-Enkephalin (Tyr-Gly-Gly-Phe-Leu).

### Präparative Trennmethoden - HPLC, Gelchromatographie

### Methode 15

Gerät: Gilson Abimed HPLC; binäres Pumpensystem; Säule: Nucleodur C₁₈ Gravity, Fa. Macherey-Nagel, 5 µm; 250 x 21 mm; Eluent A: Wasser/0.05%-0.1% TFA, Eluent B: Acetonitril; Gradient: 0-8 min 5%B, 8-40 min 5-60%B, 40-60 min 60%B, 60-75 min 60-100%B, 75-80 min 100%B, anschließend Regeneration der Chromatographiesäule; Fluss: 7-15 ml/min; UV Detektor 210 nm.

### Methode 16

Gerät: Gilson Abimed HPLC; binäres Pumpensystem; Säule: Kromasil-100A C₁₈, 5 µm; 250 x 30 mm; Eluent A: Wasser/0.05-0.5% TFA, Eluent B: Acetonitril; Gradient: 0-5 min 5%B, 5.01-10 min 10%B, 10.01-20 min 40%B, 20.01-27 min 50%B, 27.01-40 min 60%B, 40.01-45 min 90%B, 45.01-60 min 100%B; Fluss: 15-60 ml/min; UV Detektor 210 nm.

### Methode 17

Gilson Abimed HPLC; UV-Detector 210 nm; Säule: Kromasil RP-18 5 µm, 100 Å, 250 x 20 mm; Eluens A: Wasser + 0.05% TFA, Eluens B: Acetonitril + 0.05% TFA: Fluss: 10 mL/min; 0-3 min 5% B, Rampe, 35 min 90% B.

### Methode 18

Gilson Abimed HPLC; UV-Detector 210 nm; Säule: Gromsil ODS-4HE 10 µm, 250 x 40 mm; Eluens A: Wasser + 0.05% TFA, Eluens B: Acetonitril + 0.05% TFA: Fluss: 20 mL/min; 0-3 min 10% B, Rampe, 30-35 min 90% B, 35-40 min 90% B.

### Methode 19

Gilson Abimed HPLC; UV-Detektor 210 nm; Säule: Waters Symmetry-Prep™ C-18, 7 µm, 300 x 19 mm; Eluens A: Wasser + 0.05% TFA, Eluens B: Acetonitril + 0.05% TFA: Fluss: 10 mL/min; 0-3 min 10% B, Rampe, 30-38 min 90% B, 38-45 min 10% B.

### Methode 20

Gelchromatographie wird ohne Druck an Sephadex LH-20 (Fa. Pharmacia) durchgeführt. Es wird nach UV-Aktivität (UV Detektor für 254 nm, Fa. Knauer) fraktioniert (Fraktionssammler ISCO Foxy 200). Säulendimensionen: 32 x 7 cm (1000-100 µmol Maßstab); 30 x 4 cm (100-10 µmol Maßstab); 25 x 2 cm (10-1 µmol Maßstab). Als Eluent wird Methanol verwendet.

### Methode 21

Gilson Abimed HPLC; UV-Detektor 210 nm; Säule: Biotage Flash40 RP-18 oder kompatibles Modul Varian Metaflash C18 40M, 35 µm, 150 x 40 mm; Eluens A: Wasser + 0.05% TFA, Eluens B: Acetonitril + 0.05% TFA: Fluss: 40 mL/min; 0-3 min 10% B, Rampe, 30-38 min 90% B, 38-45 min 10% B.

### Allgemeine Arbeitsmethoden

### Arbeitsvorschrift 1

Das Ausgangsmaterial wird in 30% TFA (Lösung in Dichlormethan) aufgenommen und 30 min bei Raumtemp. gerührt. Dann wird das Lösungsmittel im Vakuum abdestilliert, wobei die Badtemperatur 30°C nicht übersteigen soll. Das Produkt wird danach im Ölpumpenvakuum bis zur Gewichtskonstanz getrocknet.

### A. Darstellung der Verbindungen

### Beispielverbindung 1A: N²-(tert-Butoxycarbonyl)-L-allothreonin

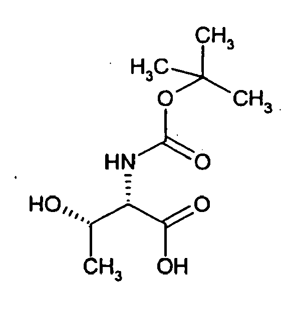

L-*allo*-Threonin (3.15 g, 26.44 mmol) wird in Wasser-Dioxan (1+2, 75 mL) gelöst, Di*tert*-butyldicarbonat (6.35 g, 29.09 mmol, 1.1 Äquivalente) und Triethylamin (4.79 mL, 34.38 mmol, 1.3 Äquivalente) werden zugegeben und die Mischung wird über Nacht bei Raumtemperatur gerührt. Dann wird das Lösungsmittel im Vakuum abgezogen. Der Rückstand wird in Ethylacetat aufgenommen und mit 1 M Citronensäure ausgeschüttelt. Die wässrige Phase wird noch mehrmals mit Ethylacetat extrahiert, bis sich darin kein Produkt mehr nachweisen lässt (HPLC, Methode 3). Dann werden die vereinigten organischen Extrakte über Natriumsulfat getrocknet, eingeengt und im Ölpumpenvakuum bis zur Gewichtskonstanz getrocknet. Das Produkt wird ohne weitere Reinigung weiter umgesetzt. Ausbeute: 6.5 g Rohprodukt.

**HPLC (Methode 3): Rₜ = 3.23 min.** LC-MS (Methode 11): Rₜ = 2.51 min, MS (ESIneg): m/z (%) = 217.8 (100) [M-H]⁻.

¹H NMR (400 MHz, *d₆*-DMSO) δ (ppm) = 1.08 (d, *J* = 5.4 Hz, 3H), 1.38 (s, 9H), 3.72-3.84 (m, 2H), 6.77 (d, *J* = 7.4 Hz, 1H).

### Beispielverbindung 2A: Benzyl N²-(tert-Butoxycarbonyl)-L-allothreoninat

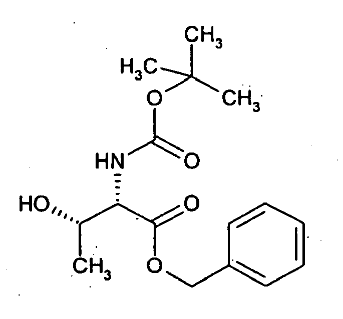

Die Methode wurde in Analogie zu folgender Literatur durchgeführt: S. B. Cohen, R. Halcomb, J. Am. Chem. Soc 2004, 124, 2534-2543. W. Jiang, J. Wanner, R. J. Lee, P.-Y. Bounaud, D. L. Boger, J. Am. Chem. Soc 2003, 125, 1877-1887.

Beispielverbindung 1A (6.8 g Rohprodukt, 26.44 mmol), wird in Methanol (177 mL) aufgenommen, Caesiumcarbonat (5.56 g, 17.06 mmol, 0.63 Äquivalente) wird hinzugefügt und bis zur vollständigen Auflösung gerührt. Dann entfernt man das Lösemittel destillativ, setzt DMF (42 mL) und dann Benzylbromid (4.06 mL, 34.12 mmol, 1.26 Äquivalente) zu. Man lässt die Mischung 16 h rühren, dann wird das DMF weitgehend im Vakuum abgezogen. Der Rückstand wird in Wasser aufgenommen und mit 3 Portionen Dichlormethan extrahiert. Die vereinigten org. Phasen werden über Natriumsulfat getrocknet, filtriert und im Vakuum eingeengt. Das Rohprodukt wird an Biotage RP18-Flash (Wasser-Acetonitril-Gradient: 0-5 min. 10% ACN, 3-30 min. 10 - 90% ACN, 30-35 min. 90% ACN; Flow: 20mL/min.) aufgereinigt. Produkthaltige Fraktionen werden vereinigt und lyophilisiert. Ausbeute: 5.00 g (16.16 mmol, 52 % d. Th.) der Titelverbindung.

HPLC (Methode 3): Rₜ = 4.36 min.

LC-MS (Methode 8): Rₜ = 2.39 min, MS (ESIpos): m/z (%) = 332.6 (25) [M+H]⁺.

¹H NMR (400 MHz, *d₆*-DMSO) δ (ppm) = 1.09 (d, *J* = 6.4, 3H), 1.37 (s, 9H), 3.82 (m, 1H), 3.95 (dd, *J* = 6.4, *J* = 8.1 Hz), 4.98 (d, *J* = 5.4 Hz, 1H), 5.09 (d, *J* = 12.7 Hz, 1H), 5.16 (d, *J* = 12.7 Hz, 1H), 7.10 (d, *J* = 8.1 Hz, 1H), 7.31-7.37 (m, 5H).

### Beispielverbindung 3A: Benzyl L-Allothreoninat-trifluoracetat

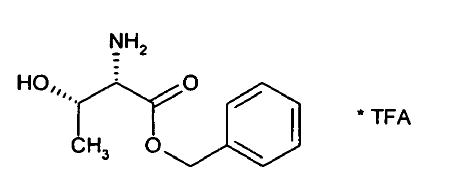

530 mg der Beispielverbindung 2A werden nach Arbeitsvorschrift 1 mit 8.0 mL der TFA-Lösung umgesetzt. Das Rohprodukt (589 mg, quant.) wird ohne weitere Reinigung weiter umgesetzt.

HPLC (Methode 3): Rₜ = 3.18 min.

LC-MS (Methode 11): Rₜ = 2.24 min, MS (ESIpos): m/z (%) = 210.0 (100) [M+H]⁺.

¹H NMR (400 MHz, *d₆*-DMSO) δ (ppm) = 1.15 (d, *J* = 6.6 Hz, 3H), 4.09-4.10 (m, 2H), 5.26 (s, 2H), 7.36-7.44 (m, 5H), 8.34 (br. S, 2H).

### Beispielverbindung 4A: Benzyl [N²-(tert-Butoxycarbonyl)-L-isoleucyl]-L-allothreoninat

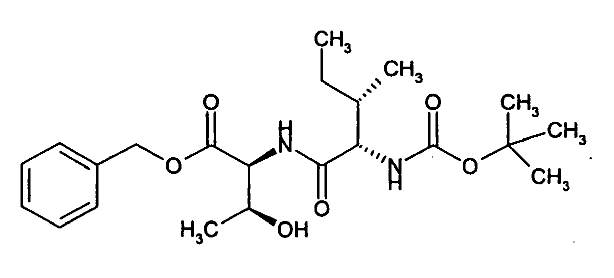

Beispielverbindung 3A (2.30 g 7.12 mmol) und *N*-(*tert*-Butoxycarbonyl)-L-isoleucin (2.14 g, 9.25 mmol, 1.3 Äquivalente) werden in DMF (21.0 mL) gelöst. 4-Methylmorpholin (1.3 mL, 12.02 mmol, 1.7 Äquivalente) und HATU (3.52 g, 9.25 mmol, 1.3 Äquivalente) werden zugegeben und der Ansatz 16 h bei Raumtemperatur gerührt. Dann wird der gesamte Ansatz zunächst nach Methode 20 und anschließend nach Methode 21 chromatographisch gereinigt. Produkthaltige Fraktionen werden vereinigt und lyophilisiert. Ausbeute: 1.75 g (4.14 mmol, 58 % d. Th.) als hellbeigefarbener amorpher Feststoff.

HPLC (Methode 3): Rₜ = 4.59 min.

LC-MS (Methode 8): Rₜ = 2.56 min, MS (ESIpos): m/z (%) = 423.8 (70) [M+H]⁺.

¹H NMR (400 MHz, *d₆*-DMSO) δ (ppm) = 0.74-0.78 (m, 6H), 1.01-1.07 (m, 2H), 1.10 (d, *J* = 6.3 Hz, 3H), 1.37 (s, 9H), 1.64-1.66 (m, 1H), 3.86-3.94 (m, 1H), 4.28 (dd, *J* = 7.3, *J* = 7.3 Hz, 1H), 5.05 (d, *J* = 5.6 Hz), 5.09 (d, *J* = 12.7 Hz, 1H), 5.13 (d, *J* = 12.7 Hz, 1H), 6.70 (d, *J*= 9.0 Hz, 1H), 7.31-7.36 (m, 5H), 8.11 (d, *J* = 8.1 Hz).

HR-TOF-MS (Methode 14): C₂₂H₃₅N₂O₆ ber. 423.2490, gef. 423.2489 [M+H]⁺.

### Beispielverbindung 5A: Benzyl L-Isoleucyl-L-allothreoninat-trifluoracetat

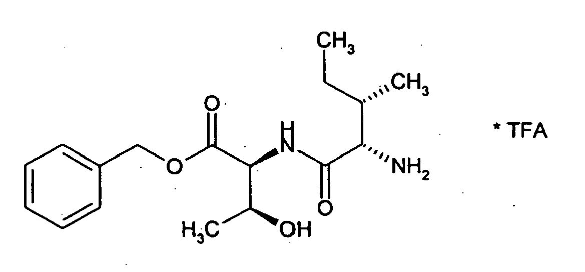

Beispielverbindung 4A (224 mg, 0.53 mmol) wird nach Arbeitsvorschrift 1 mit 8.0 mL der TFA Lösung behandelt. Man erhält 253 mg rohes Produkt Beispiel 5A (ca. 91 % rein, 0.53 mmol, quant.), das ohne weitere Reinigung umgesetzt wird.

HPLC (Methode 3): Rₜ = 3.51 min.

LC-MS (Methode 8): Rₜ = 1.58 min, MS (ESIpos): m/z (%) = 323.6 (100) [M+H]⁺.

¹H NMR (400 MHz, *d₆*-DMSO) δ (ppm) = 0.77-0.86 (m, 6H), 1.02 (m, 1H), 1.15 (d, *J* = 6.4 Hz, 3H), 1.45 (m, 1H), 1.77 (m, 1H), 3.97 (m, 1H), 4.34 (m, 1H), 5.11 (d, *J* = 12.5 Hz, 1H), 5.16 (d, *J* = 12.5 Hz, 1H), 7.37-7.39 (m, 5H), 7.47 (m, 1H), 8.07-8.08 (m, 3H), 8.69 (d, *J* = 7.3 Hz, 1H).

### Beispielverbindung 6A: Benzyl [N²-(tert-Butoxycarbonyl)-D-arginyl]-L-isoleucyl-L-allothreoninat

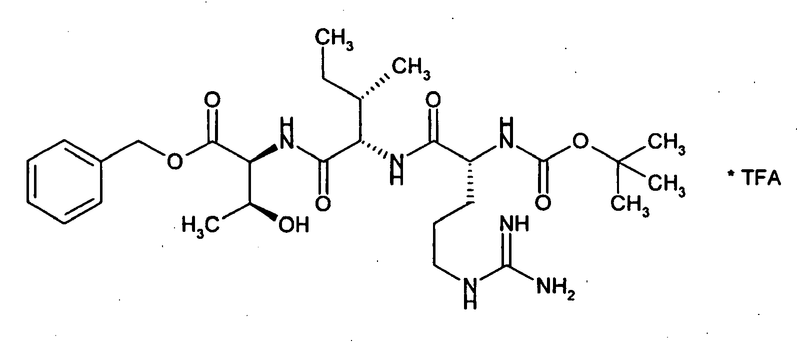

Beispielverbindung 5A (253 mg 91 % rein, 0.53 mmol) und *N*²-(*tert*-Butoxycarbonyl)-D-arginin (145 mg, 0.53 mmol, 1 Äquivalent) werden in DMF (3.0 mL) gelöst. 4-Methylmorpholin (76 µL, 0.70 mmol, 1.3 Äquivalente) und HATU (221 mg, 0.58 mmol, 1.1 Äquivalente) werden zugegeben und der Ansatz 16 h bei Raumtemperatur gerührt. Dann wird der gesamte Ansatz auf eine HPLC-Säule gegeben und chromatographisch gereinigt (Methode 18). Produkthaltige Fraktionen werden vereinigt und lyophilisiert. Ausbeute: 364 mg (0.53 mmol, 99 % d. Th.) der Titelverbindung.

HPLC (Methode 3): Rₜ = 3.91 min.

LC-MS (Methode 8): Rₜ = 2.04 min, MS (ESIpos): m/z (%) = 579.9 (100) [M+H]⁺.

¹H NMR (400 MHz, *d₆*-DMSO) δ (ppm) = 0.72-1.16 (m, 8 H), 1.37 (s, 9H), 1.46 (m, 2H), 1.60 (m, 1 H), 1.69 (m, 1H), 3.06 (m, 2H), 3.93-4.01 (m, 2H), 4.25 (m, 1H), 4.33 (m, 1H), 5.07-5.14 (m, 2H), 6.96 (d, *J* = 7.8, 1H), 7.35 (m, 5H), 7.45 (m, 1H), 7.66 (d, *J* = 8.8), 8.33 (m, 1H).

### Beispielverbindung 7A: Benzyl D-Arginyl-L-isoleucyl-L-allothreoninat-bis-trifluoracetat

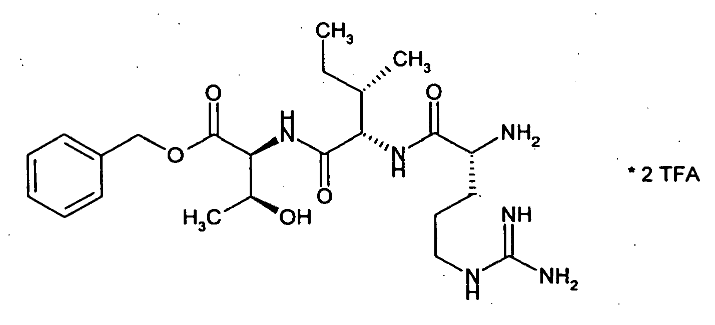

Beispielverbindung 6A (237 mg, 0.34 mmol) wird nach Arbeitsvorschrift 1 mit 2.0 mL der TFA Lösung behandelt. Man erhält 255 mg rohes Produkt Beispielverbindung 7A (94 % rein, 0.34 mmol, quant.), das ohne weitere Reinigung umgesetzt wird.

HPLC (Methode 3): Rₜ = 3.42 min.

LC-MS (Methode 11): Rₜ = 2.42 min, MS (ESIpos): m/z (%) = 479.3 (50) [M+H]⁺.

¹H NMR (400 MHz, *d₆*-DMSO) δ (ppm) = 0.73-0.81 (m, 5H), 1.11-1.19 (m, 5H), 1.33-1.49 (m, 3H), 1.74 (m, 3H), 3.10 (m, 2H), 3.88-3.95 (m, 2H), 4.25 (dd, *J* = 6.8, *J*= 7.1 Hz, 1H), 4.46 (dd, *J* = 7.3, *J* = 8.8 Hz, 1H), 5.09 (d, *J* = 12.5 Hz, 1H), 5.15 (dd, *J* = 12.5 Hz), 7.36 (m, 5H), 7.61 (m, 1H), 8.10 (m, 2H), 8.51 (d, *J* = 7.6 Hz, 1H), 8.57 (d, *J* = 9.0 Hz, 1H).

### Beispielverbindung 8A: Benzyl [N²-(tert-Butoxycarbonyl)-L-leucyl]-D-arginyl-L-isoleucyl-L-allothreoninat-trifluoracetat

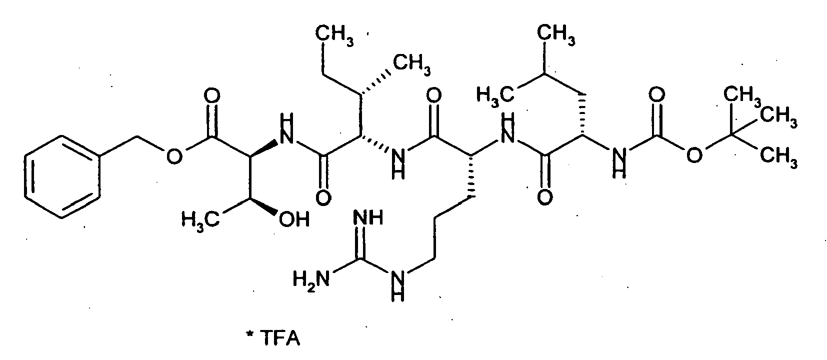

Beispielverbindung 7A (240 mg, 0.34 mmol) und *N*-(*tert*-Butoxycarbonyl)-L-leucin (79 mg, 0.34 mmol, 1 Äquivalent) werden in Dichlormethan-DMF (5+1, 6 mL) gelöst. Diisopropylethylamin (296 µL, 1.70 mmol, 5 Äquivalente) und HATU (194 mg, 0.51 mmol, 1.5 Äquivalente) werden zugegeben und der Ansatz 24 h bei Raumtemperatur gerührt. Dann wird der gesamte Ansatz auf eine Gelchromatographie-Säule gegeben und chromatographisch gereinigt (Methode 20, Eluens ist Methanol). Produkthaltige Fraktionen werden vereinigt und eingeengt. Ausbeute: 146 mg (0.18 mmol, 53 % d. Th.) der Titelverbindung.

HPLC (Methode 3): Rₜ = 4.15 min.

LC-MS (Methode 8): Rₜ = 1.92 min, MS (ESIpos): m/z (%) = 692.8 (100), [M+H]⁺.

¹H NMR (400 MHz, *d₆*-DMSO) δ (ppm) = 0.72-1.23 (m, 22H), 1.37 (s, 9H), 1.38-1.71 (m, 3H), 3.08 (m, 2H), 3.91-4.00 (m, 2H), 4.26 (m, 1H), 4.33-4.42 (m, 2H), 5.07-5.15 (m, 2H), 6.92 (d, *J* = 7.8 Hz, 1H), 7.35 (m, 5H), 7.47 (m, 1H), 7.88 (d, *J* = 8.1 Hz, 1H), 7.93 (d, *J* = 9.0 Hz, 1H), 8.35 (d, *J* = 7.3 Hz, 1H).

### Beispielverbindung 9A: Benzyl L-Leucyl-D-arginyl-L-isoleucyl-L-allothreoninat-bis-trifluoracetat

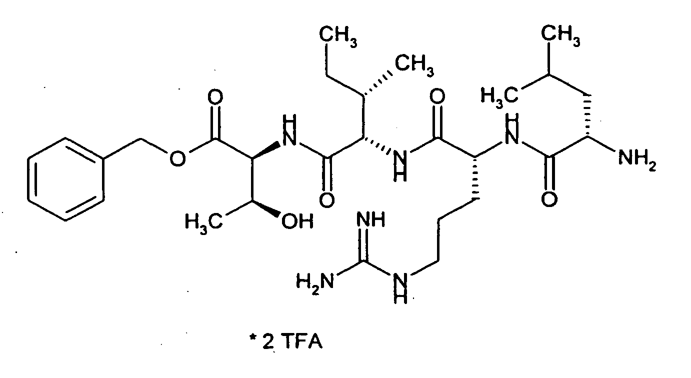

Beispielverbindung 8A (220 mg, 0.27 mmol) wird nach Arbeitsvorschrift 1 mit 2.0 mL der TFA Lösung behandelt. Man erhält 223 mg rohes Produkt Beispiel 9A (0.27 mmol, quant.), das ohne weitere Reinigung umgesetzt wird.

HPLC (Methode 2): Rₜ = 3.80.

LC-MS (Methode 11): Rₜ = 2.54 min, MS (ESIpos): m/z (%) = 592.4 (2) [M+H]⁺.

¹H NMR (400 MHz, *d₆*-DMSO) δ (ppm) = 0.73-1.11 (m, 13H), 1.22-1.74 (m, 12H), 3.11 (m, 4H), 3.60 (m, 2H), 3.87 (m, 1H), 3.95 (m, 1H), 4.25 (m, 1H), 4.38 (dd, *J* = 7.8, J = 8.6 Hz, 1H), 4.64 (dd, *J* = 7.8, *J* = 13.7 Hz, 1H), 5.09 (d, *J* = 12.7 Hz, 1H), 5.13 (d, *J* = 12.7 Hz, 1H), 7.35 (m, 5H), 7.58 (m, 1H), 8.07 (m, 2H), 8.25 (d, *J* = 8.8 Hz, 1H), 8.39 (d, *J* = 7.6 Hz, 1H), 8.77 (d, *J* = 8.3 Hz, 1H).

### Beispielverbindung 10A: Benzyl ((3R)-N²-(tert-Butoxycarbonyl)-3-hydroxy-L-leucyl]-L-leucyl-D-arginyl-L-isoleucyl-L-allothreoninat-trifluoracetat

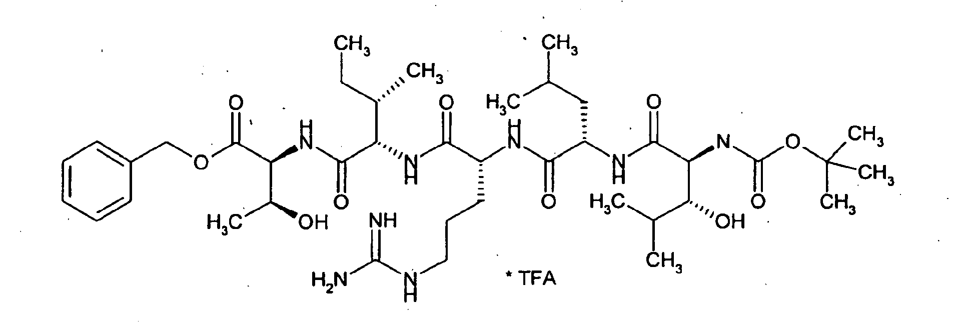

Beispielverbindung 9A (223 mg, 0.27 mmol) und *N*-(*tert*-Butoxycarbonyl)-(*3R*)-3-hydroxy-L-leucin (89 mg, 0.33 mmol, 1.22 Äquivalente) werden in DMF (6 mL) gelöst und die Lösung auf -20 °C gekühlt. 4-Methylmorpholin (150 µL, 1.36 mmol, 5 Äquivalente) und HATU (165 mg, 0.44 mmol, 1.6 Äquivalente) werden zugegeben und der Ansatz 16 h bei Raumtemperatur gerührt. Dann wird der gesamte Ansatz auf eine Gelchromatographie-Säule gegeben und chromatographisch gereinigt (Methode 20, Eluens ist Methanol). Produkthaltige Fraktionen werden vereinigt und eingeengt. Ausbeute: 188 mg (0.20 mmol, 74 % d. Th.) der Titelverbindung.

HPLC (Methode 3): Rₜ = 4.24 min.

LC-MS (Methode 9): Rₜ = 1.99 min, MS (ESIpos): m/z (%) = 821.9 (100) [M+H]⁺.

¹H NMR (400 MHz, *d₆*-DMSO) δ (ppm) = 0.71-0.90 (m, 15H), 1.00 (m, 1H), 1.10 (d, *J*= 6.4 Hz, 3H), 1.24-1.26 (m, 3H), 1.38 (s, 9H), 1.42-1.71 (m, 6H), 3.06-3.17 (m, 3H), 3.45 (m, 1H), 3.61 (m, 1H), 3.93 (m, 1H), 4.05 (m, 1H), 4.26 (m, 1H), 4.35 (m, 2H), 4.54 (d, *J* = 7.8 Hz, 1H), 5.07-5.15 (m, 2H), 5.45 (d, *J* = 9.0 Hz, 1H), 7.35 (m, 5H), 7.46 (m, 1H), 7.85 (d, *J* = 7.8 Hz, 1 H), 7.89 (d, *J* = 8.8 Hz, 1 H), 7.97 (d, *J* = 8.1 Hz, 1H), 8.35 (d, *J* = 7.6 Hz, 1H).

### Beispielverbindung 11A: [(3R)-N²-(tert-Butoxycarbonyl)-3-hydroxy-L-leucyl]-L-leucyl-D-arginyl-L-isoleucyl-L-allothreonin-trifluoracetat

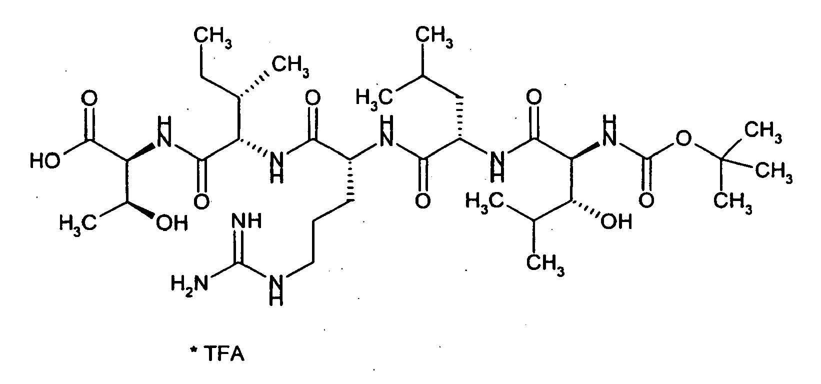

Beispielverbindung 10A (100 mg, 0.11 mmol) wird in Eisessig (4.3 mL) gelöst, 10 % Palladium auf Aktivkohle (22 mg) wird hinzugefügt und der Ansatz 2 h bei Raumtemperatur unter Normaldruck hydriert. Man filtriert vom Katalysator ab und lyophilisiert das Filtrat. Das Rohprodukt wird chromatographisch gereinigt (Methode 17). Produkthaltige Fraktionen werden vereinigt und lyophilisiert. Man erhält 58 mg (60 µmol, 55 % d. Th.) der Titelverbindung.

HPLC (Methode 3): Rₜ = 3.75 min.

LC-MS (Methode 9): Rₜ = 1.80 min, MS (ESIpos): m/z (%) = 731.8 (100) [M+H]⁺.

### Beispielverbindung 12A: [N²-(tert-Butoxycarbonyl)-glycyl]-(3S)-3-hydroxy-O⁴-methyl-L-asparaginsäure

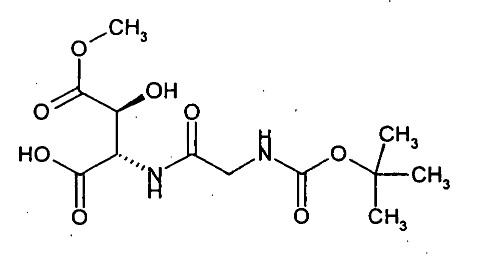

(*3S*)-3-Hydroxyasparaginsäure wird nach der Vorschrift G. Cardillo, L. Gentilucci, A. Tolomelli, C. Tomasini, Synlett 1999, 1727-1730, dargestellt und analog P. G. Mattingly, M. J. Miller, J. Org. Chem. 1983, 48, 3556-3559, unter Verwendung von Mikrowellenstrahlung in einem geschlossenen Reaktor zu (2*S*,3*S*)-2-Amino-3-hydroxy-4-methoxy-4-oxobuttersäure Hydrochlorid umgesetzt.

(2*S*,3*S*)-2-Amino-3-hydroxy-4-methoxy-4-oxobuttersäure Hydrochlorid (447 mg, 2.24 mmol) werden in DMF (9 mL) gelöst. Die Lösung wird auf 0 °C gekühlt, Boc-glycin-N-hydroxysuccinimidester (763 mg, 2.91 mmol, 1.3 Äquivalente), DMAP (14 mg, 0.11 mmol, 0.05 Äquivalente) und schließlich DIEA (1170 µL, 6.72 mmol, 3 Äquivalente) werden hinzugefügt. Die Mischung lässt man langsam auf Raumtemperatur erwärmen und dann noch 2 h rühren. Der Ansatz wird mit Eisessig angesäuert, mit Acetonitril versetzt und an Sephadex LH 20 (Methode 20) chromatographiert. Produkthaltige Fraktionen werden vereinigt, eingeengt und erneut chromatographiert (Methode 21). Produkthaltige Fraktionen werden vereinigt und lyophilisiert. Das erhaltene Produkt (761 mg, quant.) wird ohne weitere Reinigung weiter umgesetzt. Für analytische Zwecke wird eine reine Probe durch HPLC (Methode 19) erhalten.

HPLC (Methode 3): Rₜ = 3.15 min

LC-MS (Methode 8): Rₜ = 1.17 min, MS (ESIPOS) = 321.2 [M+H]⁺.

[α]²⁰_{Na} = + 39° (c = 0.55, MeOH).

¹H NMR (300 MHz, d₆-DMSO) δ (ppm) = 1.40 (s, 9 H), 3.49 - 3.60 (m, 2 H), 3.61 (s, 3 H), 4.29 (m, 1 H), 4.73 (d, *J* = 6.6 Hz, 1 H), 7.01 (m, 1 H), 7.49 (d, *J* = 6.99 Hz, 1 H).

¹³C-NMR (d₆-Aceton, 126 MHz, DEPT) δ (ppm) = 28.5 (CH₃), 42.2 (CH₂), 51.8 (CH₃), 53.7 (CH), 56.0 (CH), 79.2 (quat), 169.6 (quat), 169.7 (quat), 172.8 (quat), 173.8 (quat).

HR-TOF-MS (Methode 14): C₁₂H₂₂N₂O₈ [M+H]⁺ ber.: 321.1298, gef.: 321.1299.

### Beispielverbindung 13A: [N²-(tert-Butoxycarbonyl)-glycyl]-(3S)-3-hydroxy-L-asparagin

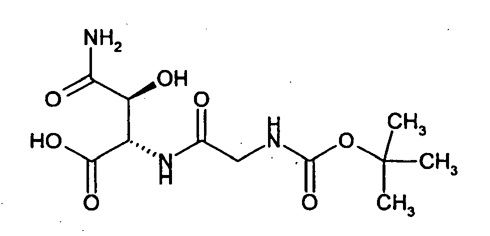

Beispielverbindung 12A (353 mg, 1.10 mmol) wird in 25 % wässrigem Ammoniak (1.70 mL) gelöst und der Ansatz ca. 2 h bei RT gerührt. Sobald die Umsetzung vollständig ist (Nachweis mit HPLC, Methode 3), wird im Ölpumpenvakuum zur Trockne eingeengt und der Rückstand mit HPLC (Methode 17) gereinigt. Produkthaltige Fraktionen werden vereinigt und lyophilisiert. Ausbeute: 172 mg (51 % d. Th.) der Titelverbindung als farbloser Feststoff.

HPLC (Methode 3): Rₜ = 2.70 min.

LC-MS (Methode 11): Rₜ = 2.21 min, MS (ESIpos): m/z (%) = 306 (70) [M+H]⁺.

### Beispielverbindung 14A: (3R)-3-Hydroxy-phenylalanin

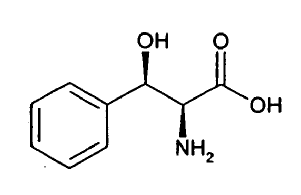

Diese Beispielverbindung wird nach der Methode von Belokon (Y. N. Belokon, K. A. Kochetkov, N. S. Ikonnikov, T. V. Strelkova, S. R. Harutyunyan, A. S. Saghiyan, Tetrahedron: Asymmetry 2001, 12, 481-485) synthetisiert.

LC-MS (Methode 12): Rₜ = 0.41 min, MS (ESIpos): m/z (%) = 182.1 (100) [M+H]⁺.

[α]²⁰_{Na} = - 21° (*c* = 0.1, MeOH). Lit (D. Alker, G. Hamblett, L. M. Harwood, S. M. Robertson, D. J. Watkin, C. E. Williams, Tetrahedron 1998, 54, 6089-6098.): [α]²²_{Na} = -20° (*c* = 0.8, MeOH).

¹H NMR (400 MHz, D₂O) δ (ppm) = 3.84 (d, *J* = 4.5 Hz, 1 H), 4.64 (d, *J* = 4.5 Hz, 1 H), 7.30-7.36 (m, 5 H).

### Beispielverbindung 15A: N-Butoxycarbonyl-(3R)-3-Hydroxy-phenylalanin

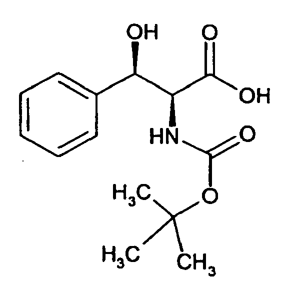

Beispielverbindung 14A (0.5 g, 2.76 mmol) wird in 1,4-Dioxan-Wasser (2+1, 9 mL) aufgenommen und mit Triethylamin (500 µL, 3.59 mmol, 1.3 Äquivalente) und Di*tert*-butyldicarbonat (660 mg, 3.04 mmol, 1.3 Äquivalente) versetzt. Der Ansatz wird 16 h bei Raumtemperatur gerührt und dann mit 1 M Citronensäure abgestoppt. Es wird mit mehreren Portionen Ethylacetat extrahiert, bis in der wässrigen Phase kein Produkt mehr per HPLC (Methode 3) nachweisbar ist. Die vereinigten organischen Phasen werden über Natriumsulfat getrocknet und eingeengt. Im Rückstand erhält man 759 mg (2.70 mmol, 98 % d. Th.) der Titelverbindung als farbloses Öl.

HPLC (Methode 3): Rₜ = 3.89 min.

LC-MS (Methode 8): Rₜ = 1.87 min, MS (ESIpos): m/z (%) = 282.3 (40) [M+H]⁺.

¹H NMR (400 MHz, d₆-DMSO) δ (ppm) = 1.27 (s, 9 H, COC(C*H*₃)₃), 4.21 (m, 1 H), 5.09 (s, 1 H), 6.30 (d, *J* = 9.8 Hz, 7.22-7.34 (m, 5 H).

Chirale HPLC (Methode 7): e.e. 94.1 %.

### Beispielverbindung 16A: Methyl N-Butoxycarbonyl-(3R)-3-hydroxy-phenylalaninat

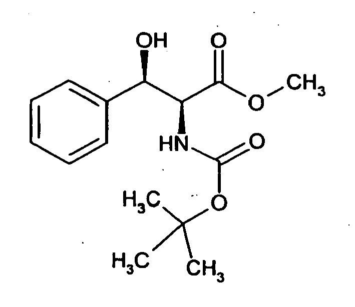

Beispielverbindung 16A (331 mg, 1.11 mmol) wird in Dichlormethan-Methanol (5+1, 12 mL) gelöst, auf 0 °C gekühlt und Trimethylsilyldiazomethan (2 M in THF, 1.66 mL, 3.32 mmol, 3 Äquivalente) wird zugetropft. Bei 0 °C wird der Ansatz noch 30 min gerührt und dann mit einigen Tropfen TFA bis zur Entfärbung versetzt. Das Lösungsmittel wird abdestilliert, als Rückstand verbleibt die Titelverbindung (345 mg, 95 % rein laut HPLC) in quantitativer Ausbeute als gelbliches Öl.

HPLC (Methode 3): Rₜ = 4.26 min.

LC-MS (Methode 8): Rₜ = 2.11 min, MS (ESIpos): m/z (%) = 296.3 (50) [M+H]⁺.

¹H NMR (300 MHz, d₆-DMSO) δ (ppm) = 1.27 (s, 9 H, COC(C*H*₃)₃), 3.60 (s, 3 H, OC*H*₃), 4.31 (dd, *J* = 3.8, *J* = 9.3 Hz, 1 H), 5.03 (d, *J* = 3.4 Hz, 1 H), 5.68 (br s, 1 H), 6.62 (d, *J* = 9.1 Hz, 1 H), 7.23-7.34 (m, 5 H).

### Beispielverbindung 17A: Methyl (3R)-3-Hydroxy-phenylalaninat-trifluoracetat

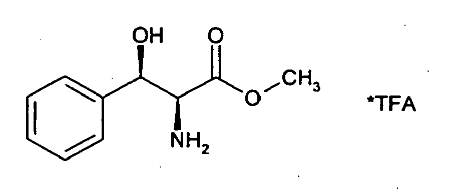

Beispielverbindung 16A (345 mg, 1.17 mmol) wird in 30 % TFA in Dichlormethan (10 mL) gelöst und 15 min bei RT gerührt. Dann wird das Lösungsmittel abdestilliert. Der Rückstand wird im Ölpumpenvakuum bis zur Gewichtskonstanz getrocknet. Ausbeute: 401 mg (quant.) als gelbes Öl, das ohne weitere Aufreinigung im nächsten Schritt eingesetzt wird.

HPLC (Methode 3): Rₜ = 2.51 min.

LC-MS (Methode 8): Rₜ = 0.30 min, MS (ESIpos): m/z (%) = 196.1 (20) [M+H]⁺.

### Beispielverbindung 18A: Methyl [N²-(Benzyloxycarbonyl)-D-leucyl]-L-leucinat

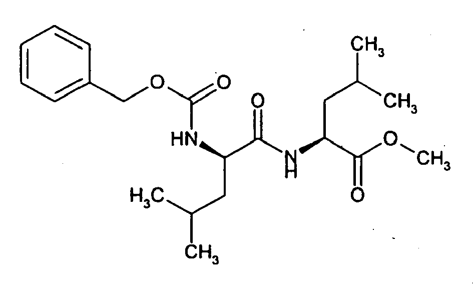

*N*²-(Benzyloxycarbonyl)-D-Leucin (BACHEM Cat No z13351.) (6.37 g, 24 mmol) und Methyl L-Leucinat (3.49 g, 24 mmol, 1 Äq.) werden bei 0°C in DMF (75 ml) gelöst, dann werden NMM (5.28 mL, 48 mmol, 2 Äq.) und HATU (13.69 g, 36 mmol, 1.5.Äq.) zugesetzt. Der Ansatz wird drei Stunden bei Raumtemperatur gerührt. Man versetzt mit MTBE und gesättigter Natriumhydrogencarbonatlösung und extrahiert. Die wässrige Phase wird mit einer zweiten Portion MTBE nachextrahiert, dann werden die vereinigten organischen Phasen mit 1 M Citronensäure sowie abermals mit gesättigter Natriumhydrogencarbonatlösung gewaschen, über Natriumsulfat getrocknet, filtriert und im Vakuum eingeengt. Der Rückstand wird in zwei Portionen chromatographisch gereinigt (Biotage 40M, Cyclohexan/Ethylacetat 3+1). Produkthaltige Fraktionen werden vereinigt und lyophilisiert. Ausbeute: 7.85 g (80% d. Th.) der Titelverbindung.

HPLC (Methode 3): Rₜ = 4.82 min.

LCMS (Methode 8): Rₜ = 2.65 min; MS (ESIpos.): m/z (%) = 393 (100) [M+H]⁺.

[α]²⁰_{Na} = -5.2 ° (*c* = 0.52, MeOH).

¹H NMR (400 MHz, *d*₆-DMSO) 8 (ppm) = 0.77-0.92 (m, 12H), 1.31-1.66 (m, 6H), 3.60 (s, 3H), 4.10 (m, 1H), 4.28 (m, 1H), 5.02 (s, 2H), 7.25-7.38 (m, 6H), 8.23 (d, 1H).

¹³C-NMR (126 MHz, *d*₆-DMSO) δ (ppm) = 21.1 (CH₃), 21.5 (CH₃), 22.8 (CH₃), 22.9 (CH₃), 24.2 (CH), 41.0 (CH₂), 50.0 (CH), 51.8 (CH₃, O*C*H₃), 52.9 (CH), 65.3 (CH₂, O*C*H₂Ph), 127.6 (CH, ar-C), 127.7 (CH, ar-C), 128.3 (CH, ar-C), 137.1 ((C quat, ar-C), 155.8 (C quat, N*C*OC(CH₃)₃), 172.4 (C quat, C=O), 172.9 (C quat, C=O).

### Beispielverbindung 19A: [N²- (Benzyloxycarbonyl)-D-leucyl]-L-leucin

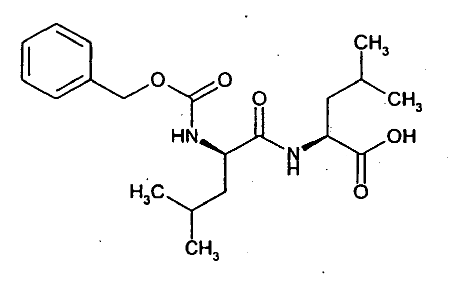

Beispielverbindung 18A (7.70 g, 19.62 mmol) wird in 200 mL THF/ Wasser (3+1) aufgenommen, auf 0°C gekühlt und mit Lithiumhydroxid-Monohydrat (1.65 g, 39.24 mmol, 2 Äq.) versetzt. Man lässt bei 0°C rühren, bis nach HPLC- Kontrolle (Methode 3) die Umsetzung vollständig abgelaufen ist (ca. 45 min). Man destilliert den Großteil des THF im Vakuum ab, stellt dann durch Zusatz von Citronensäure auf ca. pH 4 und extrahiert mit 2 Portionen Ethylacetat. Die vereinigten org. Phasen werden über Natriumsulfat getrocknet, filtriert und eingeengt. Das Produkt wird als farblose amorphe Substanz in einer Ausbeute von 6.87 g (89% d. Th.) der Titelverbindung erhalten.

HPLC (Methode 3): Rₜ = 4.45 min.

LCMS (Methode 8): Rₜ = 2.39 min, MS (ESIpos.) m/z (%) = 379 (100) [M+H]⁺, 757 (40) [2M+H]⁺.

[α]²⁰_{Na} = +4.7 ° (*c* = 0.50, MeOH).

¹H NMR (300 MHz,*d₆*-DMSO) δ (ppm) = 0.77-0.92 (m, 12H), 1.34-1.68 (m, 6H), 4.04-4.26 (m, 2H), 5.02 (s, 2H), 7.25-7.38 (m, 6H), 8.12 (d, 1H), 12.50 (br. s, 1H).

HR-TOF-MS (Methode 14): C₂₀H₃₁N₂O₅ [M+H]⁺ ber. 379.2228 , gef. 379.2216.

### Beispielverbindung 20A: Methyl [N²-(Benzyloxycarbonyl-D-leucyl]-L-leucyl-(3R)-3-hydroxy-phenylalaninat

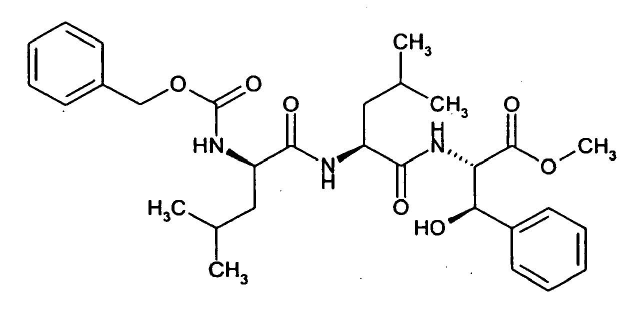

Beispielverbindung 19A (550 mg, 1.45 mmol) und Beispielverbindung 17A (449 mg, 1.45 mmol, 1 Äquivalent) werden in DMF (12 mL) bei 0 °C gelöst. Dann gibt man 4-Methylmorpholin (320 µL, 2.9 mmol, 2 Äquivalente) und HATU (663 mg, 1.74 mmol, 1.2 Äquivalente) dazu und rührt 15 min bei 0 °C. Anschließend wird weiteres 4-Methylmorpholin (160 µL, 1.45 mmol, 1 Äquivalent) dazugegeben und 16 h lang bei RT gerührt. Danach wird zwischen Ethylacetat und konz. Natriumhydrogencarbonat ausgeschüttelt, die organische Phase mit 0.5 M Citronensäure und nochmals mit konz. Natriumhydrogencarbonat gewaschen, über Natriumsulfat getrocknet und eingeengt. Der Rückstand wird chromatographisch gereinigt (Methode 17). Produkthaltige Fraktionen werden vereinigt und lyophilisiert. Ausbeute: 626 mg (1.13 mmol, 78 % d. Th.) der Titelverbindung.

HPLC (Methode 3): Rₜ = 4.69 min.

LC-MS (Methode 8): Rₜ = 2.58 min, MS (ESIpos): m/z (%) = 556.5 (100) [M+H]⁺.

¹H NMR (300 MHz, d₆-DMSO) δ (ppm) = 0.76-0.88 (m, 12 H), 1.23-1.60 (m, 6 H), 3.54 (s, 3 H, OC*H*₃), 4.06-4.11 (m, 1 H), 4.43 (dd, *J* = 8.3, *J* = 14.9 Hz, 1 H), 4.52 (dd, *J* = 4.1, *J* = 7.7 Hz, 1 H), 5.02-5.06 (m, 3 H), 5.87 (d, *J* = 4.5 Hz, 1 H), 7.20-7.40 (m, 11 H), 8.01 (d, *J* = 8.7 Hz, 1 H), 8.08 (d, *J* = 8.5 Hz, 1 H).

### Beispielverbindung 21A: [N²-(Benzyloxy)carbonyl-D-leucyl]-L-leucyl-(3R)- 3-hydroxy-phenylalanin

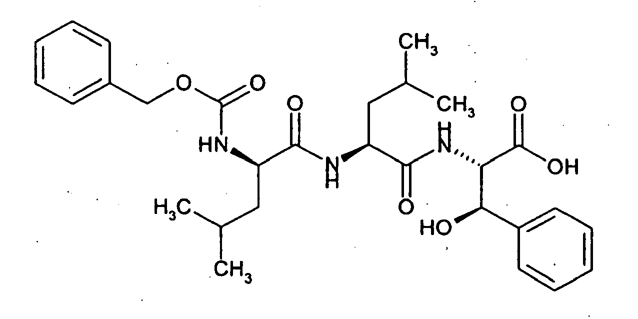

Beispielverbindung 20A (650 mg, 1.17 mmol) wird unter Argon in THF-Wasser (2+1, 30 mL) gelöst. Bei 0 °C wird eine wässrige Lösung von Lithiumhydroxid (57 mg, 2.40 mmol, 4 Äquivalente in 8.65 mL Wasser) zugetropft. Nach 45 min ist die Umsetzung vollständig abgelaufen (HPLC, Methode 1). Es wird mit Eisessig versetzt und eingeengt. Das Rohprodukt wird chromatographisch gereinigt (Methode 16). Produkthaltige Fraktionen werden vereinigt und lyophilisiert. Ausbeute: 618 mg (98 % d. Th.) der Titelverbindung.

HPLC (Methode 1): Rₜ = 2.44 min.

LC-MS (Methode 13) Rₜ = 6.04; MS (ESIpos) m/z (%) = 542.5 (100) [M+H]⁺, 183.8 (80) [2M+H]⁺; MS (ESIneg) m/z (%) = 540.4 (40) [M-H]⁻; 1081.70 (100) [2M-H]⁻.

### Beispielverbindung 22A: 2-(Trimethylsilyl)ethyl-N²-[Benzyloxycarbonyl-D-leucyl]-L-leucyl-(3R)-3-hydroxy-L-phenylalaninat

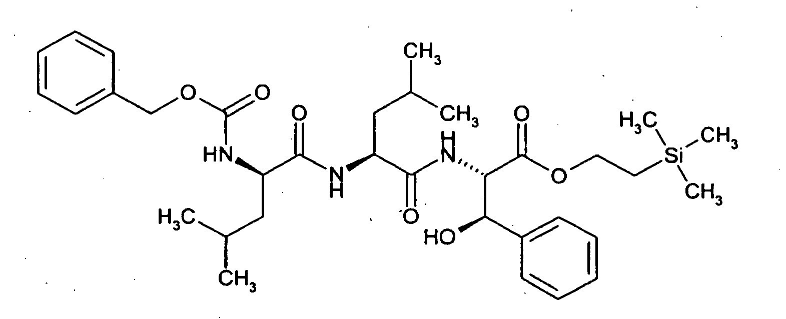

Beispielverbindung 21A (150 mg, 277 µmol) und 2-(Trimethylsilyl)ethanol (790 µL, 5.54 mmol, 20 Äquivalente) und etwas Molekularsieb 4 Å werden in trockenem Dichlormethan (3.0 mL) gelöst und ca. 1 h bei -30 °C gerührt. Dann werden DCC (114 mg, 553 mol, 2 Äquivalente) und DMAP (34 mg, 277 µmol, 1 Äquivalent) zugegeben und der Ansatz über Nacht gerührt und dabei langsam auf Raumtemperatur kommen gelassen. Dann wird der Ansatz im Vakuum eingeengt und chromatographiert (Methode 16). Produkthaltige Fraktionen werden vereinigt und lyophilisiert. Ausbeute: 108 mg, 60 % d. Th.) der Titelverbindung.

HPLC (Methode 1): Rₜ = 3.14 min.

LC-MS (Methode 10): Rₜ = 2.97 min, MS (ESIpos): m/z (%) = 642.3 (100) [M+H]⁺.

### Beispielverbindung 23A: 2-(Trimethylsilyl)ethyl N²-[(Benzyloxy)carbonyl]-D-leucyl-L-leucyl-(3R)-3-{[N²-(tert-butoxycarbonyl)-O³-(tert-butyl)-L-seryl]oxy}-L-phenylalaninat

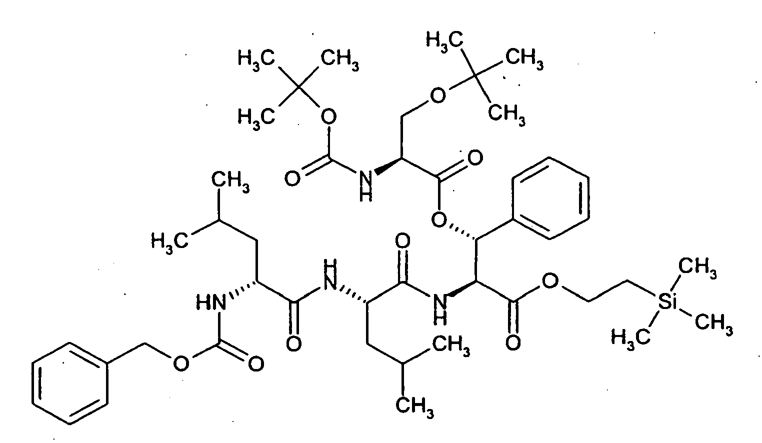

Beispielverbindung 22A (104 mg, 162 µmol) und *N*²-(*tert*-Butoxycarbonyl)-O³-*tert-*butyl-L-serin (47 mg, 178 µmol, 1.1 Äquivalente) werden in trockenem Dichlormethan (2.0 mL) gelöst und etwas Molekularsieb 4 Å dazugegeben. Dann werden DCC (70 mg, 340 mol, 2.1 Äquivalente) und DMAP (23 mg, 194 µmol, 1.2 Äquivalente) zugegeben und der Ansatz über Nacht gerührt und dabei langsam auf Raumtemperatur kommen gelassen. Dann wird der Ansatz im Vakuum eingeengt und chromatographiert (Methode 16). Produkthaltige Fraktionen werden vereinigt und lyophilisiert. Ausbeute: 120 mg (84 % d. Th.) der Titelverbindung.

HPLC (Methode 1): Rₜ = 3.49 min.

LC-MS (Methode 10): Rₜ = 3.38 min, MS (ESIpos): m/z (%) = 885.6 (100) [M+H]⁺.

HR-TOF-MS (Methode 14): C₄₆H₇₃N₄O₁₁Si ber. 885.5040, gef. 885.5031 [M+H]⁺.

### Beispielverbindung 24A: 2-(Trimethylsilyl)ethyl N²-[(Benzyloxy)carbonyl]-D-leucyl-L-leucyl-(3R)-3-{[O³-(tert-butyl)-L-seryl]oxy}-L-phenylalaninat-trifluoracetat

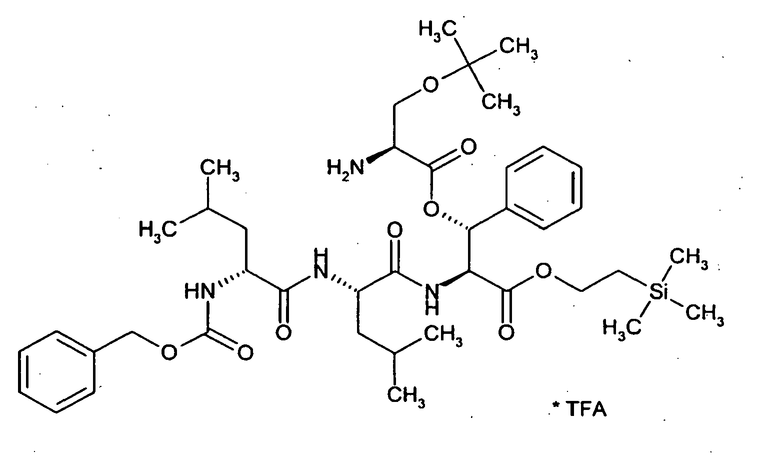

Beispielverbindung 23A (117 mg, 132 µmol) wird in Dichlormethan (3 mL) gelöst. 15 % TFA in Dichlormethan (20 mL) wird zugesetzt, nach 10 min wird zur Trockne eingeengt. Der Rückstand wird chromatographisch gereinigt (Methode 16). Produkthaltige Fraktionen werden vereinigt und lyophilisiert. Ausbeute: 100 mg (83 % d. Th.).

HPLC (Methode 1): Rₜ = 2.40 min.

LC-MS (Methode 10): Rₜ = 2.28 min, MS (ESIpos): m/z (%) = 785.4 (100) [M+H]⁺.

### Beispielverbindung 25A: 2-(Trimethylsilyl)ethyl N²-[(Benzyloxy)carbonyl]-D-leucyl-L-leucyl-(3R)-3-{[N²-(tert-butoxycarbonyl)glycyl-(3S)-3-hydroxy-L-asparaginyl-O³-(tert-butyl)seryl]oxy}-L-phenylalaninat

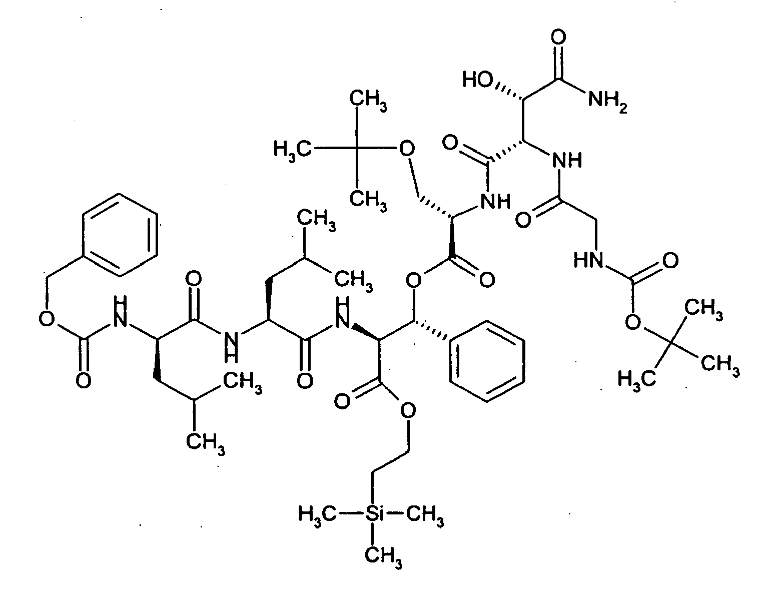

Beispielverbindung 24A (96 mg, 107 µmol) und Beispielverbindung 13A (33 mg, 107 µmol, 1 Äquivalent) werden in DMF (2.0 mL) gelöst und auf -30 °C gekühlt. HATU (122 mg, 320 µmol, 3 Äquivalente) und 4-Methylmorpholin (86 mg, 854 µmol, 8 Äquivalente) werden zugegeben, dann wird der Ansatz langsam auf ca. 4 °C erwärmen gelassen und über 12 h bei dieser Temperatur stehengelassen. Die rohe Reaktionslösung wird chromatographiert (Methode 15), produkthaltige Fraktionen werden vereinigt und lyophilisiert. Ausbeute: 92 mg (89 % d. Th.) der Titelverbindung.

HPLC (Methode 1): Rₜ = 3.22 min.

LC-MS (Methode 9): Rₜ = 3.21 min, MS (ESIpos): m/z (%) = 1072.6 (100) [M+H]⁺; MS (ESIneg): m/z (%) = 1070.5 (100) [M-H].

HR-TOF-MS (Methode 14): C₅₂H₈₂N₇O₁₅Si ber. 1072.5633, gef. 1072.5667 [M+H]⁺.

### Beispielverbindung 26A: 2-(Trimethylsilyl)ethyl N²-[(Benzyloxy)carbonyl]-D-leucyl-L-leucyl-(3R)-3-{[glycyl-(3S)-3-hydroxy-L-asparaginyl-O³-(tert-butyl)seryl]oxy}-L-phenylalaninat-trifluoracetat

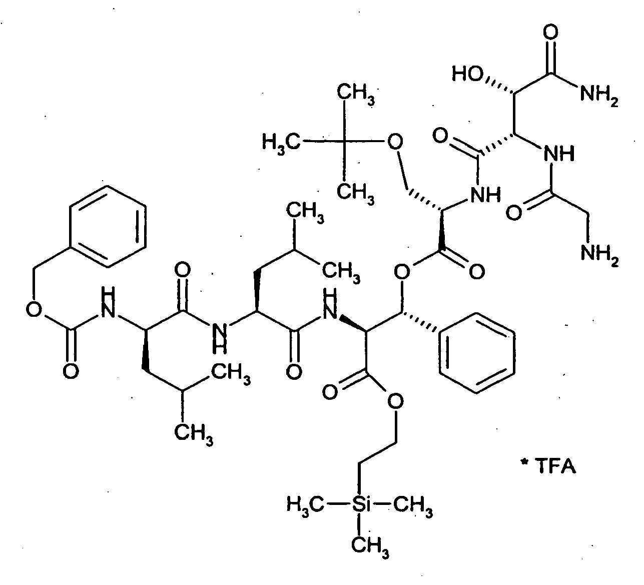

Beispielverbindung 25A (90 mg, 84 µmol) wird in Dichlormethan (3.0 mL) gelöst. 15 % TFA in Dichlormethan (20 mL) wird zugesetzt, nach 10 min wird zur Trockne eingeengt. Der Rückstand wird chromatographisch gereinigt (Methode 16). Produkthaltige Fraktionen werden vereinigt und lyophilisiert. Ausbeute: 73 mg (80 % d. Th.) der Titelverbindung.

HPLC (Methode 1): Rₜ = 2.65 min.

LC-MS (Methode 10): Rₜ = 2.13 min, MS (ESIpos): m/z (%) = 972.6 (100) [M+H]⁺; MS (ESIneg): m/z (%) = 970.7 (100) [M-H].

HR-TOF-MS (Methode 14): C₄₇H₇₄N₇O₁₃Si ber. 972.5109, gef. 972.5103 [M+H]⁺.

### Beispielverbindung 27A: 2-(Trimethylsilyl)ethyl N²-[(Benzyloxy)carbonyl]-D-leucyl-L-leucyl-(3R)-3-{[N²-(tert-butoxycarbonyl)-(3R)-3-hydroxy-L-leucyl-L-leucyl-D-arginyl-L-isoleucyl-L-allothreonyl-glycyl-(3S)-3-hydroxy-L-asparaginyl-O³-(tert-butyl)seryl]oxy}-L-phenylalaninat-trifluoracetat

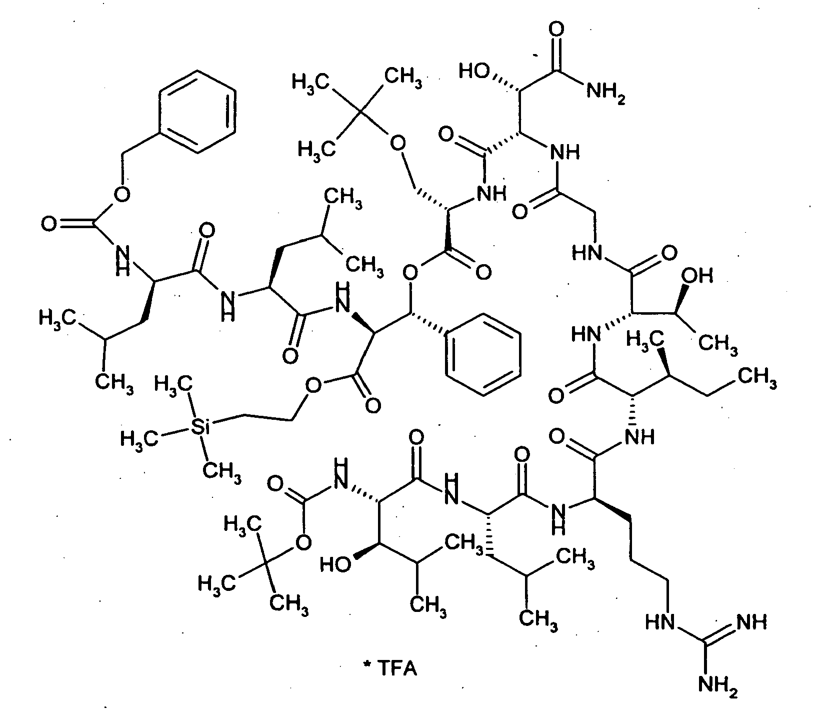

Beispielverbindung 26A (10.0 mg, 9.2 µmol) und Beispielverbindung 11A (8.2 mg, 107 µmol, 1 Äquivalent) werden in DMF (0.5 mL) gelöst und auf -30 °C gekühlt. HATU (10.5 mg, 27.6 µmol, 3 Äquivalente) und 4-Methylmorpholin (7.5 mg, 74 µmol, 8 Äquivalente) werden zugegeben, dann wird der Ansatz langsam auf ca. 4 °C erwärmen gelassen und über 12 h bei dieser Temperatur stehen gelassen. Die rohe Reaktionslösung wird chromatographiert (Methode 15), produkthaltige Fraktionen werden vereinigt und lyophilisiert. Ausbeute: 10.7 mg (65 % d. Th.) der Titelverbindung.

HPLC (Methode 1): Rₜ = 2.99 min.

LC-MS (Methode 9): Rₜ = 2.40 min, MS (ESIpos): m/z (%) = 1685.8 (50) [M+H]⁺; MS (ESIneg): m/z (%) = 1683.8 (50) [M-H], 1728.8 (100) [M+HCOOH].

HR-TOF-MS (Methode 14): C₈₀H₁₃₄N₁₅O₂₂Si ber. 1684.9592, gef. 1684.9573 [M+H]⁺.

### Beispielverbindung 28A: N²-[(Benzyloxy)carbonyl]-D-leucyl-L-leucyl-(3R)-3-{[N²-(tert-butoxycarbonyl)-(3R)-3-hydroxy-L-leucyl-L-leucyl-D-arginyl-L-isoleucyl-L-allothreonyl-glycyl-(3S)-3-hydroxy-L-asparaginyl-O³-(tert-butyl)seryl]oxy}-L-phenylalanin-trifluoracetat

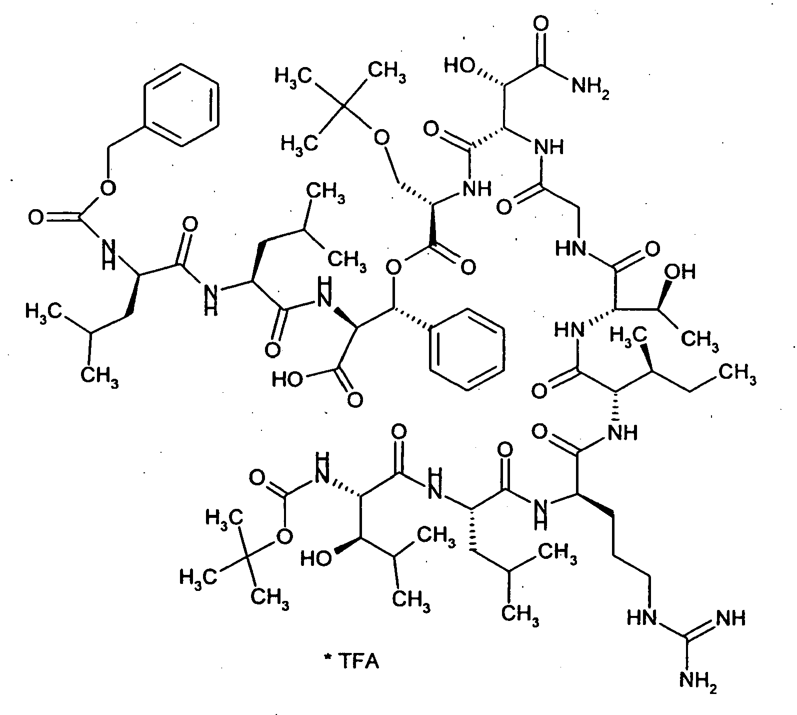

### Methode A:

Beispielverbindung 27A (10 mg, 5.6 µmol) wird in abs. THF (0.5 mL) gelöst. TBAF (17.4 mg, 67 µmol, 12 Äquivalente) wird zugegeben und der Ansatz wird 1 h bei RT gerührt. Nach HPLC-Analytik (Methode 1) ist die Umsetzung vollständig, es wird mit Eisessig (6 µL) abgestoppt, eingeengt und chromatographiert (Methode 15). Produkthaltige Fraktionen werden vereinigt und lyophilisiert. Ausbeute: 2.5 mg (ca. 69 % rein, 18 % d. Th.) der Titelverbindung.

### Methode B:

Verbindung 27A (25 mg, 13.9 µmol) wird in abs. THF (2.5 ml) gelöst. Natriumsulfat (wasserfrei, 200 mg, 1,4 mmol) wird zugegeben und die Suspension 30 min gerührt. Es wird TBAF-Lösung (1M wasserfrei in THF, 84µl, 6 Äquivalente) zugeben und 45 Minuten bei RT gerührt. Nach HPLC-Analytik (Methode 1) ist die Umsetzung vollständig. Es wird mit Eisessig (16 µl) abgestoppt, filtriert, eingeengt und chromatographiert (Methode 15). Ausbeute: 21 mg (>95% rein, 89 % d. Th.) der Titelverbindung.

HPLC (Methode 6): Rₜ = 2.99 min.

LC-MS (Methode 9): Rₜ = 2.34 min, MS (ESIpos): m/z (%) = 1585.6 (20) [M+H]⁺; MS (ESIneg): m/z (%) = 1583.4 (100) [M-H]⁻.

HR-TOF-MS (Methode 14): C₇₅H₁₂N₁₅O₂₃ ber. 1584.8884, gef. 1584.8843 [M+H]⁺.

### Beispielverbindung 29A: N²-[(Benzyloxy)carbonyl]-D-leucyl-L-leucyl-(3R)-3-{[(3R)-3-hydroxy-L-leucyl-L-leucyl-D-arginyl-L-isoleucyl-L-allothreonyl-glycyl-(3S)-3-hydroxyL-asparaginyl-seryl]oxy}-L-phenylalanin-bis-trifluoracetat

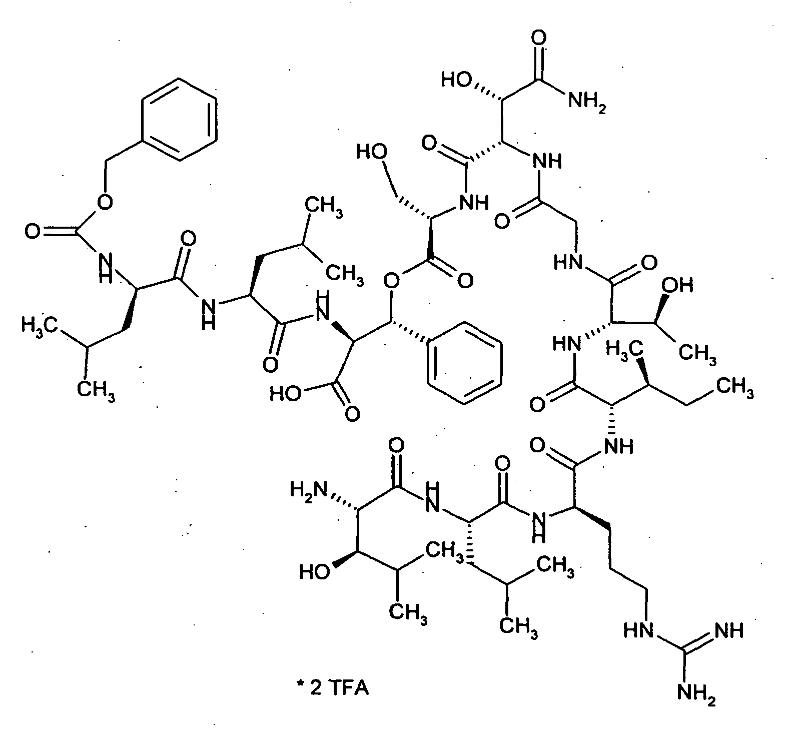

Beispielverbindung 28A (2.5 mg, 1.5 µmol) wird mit Triisopropylsilan (12.5 µL) und Wasser (2.8 µL) umgesetzt und 0.5 mL TFA versetzt. Dann wird 1 h bei Raumtemperatur gerührt und schließlich das Lösemittel im Vakuum abgezogen. Der Rückstand wird chromatographiert (Methode 15). Produkthaltige Fraktionen werden vereinigt und lyophilisiert. Ausbeute: 2 mg (82 % d. Th.) der Titelverbindung.

HPLC (Methode 6): Rₜ = 2.00 min.

LC-MS (Methode 9): Rₜ = 1.60 min, MS (ESIpos): m/z (%) = 714.9 (100) [M+2H]²⁺; MS (ESIneg) m/z (%) = 1427.7 (100) [M-H]⁻.

HR-TOF-MS (Methode 14): C₆₆H₁₀₆N₁₅O₂₀ ber. 1428.7734, gef. 1428.7700 [M+H]⁺.

### Beispielverbindung 30A: N-Benzyloxycarbonyl-lysobactin-trifluoracetat

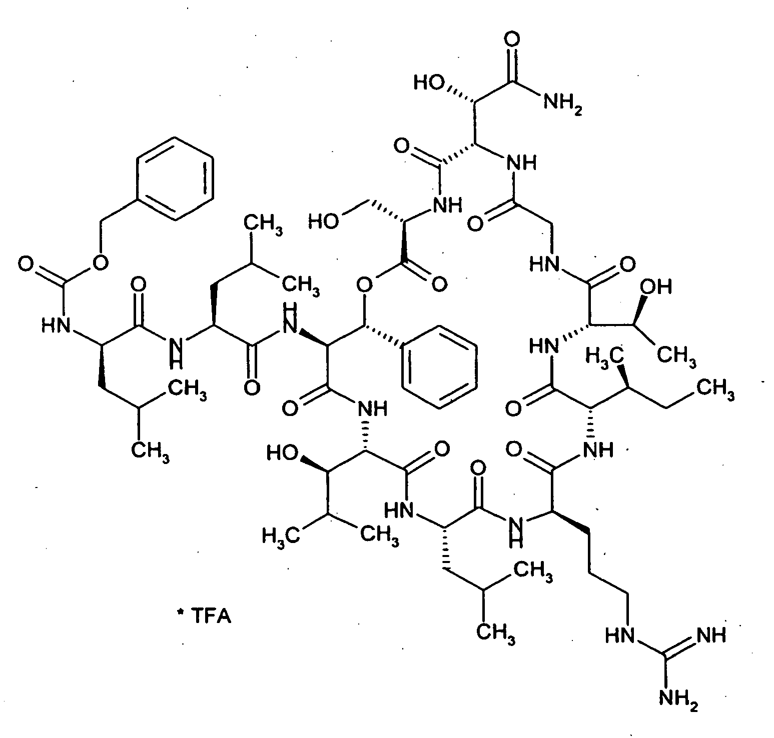

Beispielverbindung 29A (1.0 mg, 1.1 µmol) wird in DMF (0.9 mL) gelöst und auf -15 °C gekühlt. HATU (1.2 mg, 3.3 µmol, 3 Äquivalente) und 4-Methylmorpholin (11 µL einer Lösung von 100 µL 4-Methylmorpholin in 0.9 mL DMF, 8.7 µmol, 8 Äquivalente) werden zugegeben, dann wird der Ansatz langsam auf ca. 4 °C erwärmen gelassen und über 3 h bei Raumtemperatur gerührt. Die rohe Reaktionslösung wird chromatographiert (Methode 15), produkthaltige Fraktionen werden vereinigt und lyophilisiert. Ausbeute: 1.2 mg (73 % d. Th.) der Titelverbindung.

HPLC (Methode 1): Rₜ = 2.17 min.

LC-MS (Methode 9): Rₜ = 2.00 min, MS (ESIpos): m/z (%) = 1410.8 (100) [M+H]⁺; MS (ESIneg) m/z (%) = 1408.7 (100) [M-H].

HR-TOF-MS (Methode 14): C₆₆H₁₀₄N₁₅O₁₉ ber. 1410.7628, gef. 1410.7639 [M+H]⁺.

### Beispielverbindung 31A: Lysobactin-bis-trifluoracetat

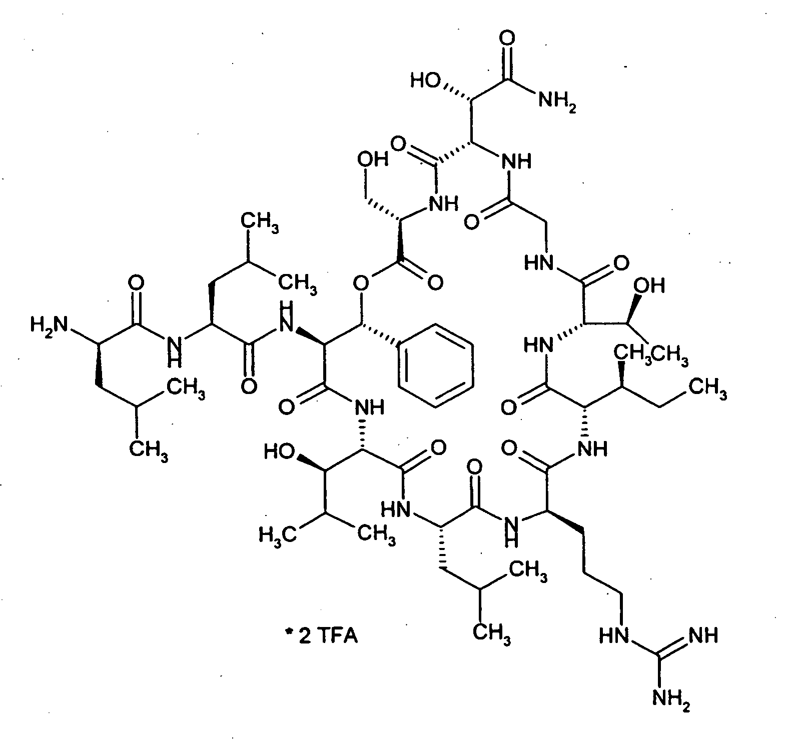

Beispielverbindung 30A (1.0 mg, 0.66 µmol) wird in Dioxan (0.5 mL) gelöst, 0.75 mL 0.1 % wässr. TFA und eine Spatelspitze 10 % Pd/C werden zugegeben und der Ansatz 15 min bei RT unter Normaldruck hydriert. Das Produkt wird vom Katalysator abfiltriert, eingeengt und chromatographisch gereinigt (Methode 15). Ausbeute: 0.6 mg (61 % d. Th.) der Titelverbindung.

HPLC (Methode 4): Rₜ = 16.31 min. Die Identität des Syntheseproduktes 31A wurde durch Koinjektion mit authentischem Lysobactin (Erhalten nach der in WO 2004/099239(A1) beschriebenen Methode) bestätigt.

HPLC (Methode 5) Rₜ = 38.10 min. Die Identität des Syntheseproduktes 31A wurde durch Koinjektion mit authentischem Lysobactin (Erhalten nach der in WO 2004/099239(A1) beschriebenen Methode) bestätigt.

LC-MS (Methode 9): Rₜ = 1.40 min, MS (ESIpos): m/z (%) = 638.9 (100) [M+2H]²⁺, 1276.8 (5) [M+H]⁺; MS (ESIneg): m/z (%) = 637.0 (100) [M-2H]²⁻, 1274.7 (40) [M-H]⁻.

HR-TOF-MS (Methode 14): C₅₈H₉₈N₁₅O₁₇ ber. 1276.7260, gef. 1276.7264 [M+H]⁺.

### B. Bewertung der physiologischen Wirksamkeit

Die *in vitro*-Wirkung der erfindungsgemäßen Verbindungen kann in folgendem Assay gezeigt werden:

### Bestimmung der minimalen Hemmkonzentration (MHK):

Die MHK wird im Flüssigdilutionstest gemäß der NCCLS-Richtlinien bestimmt. Übernachtkulturen von *Staphylococcus aureus* 133, *Entercococcus faecalis* ICB27159 und *Streptococcus pneumoniae* G9a werden mit den beschriebenen Testsubstanzen in einer 1:2 Verdünnungsreihe inkubiert. Die MHK-Bestimmung wird mit einer Zellzahl von 10⁵ Keimen pro ml in Isosensitest-Medium (Fa. Difco, Irvine/USA) durchgeführt, mit Ausnahme von *S. pneumoniae,* der in BHI-Bouillon (Fa. Difco, Irvine/USA) mit 10% Rinderserum bei einer Zellzahl von 10⁶ Keimen pro ml getestet wird. Die Kulturen werden bei 37°C für 18-24 Stunden inkubiert, *S. pneumoniae* in Gegenwart von 10% CO₂.

Die jeweils niedrigste Substanzkonzentration, bei der kein sichtbares Bakterienwachstum mehr auftritt, wird als MHK definiert. Die MHK-Werte werden in µg/ml angegeben.

Es ergaben sich keine signifikanten Unterschiede in der physiologischen Wirksamkeit zwischen vollsynthetisch hergestelltem und fermentiertem Lysobactin.

## Patentansprüche

1. Verfahren zum Herstellen von cyclischen Depsipeptiden der folgenden Formel (1) 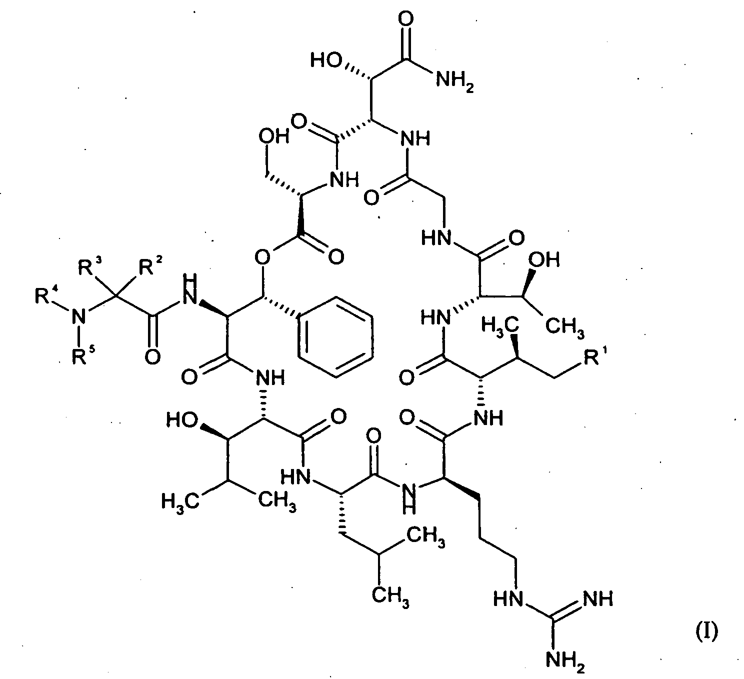
in der R¹ = H oder CH₃ ist,
in der R² = Wasserstoff, C₃-C₆-Cycloalkyl, C₅-C₆-Cycloalkenyl, C₃-C₆ Cycloalkylmethyl, 5- bis 7-gliedriges Heterocyclylmethyl, Methyl, Ethyl, n-Propyl, iso-Propyl, 1-Methylprop-1-yl, 2-Methylprop-1-yl, 2,2-Dimethylprop-1-yl, 1,1-Dimethylprop-1-yl, 1-Ethyl-prop-1-yl, 1-Ethyl-1-methylprop-1-yl, n-Butyl, 2-Methylbut-1-yl, 3-Methylbut-1-yl, 1-Ethylbut-1-yl, *tert*-Butyl, 4-Methylpent-1-yl, n-Hexyl, Alkenyl oder Aryl ist,
wobei R² substituiert sein kann mit 0, 1, 2 oder 3 Substituenten unabhängig voneinander ausgewählt aus der Gruppe bestehend aus Halogen, Hydroxy, Amino, Cyano, Trimethylsilyl, Alkyl, Alkoxy, Benzyloxy, C₃-C₆-Cycloalkyl, Aryl, 5- bis 10-gliedriges Heteroaryl, Alkylamino, Arylamino, Alkylcarbonylamino, Arylcarbonylamino, Alkylcarbonyl, Alkoxycarbonyl, Arylcarbonyl und Benzyloxycarbonylamino,
worin Aryl und Heteroaryl ihrerseits substituiert sein können mit 0, 1, 2 oder 3 Substituenten unabhängig voneinander ausgewählt aus der Gruppe bestehend aus Halogen, Hydroxy, Amino, Cyano, Nitro, Alkyl, Alkoxy und Phenyl,
in der R³ = Wasserstoff oder C₁-C₄-Alkyl ist,
oder
in der R² und R³ zusammen mit dem Kohlenstoffatom, an das sie gebunden sind, einen C₃-C₆-Cycloalkyl-Ring oder einen 5- bis 7-gliedrigen Heterocyclyl-Ring bilden, wobei der Cycloalkyl-Ring und der Heterocyclyl-Ring substituiert sein können mit 0, 1, 2 oder 3 Substituenten unabhängig voneinander ausgewählt aus der Gruppe bestehend aus Trifluormethyl, Alkyl, Alkoxy und Alkylcarbonyl,
in der R⁴ = Alkyl, C₃-C₆-Cycloalkyl, 5- bis 7-gliedriges Heterocyclyl, Aryl, 5-oder 6-gliedriges Heteroaryl, Alkylcarbonyl, Alkoxycarbonyl, C₃-C₆-Cycloalkylcarbonyl, 5- bis 7-gliedriges Heterocyclylcarbonyl, Arylcarbonyl, 5- oder 6-gliedriges Heteroarylcarbonyl oder Alkylaminocarbonyl ist,
wobei Alkyl, Cycloalkyl, Heterocyclyl, Aryl, Heteroaryl, Alkoxycarbonyl, Cycloalkylcarbonyl, Heterocyclylcarbonyl, Arylcarbonyl, Heteroarylcarbonyl und Alkylaminocarbonyl substituiert sein können mit 0, 1, 2 oder 3 Substituenten unabhängig voneinander ausgewählt aus der Gruppe bestehend aus Halogen, Hydroxy, Amino, Alkylamino und Phenyl,
und
wobei Alkylcarbonyl substituiert ist mit einem Substituenten Amino oder Alkylamino,
und
wobei Alkylcarbonyl substituiert sein kann mit weiteren 0, 1 oder 2 Substituenten unabhängig voneinander ausgewählt aus der Gruppe bestehend aus Halogen, Hydroxy, Trimethylsilyl, Alkoxy, Alkylthio, Benzyloxy, C₃-C₆-Cycloalkyl, Phenyl, Naphthyl, 5- bis 10-gliedriges Heteroaryl, Alkylcarbonylamino, Alkoxycarbonylamino, Arylcarbonylamino, Arylcarbonyloxy, Benzyloxycarbonyl und Benzyloxycarbonylamino,
wobei Phenyl und Heteroaryl ihrerseits substituiert sein können mit 0, 1, 2 oder 3 Substituenten unabhängig voneinander ausgewählt aus der Gruppe bestehend aus Halogen, Hydroxy,Nitro, Alkyl, Alkoxy und Phenyl,
oder zwei Substituenten an demselben Kohlenstoffatom im Alkylcarbonyl zusammen mit dem Kohlenstoffatom, an das sie gebunden sind, einen C₃-C₆-Cycloalkyl-Ring oder einen 5- bis 7-gliedrigen Heterocyclyl-Ring bilden,
wobei der Cycloalkyl-Ring und der Heterocyclyl-Ring substituiert sein können mit 0, 1, 2 oder 3 Substituenten unabhängig voneinander ausgewählt aus der Gruppe bestehend aus Trifluormethyl, Alkyl und Alkoxy,
oder wobei der Cycloalkyl-Ring Benzo-anneliert sein kann,
in der R⁵ = Wasserstoff, C₁-C₄-Alkyl, Cyclopropyl oder Cyclopropylmethyl ist,
oder
in der R⁴ und R⁵ zusammen mit dem Stickstoffatom, an das sie gebunden sind, einen 5- bis 7-gliedrigen Heterocyclyl-Ring bilden, wobei der Heterocyclyl-Ring substituiert sein kann mit 0, 1, 2 oder 3 Substituenten unabhängig voneinander ausgewählt aus der Gruppe bestehend aus Halogen, Hydroxy, Amino, Cyano, Alkyl, Alkoxy und Alkylamino,
durch intramolekulare Cyclisierung einer Verbindung der folgenden Formel (II) 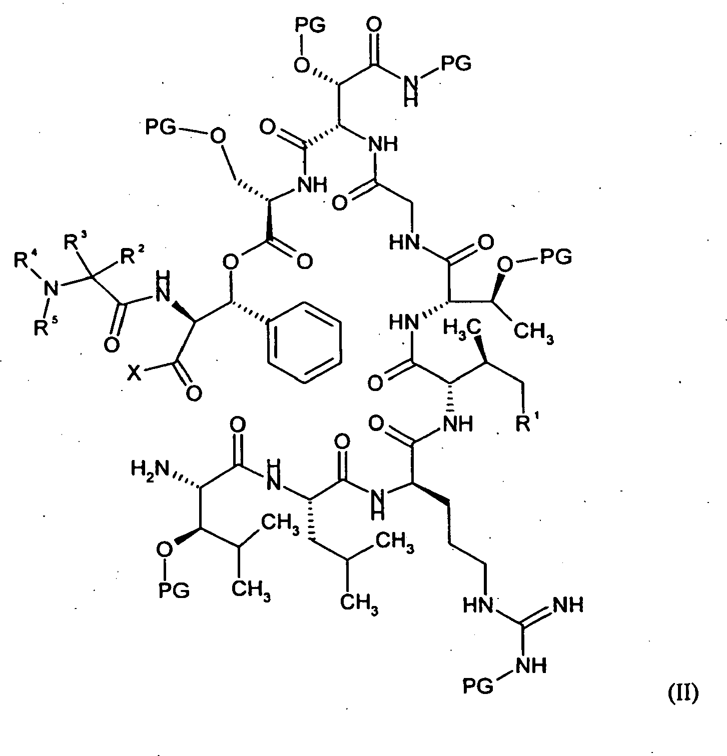
in der R¹ bis R⁵ wie zuvor definiert sind,
in der X = OH, ein Aktivester, ein Pseudohalogen oder ein Halogen ist, und
in der PG = H oder eine geeignete Schutzgruppe ist,
und anschließendes Entschützen des cyclischen Zwischenprodukts unter Bildung des cyclischen Depsipeptids der Formel (I).

2. Verfahren nach Anspruch 1, **dadurch gekennzeichnet, dass** die Verbindung der Formel (II) eine Verbindung der folgenden Formel (IIa) ist 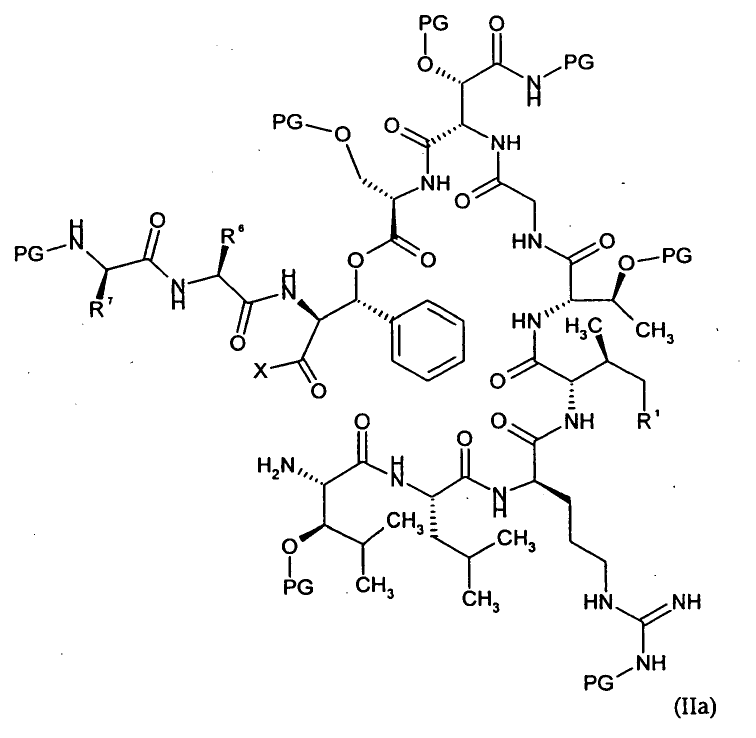
in der X und R¹ wie in Anspruch 1 definiert sind, in der R⁶ = Isopropylmethyl, *tert*-Butylmethyl, 2,2-Dimethylbut-1-yl, 2-Ethyl-2-methylbut-1-yl, 2,2-Diethylbut-1-yl, 2,2-Dimethylpent-1-yl, 3-Pyridylmethyl, 4-Trifluormethyl-3-pyridylmethyl, Benzyl oder Trimethylsilylmethyl ist,
in der R⁷ = Isopropylmethyl, *tert*-Butylmethyl, 2,2-Dimethylbut-1-yl, 2-Ethyl-2-methylbut-1-yl, 2,2-Diethylbut-1-yl, 2,2-Dimethylpent-1-yl, Trimethylsilylmethyl oder Benzyl ist, und
in der PG = H oder eine geeignete Schutzgruppe ist.

3. Verfahren nach Anspruch 2, **dadurch gekennzeichnet, dass**
R⁶ = Isopropylmethyl, *tert*-Butylmethyl oder 3-Pyridylmethyl ist, und
R⁷ = Isopropylmethyl, *tert*-Butylmethyl oder Trimethylsilylmethyl ist.

4. Verfahren nach Anspruch 3, **dadurch gekennzeichnet, dass** R⁶ = R⁷ = Isopropylmethyl ist.

5. Verfahren nach einem der Ansprüche 1 bis 4, **dadurch gekennzeichnet, dass** X = OH ist.

6. Verfahren nach einem der Ansprüche 1 bis 5, **dadurch gekennzeichnet, dass** R¹ = CH₃ ist.

7. Verfahren nach Anspruch 1 **gekennzeichnet durch** das Herstellen der Verbindung der Formel (II) **durch** Kuppeln einer Verbindung der folgenden Formel (III) mit einer Verbindung der folgenden Formel (IV) 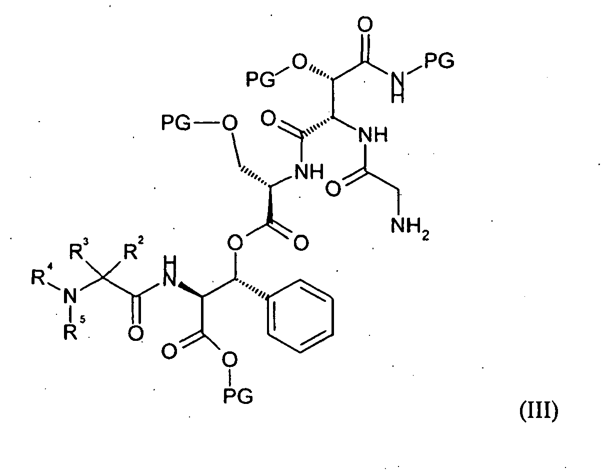 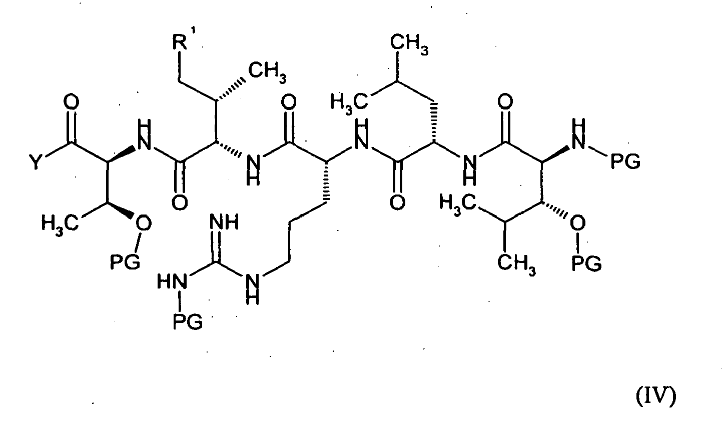
in denen R¹ bis R⁵ wie in Anspruch 1 definiert sind,
in denen Y = OH, ein Aktivester, ein Pseudohalogen oder ein Halogen ist, und
in denen PG = H oder eine geeignete Schutzgruppe ist,
und ggf. teilweises oder vollständiges Entschützen des Zwischenprodukts, sowie ggf. Umwandeln der Carboxylgruppe des 3-Hydroxy-phenylalanins in eine Gruppe der Formel -C(=O)X, in der X wie in Anspruch 1 definiert ist.

8. Verfahren nach Anspruch 7, **dadurch gekennzeichnet, dass** die Verbindung der Formel (III) eine Verbindung der folgenden Formel (IIIa) ist 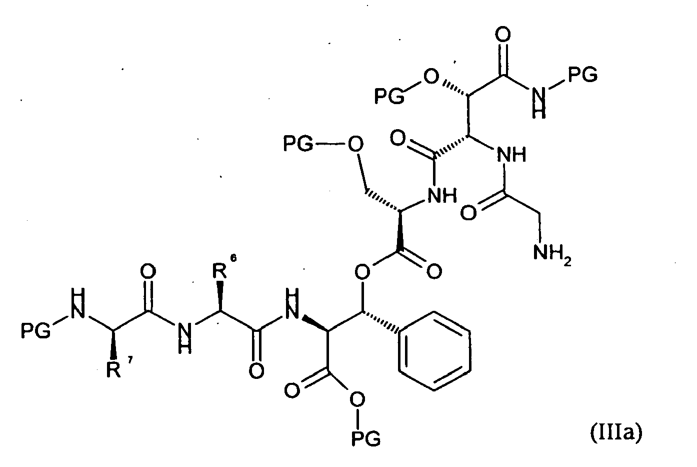
in der R⁶ und R⁷ wie in den Ansprüchen 2 bis 4 definiert sind, und
in der PG = H oder eine geeignete Schutzgruppe ist.

9. Verfahren nach Anspruch 7, **gekennzeichnet durch** das Herstellen der Verbindung der Formel (III) **durch** das Kuppeln einer Verbindung der folgenden Formel (V) mit einer Verbindung der folgenden Formel (VI) 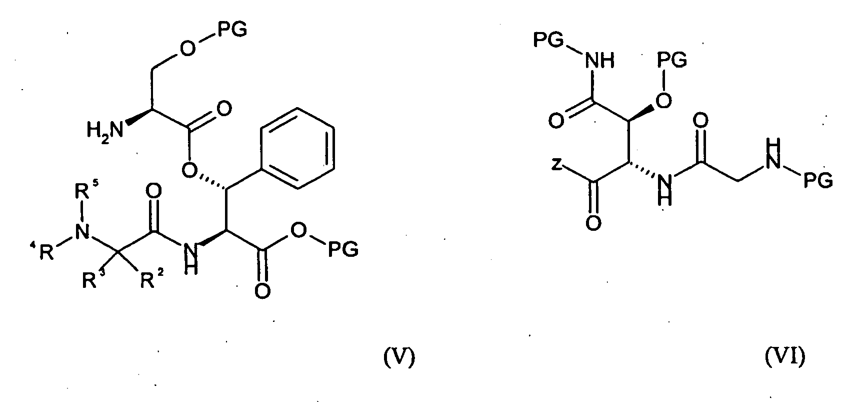
in denen R² bis R⁵ wie in Anspruch 1 definiert sind,
in denen Z = OH, ein Aktivester, ein Pseudohalogen oder ein Halogen ist, und
in denen PG = H oder eine geeignete Schutzgruppe ist,
und ggf. das teilweise oder vollständige Entschützen des Zwischenprodukts.

10. Verfahren nach Anspruch 9, **dadurch gekennzeichnet, dass** die Verbindung der Formel (V) eine Verbindung der folgenden Formel (Va) ist 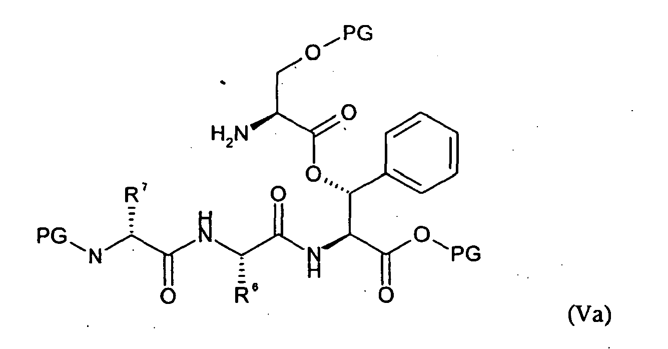
in der R⁶ und R⁷ wie in Ansprüchen 2 bis 4 definiert sind, und
in der PG = H oder eine geeignete Schutzgruppe ist.

11. Verbindung der folgenden Formel (III) 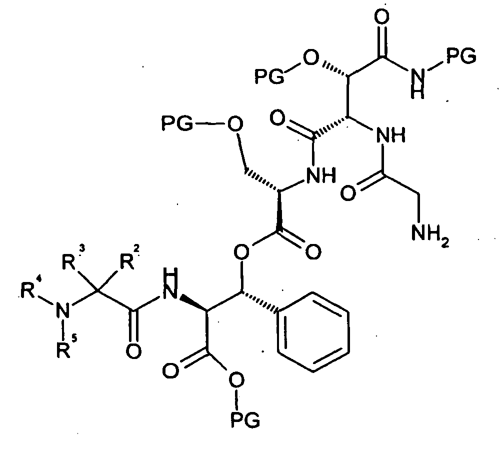
in der R² bis R⁵ wie in Anspruch 1 definiert sind, und
in der PG = H oder eine geeignete Schutzgruppe ist.

12. Verbindung nach Anspruch 11, mit der folgenden Formel (IIIa) 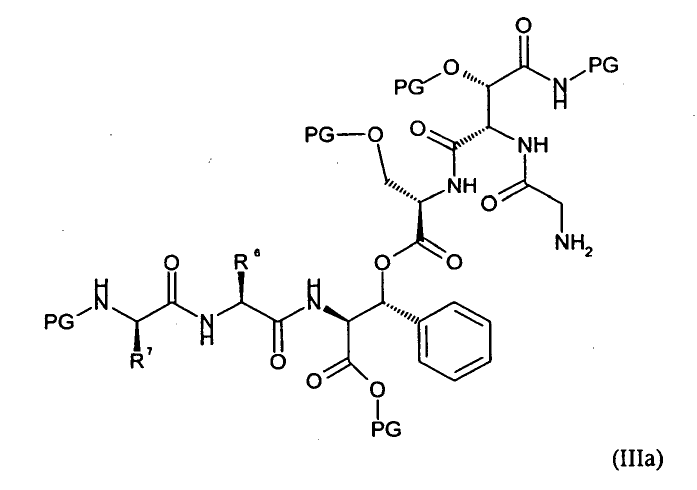
in der R⁶ und R⁷ wie in den Ansprüchen 2 bis 4 definiert sind, und
in der PG = H oder eine geeignete Schutzgruppe ist.

13. Verbindung nach Anspruch 12, mit der folgenden Formel (IIIb)

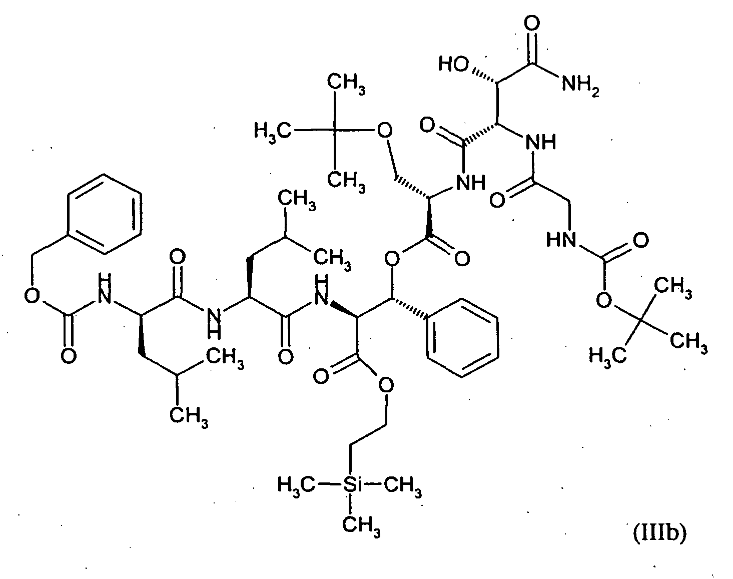

14. Verfahren zum Herstellen einer Verbindung nach Anspruch 11, das das Kuppeln einer Verbindung der Formel (V) mit einer Verbindung der Formel (VI) 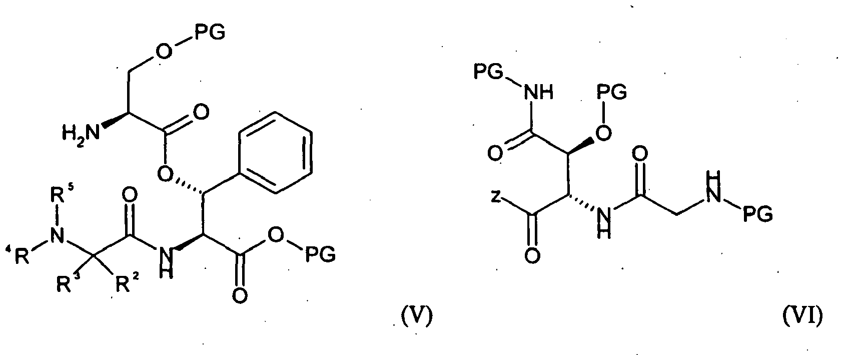
in denen R² bis R⁵ wie in Anspruch 1 definiert sind,
in denen Z = OH, ein Aktivester, ein Pseudohalogen oder ein Halogen ist, und
in denen PG = H oder eine geeignete Schutzgruppe ist,
und ggf. das teilweise oder vollständige Entschützen des Zwischenprodukts aufweist.

15. Verbindung der folgenden Formel (VI) 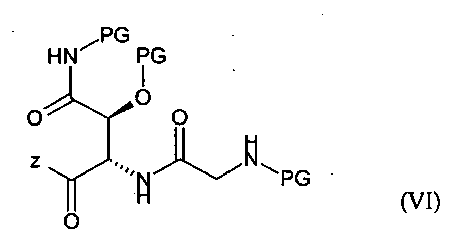
in der Z = OH, ein Aktivester, ein Pseudohalogen oder ein Halogen ist, und
in der PG = H oder eine geeignete Schutzgruppe ist.

16. Verbindung nach Anspruch 15, mit der folgenden Formel (VIa)

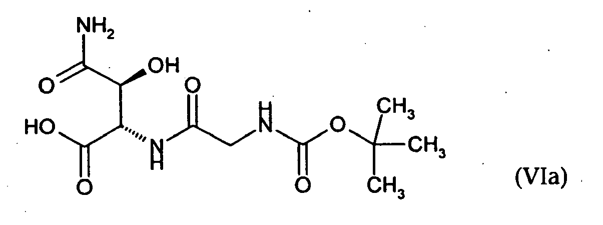

17. Verfahren zum Herstellen einer Verbindung nach Anspruch 15, das das Kuppeln einer Verbindung der folgenden Formel (VII) mit einer Verbindung der folgenden Formel (VIII) 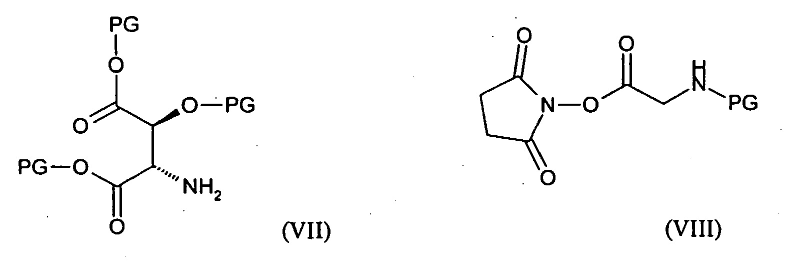
in denen PG = H oder eine geeignete Schutzgruppe ist
und ggf. das vollständige oder teilweise Entschützen des Zwischenprodukts aufweist.

18. Verfahren nach Anspruch 17, **dadurch gekennzeichnet, dass** die Verbindung der Formel (VII) eine Verbindung der folgenden Formel (VIIa) ist.

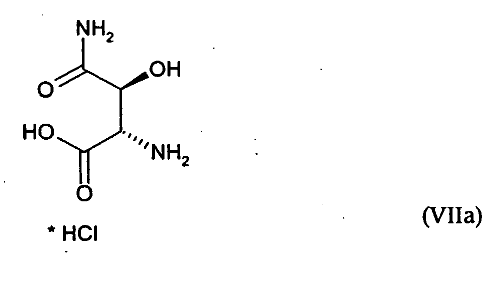

19. Verfahren nach Anspruch 17 oder 18, **dadurch gekennzeichnet, dass** die Verbindung der Formel (VIII) eine Verbindung der folgenden Formel (VIIIa) ist.

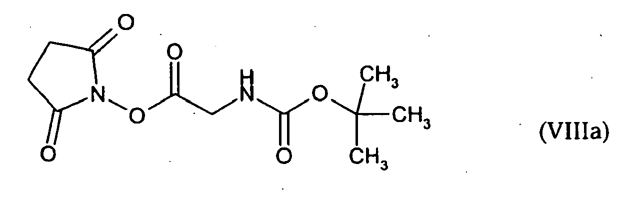

## Claims

1. Method for preparing cyclic depsipeptides of the following formula (I) 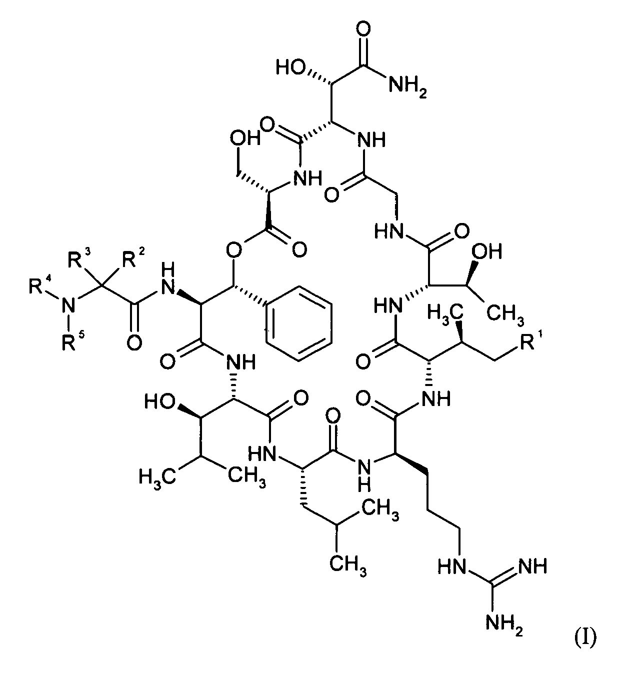
in which R¹ is H or CH₃,
in which R² is hydrogen, C₃-C₆-cycloalkyl, C₅-C₆-cycloalkenyl, C₃-C₆ cycloalkylmethyl, 5- to 7-membered heterocyclylmethyl, methyl, ethyl, n-propyl, isopropyl, 1-methylprop-1-yl, 2-methylprop-1-yl, 2,2-dimethylprop-1-yl, 1,1-dimethylprop-1-yl, 1-ethylprop-1-yl, 1-ethyl-1-methylprop-1-yl, n-butyl, 2-methylbut-1-yl, 3-methylbut-1-yl, 1-ethylbut-1-yl, *tert*-butyl, 4-methylpent-l-yl, n-hexyl, alkenyl or aryl, whereby R² may be substituted with 0, 1, 2 or 3 substituents selected independently of one another from the group consisting of halogen, hydroxy, amino, cyano, trimethylsilyl, alkyl, alkoxy, benzyloxy, C₃-C₆-cycloalkyl, aryl, 5- to 10-membered heteroaryl, alkylamino, arylamino, alkylcarbonylamino, arylcarbonylamino, alkylcarbonyl, alkoxycarbonyl, arylcarbonyl and benzyloxycarbonylamino,
wherein aryl and heteroaryl in turn may be substituted with 0, 1, 2 or 3 substituents selected independently of one another from the group consisting of halogen, hydroxy, amino, cyano, nitro, alkyl, alkoxy and phenyl,
in which R³ is hydrogen or C₁-C₄-alkyl,
or
in which R² and R³ together with the carbon atom to which they are bonded form a C₃-C₆-cycloalkyl ring or a 5- to 7-membered heterocyclyl ring, whereby the cycloalkyl ring and the heterocyclyl ring may be substituted with 0, 1, 2 or 3 substituents selected independently of one another from the group consisting of trifluoromethyl, alkyl, alkoxy and alkylcarbonyl,
in which R⁴ is alkyl, C₃-C₆-cycloalkyl, 5- to 7-membered heterocyclyl, aryl, 5-or 6-membered heteroaryl, alkylcarbonyl, alkoxycarbonyl, C₃-C₆-cycloalkylcarbonyl, 5- to 7-membered heterocyclylcarbonyl, arylcarbonyl, 5- or 6-membered heteroarylcarbonyl or alkylaminocarbonyl,
whereby alkyl, cycloalkyl, heterocyclyl, aryl, heteroaryl, alkoxycarbonyl, cycloalkylcarbonyl, heterocyclylcarbonyl, arylcarbonyl, heteroarylcarbonyl and alkylaminocarbonyl may be substituted with 0, 1, 2 or 3 substituents selected independently of one another from the group consisting of halogen, hydroxy, amino, alkylamino and phenyl, and
whereby alkylcarbonyl is substituted with an amino or alkylamino substituent,
and
whereby alkylcarbonyl may be substituted with a further 0, 1 or 2 substituents selected independently of one another from the group consisting of halogen, hydroxy, trimethylsilyl, alkoxy, alkylthio, benzyloxy, C₃-C₆-cycloalkyl, phenyl, naphthyl, 5- to 10-membered heteroaryl, alkylcarbonylamino, alkoxycarbonylamino, arylcarbonylamino, arylcarbonyloxy, benzyloxycarbonyl and benzyloxycarbonylamino,
whereby phenyl and heteroaryl in turn may be substituted with 0, 1, 2 or 3 substituents selected independently of one another from the group consisting of halogen, hydroxy, nitro, alkyl, alkoxy and phenyl,
or two substituents on the same carbon atom in the alkylcarbonyl together with the carbon atom to which they are bonded form a C₃-C₆-cycloalkyl ring or a 5- to 7-membered heterocyclyl ring,
whereby the cycloalkyl ring and the heterocyclyl ring may be substituted with 0, 1, 2 or 3 substituents selected independently of one another from the group consisting of trifluoromethyl, alkyl and alkoxy,
or
whereby the cycloalkyl ring may be benzo-fused,
in which R⁵ is hydrogen, C₁-C₄-alkyl, cyclopropyl or cyclopropylmethyl, or
in which R⁴ and R⁵ together with the nitrogen atom to which they are bonded form a 5- to 7-membered heterocyclyl ring, whereby the heterocyclyl ring may be substituted with 0, 1, 2, or 3 substituents selected independently of one another from the group consisting of halogen, hydroxy, amino, cyano, alkyl, alkoxy and alkylamino,
by intramolecular cyclization of a compound of the following formula (II) 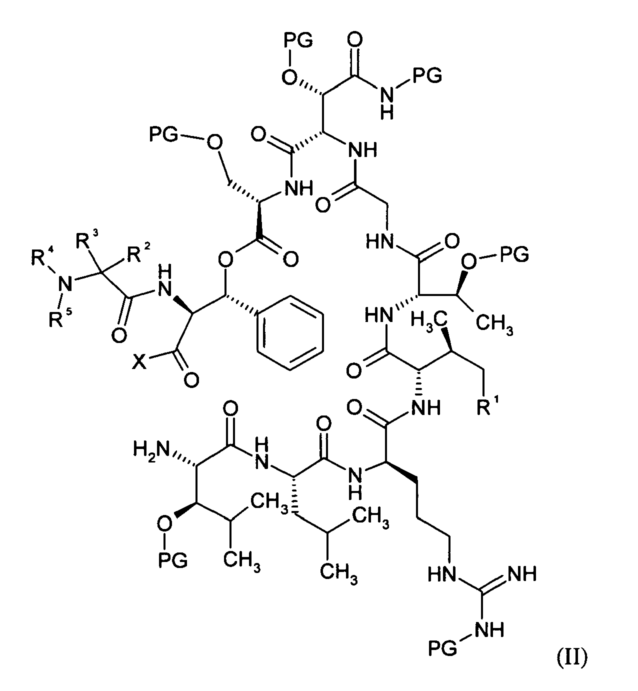
in which R¹ to R⁵ are as defined above,
in which X is OH, an active ester, a pseudohalogen or a halogen, and in which PG is H or a suitable protecting group,
and subsequent deprotection of the cyclic intermediate to form the cyclic depsipeptide of formula (I).

2. Method according to claim 1, **characterized in that** the compound of formula (II) is a compound of the following formula (IIa) 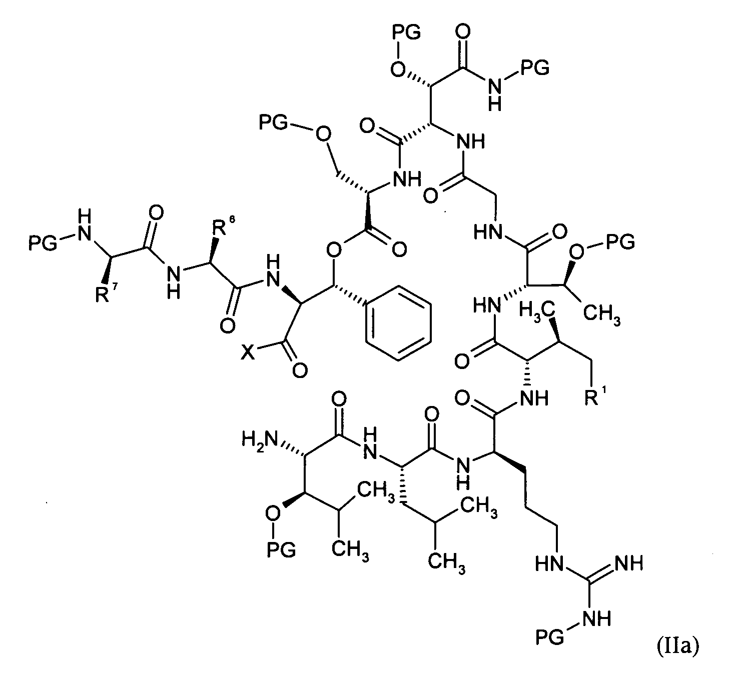
in which X and R¹ are as defined in claim 1,
in which R⁶ is isopropylmethyl, *tert*-butylmethyl, 2,2-dimethylbut-1-yl, 2-ethyl-2-methylbut-1-yl, 2,2-diethylbut-1-yl, 2,2-dimethylpent-1-yl, 3-pyridylmethyl, 4-trifluoromethyl-3-pyridylmethyl, benzyl or trimethylsilylmethyl, in which R⁷ is isopropylmethyl, *tert*-butylmethyl, 2,2-dimethylbut-1-yl, 2-ethyl-2-methylbut-1-yl, 2,2-diethylbut-1-yl, 2,2-dimethylpent-1-yl, trimethylsilylmethyl or benzyl, and
in which PG is H or a suitable protecting group.

3. Method according to claim 2, **characterized in that**
R⁶ is isopropylmethyl, *tert*-butylmethyl or 3-pyridylmethyl, and
R⁷ is isopropylmethyl, *tert*-butylmethyl or trimethylsilylmethyl.

4. Method according to claim 3, **characterized in that** R⁶ = R⁷ and is isopropylmethyl.

5. Method according to any one of claims 1 to 4, **characterized in that** X is OH.

6. Method according to any one of claims 1 to 5, **characterized in that** R¹ is CH₃.

7. Method according to claim 1, **characterized by** the preparation of the compound of formula (II) by coupling a compound of the following formula (III) with a compound of the following formula (IV) 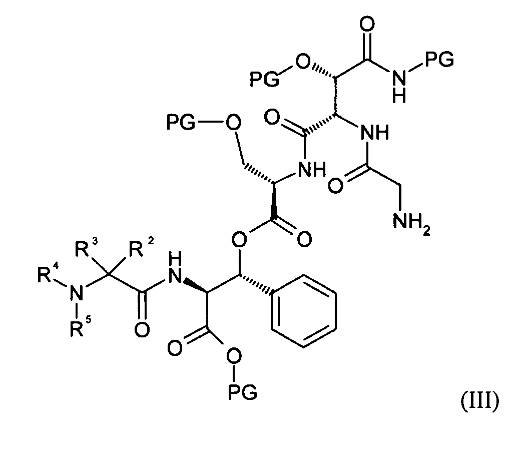 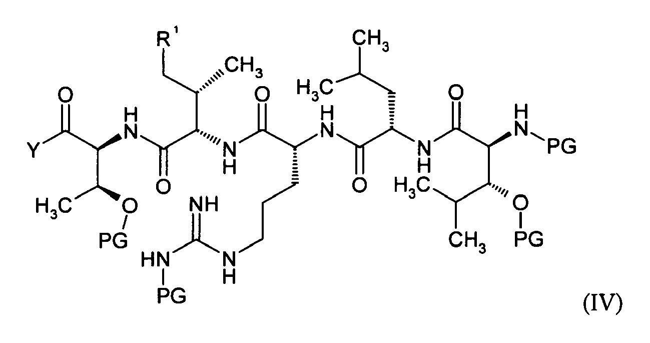
in which R¹ to R⁵ are as defined in claim 1,
in which Y is OH, an active ester, a pseudohalogen or a halogen, and
in which PG is H or a suitable protecting group,
and, where appropriate, partial or complete deprotection of the intermediate, as well as where appropriate conversion of the carboxy group of the 3-hydroxyphenylalanine into a group of formula -C(=O)X in which X is as defined in claim 1.

8. Method according to claim 7, **characterized in that** the compound of formula (III) is a compound of the following formula (IIIa) 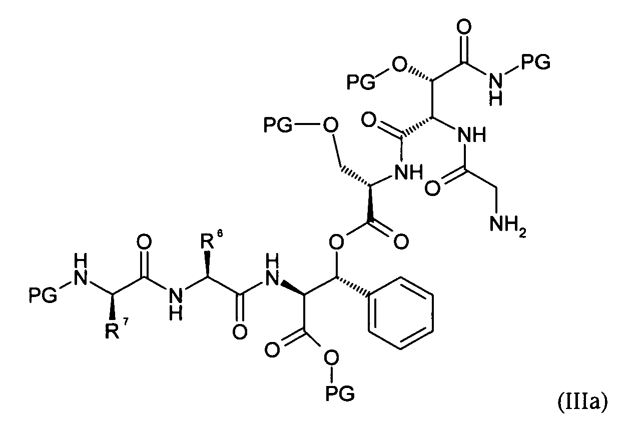
in which R⁶ and R⁷ are as defined in claims 2 to 4, and
in which PG is H or a suitable protecting group.

9. Method according to claim 7, **characterized by** the preparation of the compound of formula (III) by coupling a compound of the following formula (V) with a compound of the following formula (VI) 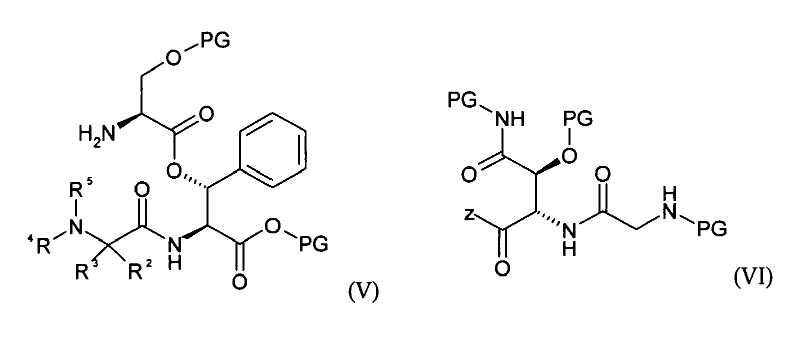
in which R² to R⁵ are as defined in claim 1,
in which Z is OH, an active ester, a pseudohalogen or a halogen, and
in which PG is H or a suitable protecting group,
and, where appropriate, partial or complete deprotection of the intermediate.

10. Method according to claim 9, **characterized in that** the compound of formula (V) is a compound of the following formula (Va) 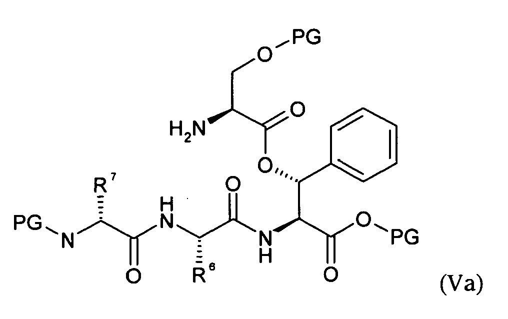
in which R⁶ and R⁷ are as defined in claims 2 to 4, and in which PG is H or a suitable protecting group.

11. Compound of the following formula (III) 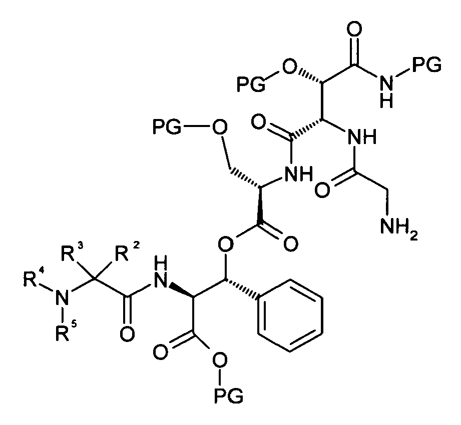
in which R² to R⁵ are as defined in claim 1, and
in which PG is H or a suitable protecting group.

12. Compound according to claim 11, having the following formula (IIIa) 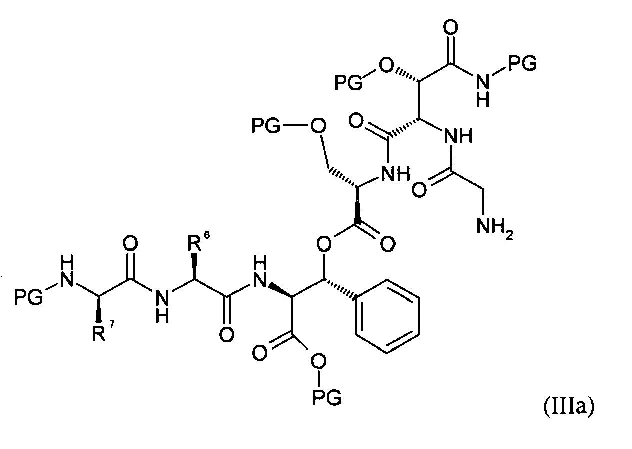
in which R⁶ and R⁷ are as defined in claims 2 to 4, and
in which PG is H or a suitable protecting group.

13. Compound according to claim 12, having the following formula (IIIb)

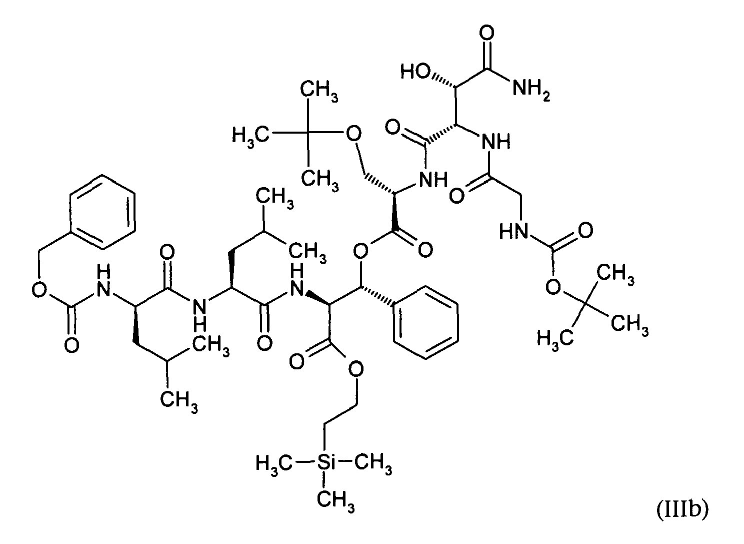

14. Method for preparing a compound according to claim 11, comprising the coupling of a compound of formula (V) with a compound of formula (VI) 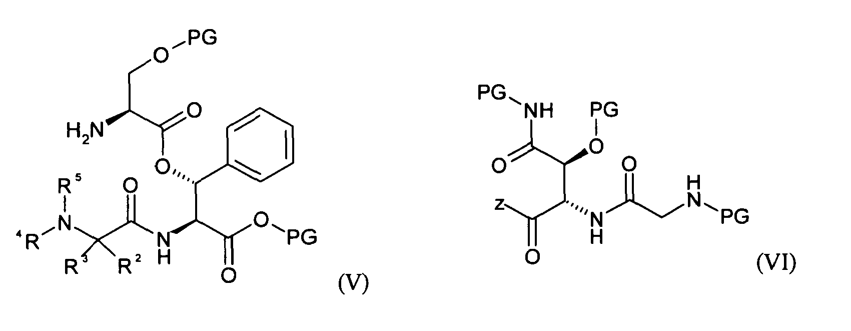
in which R² to R⁵ are as defined in claim 1,
in which Z is OH, an active ester, a pseudohalogen or a halogen, and
in which PG is H or a suitable protecting group,
and, where appropriate, partial or complete deprotection of the intermediate.

15. Compound of the following formula (VI) 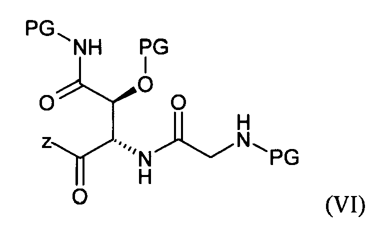
in which Z is OH, an active ester, a pseudohalogen or a halogen, and
in which PG is H or a suitable protecting group.

16. Compound according to claim 15, having the following formula (VIa)

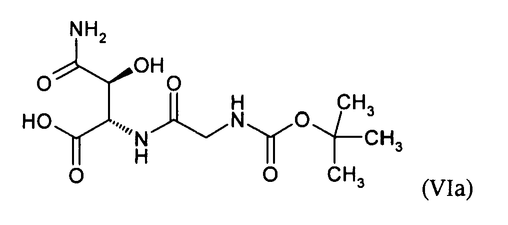

17. Method for preparing a compound according to claim 15, comprising the coupling of a compound of the following formula (VII) with a compound of the following formula (VIII) 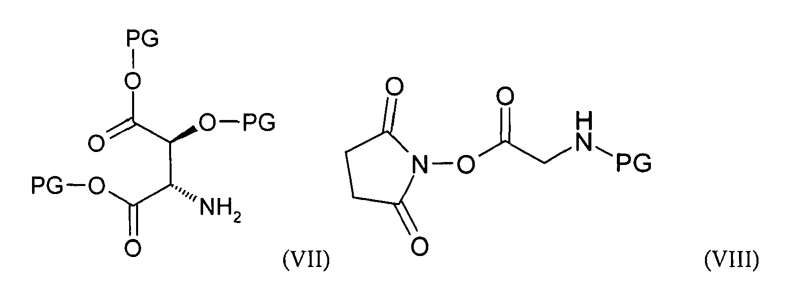
in which PG is H or a suitable protecting group,
and, where appropriate, complete or partial deprotection of the intermediate.

18. Method according to claim 17, **characterized in that** the compound of formula (VII) is a compound of the following formula (VIIa).

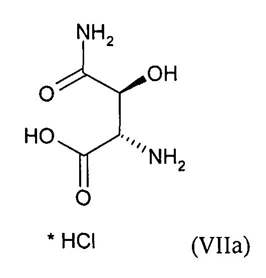

19. Method according to claim 17 or 18, **characterized in that** the compound of formula (VIII) is a compound of the following formula (VIIIa).

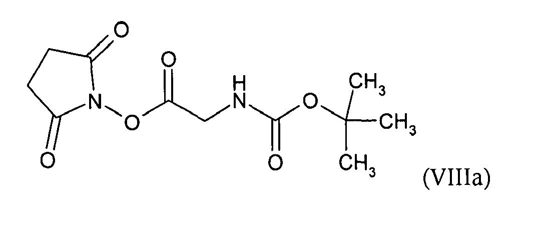

## Revendications

1. Procédé de préparation de depsipeptides cycliques de la formule suivante (1) 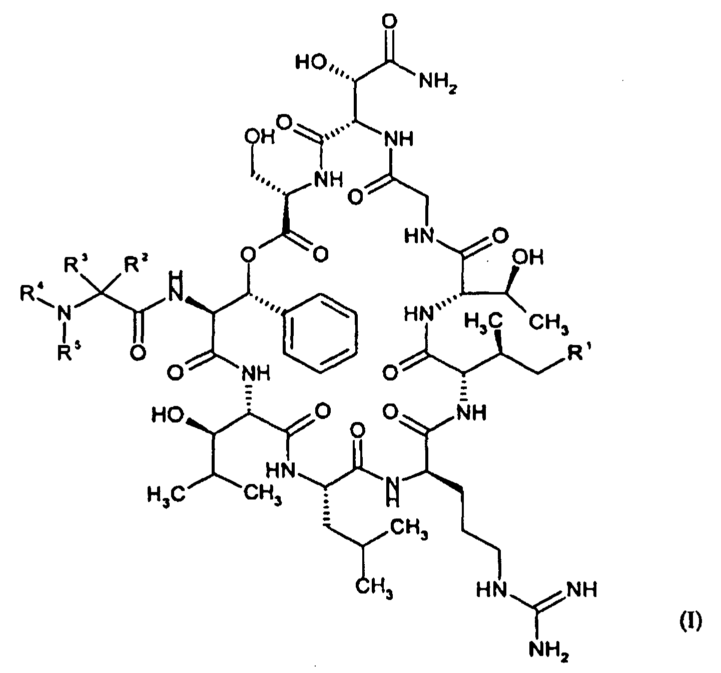
dans laquelle R¹ = H ou CH₃,
dans laquelle R² = un hydrogène, un cycloalkyle en C₃ à C₆, un cycloalcényle en C₃ à C₆, un cycloalkylméthyle en C₃ à C₆, un hétérocyclylméthyle à 5 à 7 atomes dans le cycle, un méthyle, un éthyle, un n-propyle, un isopropyle, un 1-méthylprop-1-yle, un 2-méthylprop-1-yle, un 2,2-diméthylprop-1-yle, un 1,1-diméthylprop-1-yle, un 1-éthyleprop-1-yle, un 1-éthyle-1-méthylprop-1-yle, un n-butyle, un 2-méthylbut-l-yle, u 3-méthylbut-1-yle, un 1-éthylbut-l-yle, un tert-butyle, un 4-méthylpent-1-yle, un n-hexyle, un alcényle ou un aryle,
sachant que R² peut être substitué par 0, 1, 2 ou 3 substituants, choisis indépendamment l'un de l'autre parmi le groupe consistant en un halogène, un hydroxyle, un amino, un cyano, un triméthylsilyle, un alkyle, un alcoxyle, un benzyloxyle, un cycloalkyle en C₃ à C₆, un aryle, un hétéroaryle à 5 à 10 atomes dans le cycle, un alkylamino, un arylamino, un alkylcarbonylamino, un arylcarbonylamino, un alkylcarbonyle, un alcoxycarbonyl, un arylcarbonyl et un benzyloxycarbonylamino,
où l'aryle et l'hétéroaryle peuvent être de leur côté substitués par 0, 1, 2 ou 3 substituants, choisis indépendamment l'un de l'autre parmi le groupe consistant en un halogène, un hydroxyle, un amino, un cyano, un nitro, un alkyle, un alcoxyle et un phényle,
dans laquelle R³ = un hydrogène ou un alkyle en C₁ à C₄,
ou
dans laquelle R² et R³ conjointement avec l'atome de carbone auquel ils sont liés, forment un cycle cycloalkyle en C₃ à C₆ ou un cycle hétérocyclyl à 5 à 7 atomes dans le cycle, sachant que le cycle cycloalkyle et le cycle hétérocyclyl peuvent être substitués par 0, 1, 2 ou 3 substituants, choisis indépendamment l'un de l'autre parmi le groupe consistant en le trifluorométhyle, l'alkyle, l'alcoxyle et l'alkylcarbonyle,
dans laquelle R⁴ = un alkyle, un cycloalkyle en C₃ à C₆, un hétérocyclyle à 5 à 7 atomes dans le cycle, un aryle, un hétéroaryle à 5 ou 6 atomes dans le cycle, un alkylcarbonyle, un alcoxycarbonyl, un cycloalkylcarbonyle en C₃ à C₆, un hétérocyclylcarbonyle à 5 à 7 atomes dans le cycle, un arylcarbonyle, un hétéroarylcarbonyle à 5 ou 6 atomes dans le cycle ou un alkylaminocarbonyle,
sachant que l'alkyle, le cycloalkyle, l'hétérocyclyle, l'aryle, l'hétéroaryle, l'alcoxycarbonyle, le cycloalkylcarbonyle, l'hétérocyclylcarbonyle, l'arylcarbonyle, l'hétéroarylcarbonyle et l'alkylaminocarbonyle peuvent être substitués par 0, 1, 2 ou 3 substituants, choisis indépendamment l'un de l'autre parmi le groupe consistant en un halogène, un hydroxyle, un amino, un alkylamino et un phényle,
et
sachant que l'alkylcarbonyle est substitué par un substituant amino ou alkylamino,
et
sachant que l'alkylcarbonyle peut être substitué par 0, 1 ou 2 substituants supplémentaires, choisis indépendamment l'un de l'autre parmi le groupe consistant en un halogène, un hydroxyle, un triméthylsilyle, un alcoxyle, un alkylthio, un benzyloxyle, un cycloalkyle en C₃ à C₆, un phényle, un naphthyle, un hétéroaryle 5 à 10 atomes dans le cycle, un alkylcarbonylamino, un alcoxycarbonylamino, un arylcarbonylamino, un arylcarbonyloxyle, un benzyloxycarbonyle et un benzyloxycarbonylamino,
sachant que le phényle et l'hétéroaryle peuvent être substitués de leur côté par 0, 1, 2 ou 3 substituants choisis indépendamment l'un de l'autre parmi le groupe consistant en un halogène, un hydroxyle, un nitro, un alkyle, un alcoxyle et un phényle,
ou deux substituants sur le même atome de carbone dans l'alkylcarbonyle, conjointement avec l'atome de carbone, auquel ils sont liés, forment un cycle cycloalkyle en C₃ à C₆ ou un cycle hétérocyclyle à 5 à 7 atomes dans le cycle,
sachant que le cycle cycloalkyle et le cycle hétérocyclyle peuvent être substitués par 0, 1, 2 ou 3 substituants, choisis indépendamment l'un de l'autre parmi le groupe consistant en un trifluorométhyle, un alkyle et un alcoxyle,
ou
sachant que le cycle cycloalkyle peut être fermé en benzène,
dans laquelle R⁵ = un hydrogène, un alkyle en C₁ à C₄, un cyclopropyle ou un cyclopropylméthyle,
ou
dans laquelle R⁴ et R⁵ conjointement avec l'atome d'azote auquel ils sont liés, forment un cycle hétérocyclyle à 5 à 7 atomes dans le cycle, sachant que le cycle hétérocyclyle peut être substitué par 0, 1, 2 ou 3 substituants choisis indépendamment l'un de l'autre parmi le groupe consistant en un halogène, un hydroxyle, un amino, un cyano, un alkyle, un alcoxyle et un alkylamino,
par cyclisation intramoléculaire d'un composé de la formule suivante (II) 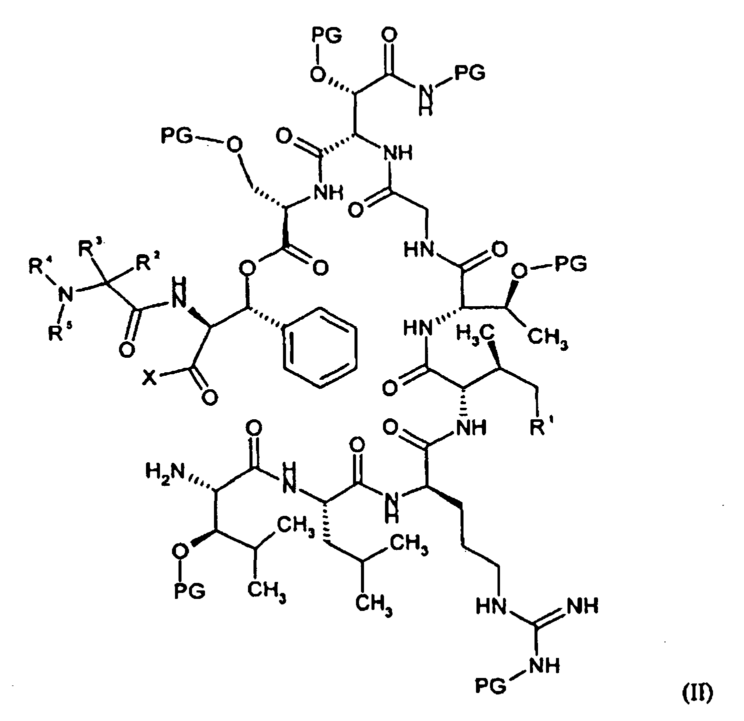
dans laquelle R¹ à R⁵ sont définis comme précédemment,
dans laquelle X = OH, un ester actif, un pseudohalogène ou un halogène, et
dans laquelle PG = H ou un groupe de protection approprié,
suivie d'une déprotection du produit intermédiaire cyclique moyennant la formation du depsipeptide cyclique de la formule (I).

2. Procédé selon la revendication 1, **caractérisé en ce que** le composé de la formule (II) est un composé de la formule suivante (lia) 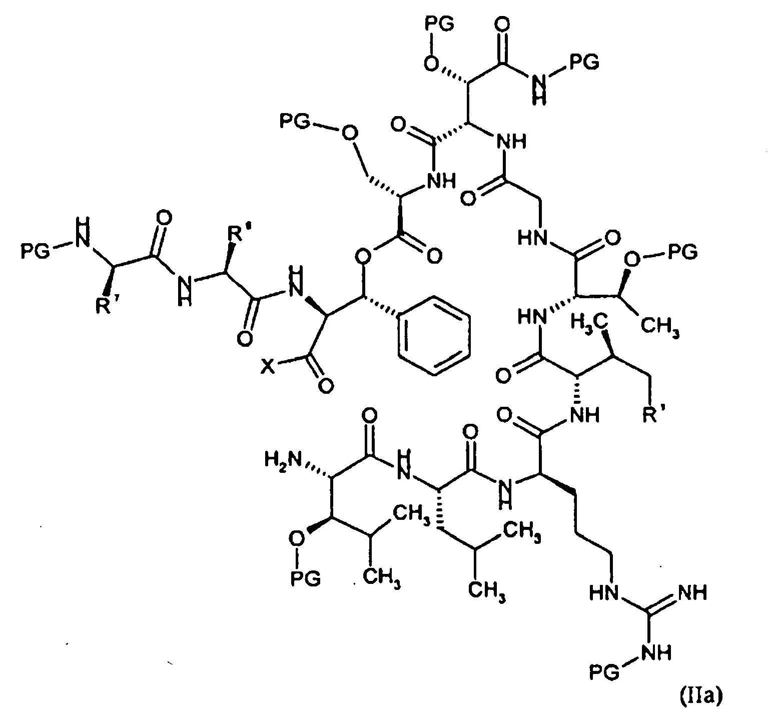
dans laquelle X et R¹ sont définis comme dans la revendication 1,
dans laquelle R⁶ = un isopropylméthyle, un tert-butylméthyl, un 2,2-diméthylbut-1-yle, un 2-éthyle-2-méthylbut-1-yle, un 2,2-diéthylbut-1-yle, un 2,2-diméthylpent-1-yle, un 3-pyridylméthyle, un 4-trifluorométhyle-3-pyridylméthyle, un benzyle ou un triméthylsilylméthyle,
dans laquelle R⁷= un isopropylméthyle, un tert-butylméthyle, un 2,2-diméthylbut-1-yle, un 2-éthyle-2-méthylbut-1-yle, un 2,2-diéthylbut-1-yle, un 2,2-diméthylpent-1-yle, un triméthylsilylméthyle ou un benzyle, et
dans laquelle PG = H ou un groupe de protection approprié.

3. Procédé selon la revendication 2, **caractérisé en ce que**
R⁶ = un isopropylméthyle, un tert-butylméthyle ou un 3-pyridylméthyle, et
R⁷ = un isopropylméthyle, un tert-butylméthyle ou un triméthylsilylméthyle.

4. Procédé selon la revendication 3, **caractérisé en ce que** R⁶ = R⁷ = un isopropylméthyle.

5. Procédé selon l'une quelconque des revendications 1 à 4, **caractérisé en ce que** X = OH.

6. Procédé selon l'une quelconque des revendications 1 bis 5, **caractérisé en ce que** R¹ = CH₃.

7. Procédé selon la revendication 1, **caractérisé par** la préparation du composé de la formule (II) par accouplement d'un composé de la formule suivante (III) avec un composé de la formule suivante (IV) 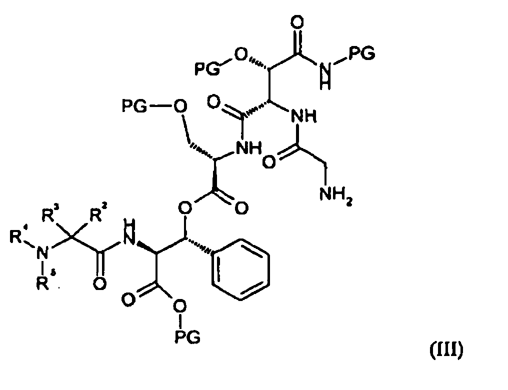 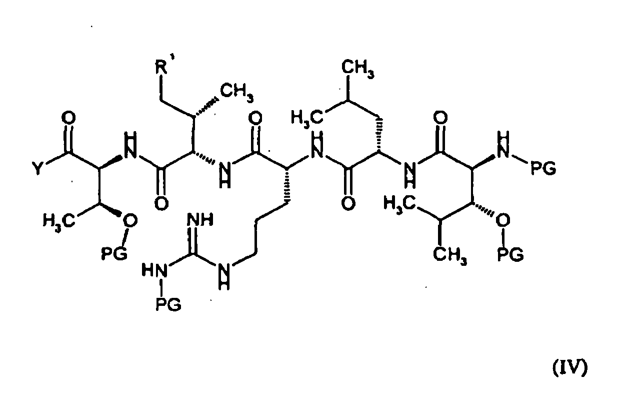
dans lesquelles R¹ à R⁵ sont définis comme dans la revendication 1,
dans lesquelles Y = OH, un ester actif, un pseudohalogène ou un halogène, et
dans lesquelles PG = H ou un groupe de protection approprié,
et le cas échéant par une déprotection partielle ou totale du produit intermédiaire, ainsi que le cas échéant par conversion du groupe carboxyle de la 3-hydroxy-phénylalanine en un groupe de la formule C(=O)X, dans laquelle X est défini comme dans la revendication 1.

8. Procédé selon la revendication 7, **caractérisé en ce que** le composé de la formule (III) est un composé de la formule suivante (IIIa) 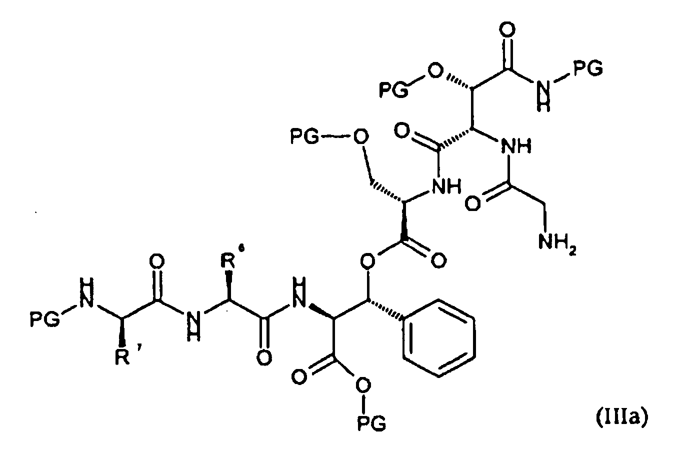 dans laquelle R⁶ et R⁷ sont définis comme dans les revendications 2 à 4, et dans laquelle PG = H ou un groupe de protection approprié.

9. Procédé selon la revendication 7, **caractérisé par** la préparation du composé de la formule (III) par l'accouplement d'un composé de la formule suivante (V) avec un composé de la formule suivante (VI) 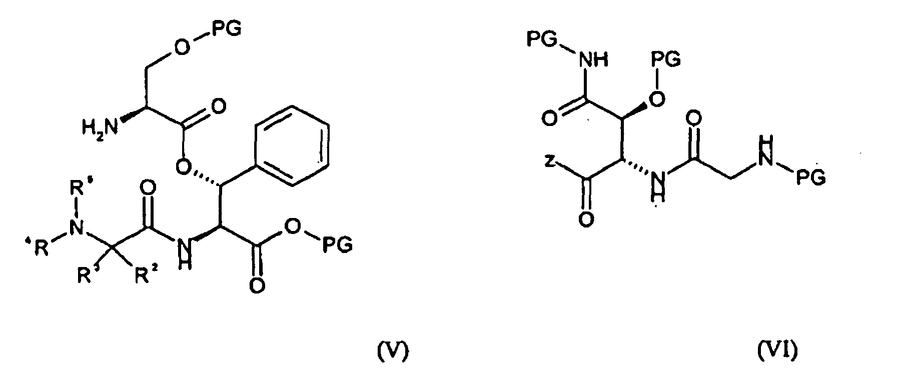
dans lesquelles R² à R⁵ sont définis comme dans la revendication 1,
dans lesquelles Z = OH, un ester actif, un pseudohalogène ou un halogène, et
dans lesquelles PG = H ou un groupe de protection approprié,
et, le cas échéant, par la déprotection partielle ou totale du produit intermédiaire.

10. Procédé selon la revendication 9, **caractérisé en ce que** le composé de la formule (V) est un composé de la formule suivante (Va) 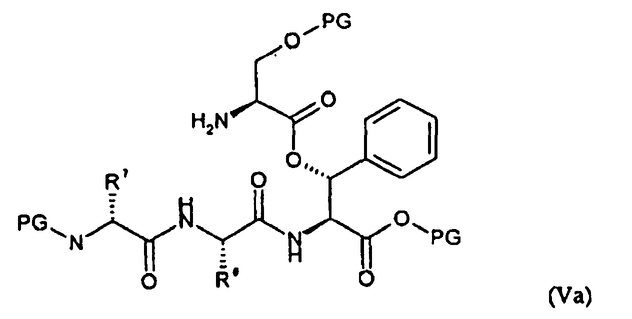
dans laquelle R⁶ et R⁷ sont définis comme dans les revendications 2 à 4, et
dans laquelle PG = H ou un groupe de protection approprié.

11. Composé de la formule suivante (III) 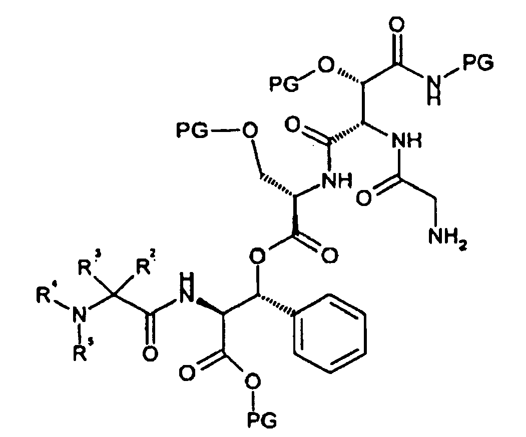
dans laquelle R² à R⁵ sont définis comme dans la revendication 1, et
dans laquelle PG = H ou un groupe de protection approprié.

12. Composé selon la revendication 11, avec la formule suivante (IIIa) 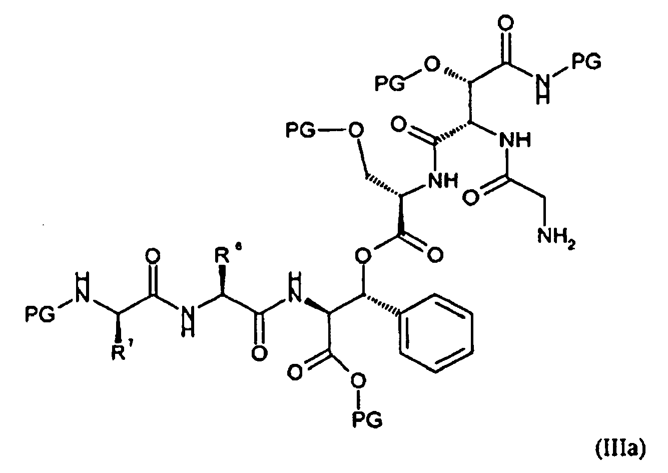
dans laquelle R⁶ et R⁷ sont définis comme dans les revendications 2 à 4, et
dans laquelle PG = H ou un groupe de protection approprié.

13. Composé selon la revendication 12, avec la formule suivante (IIIb)

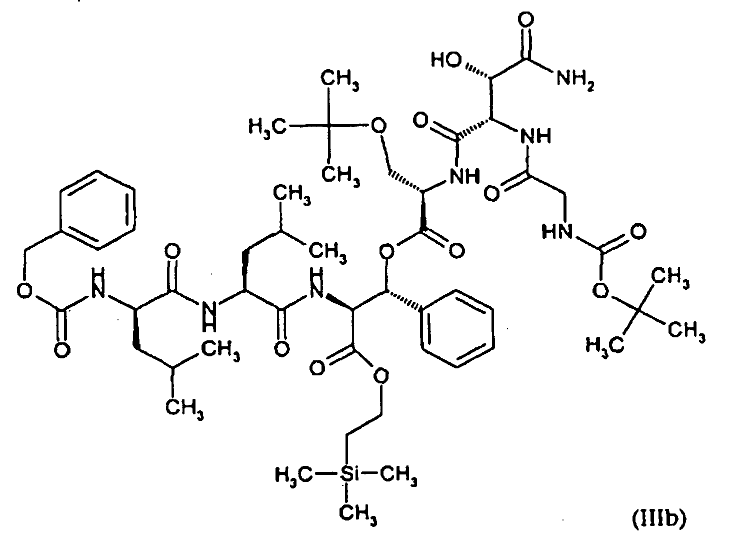

14. Procédé pour la préparation d'un composé selon la revendication 11, qui comporte l'accouplement d'un composé d la formule (V) avec un composé de la formule (VI) 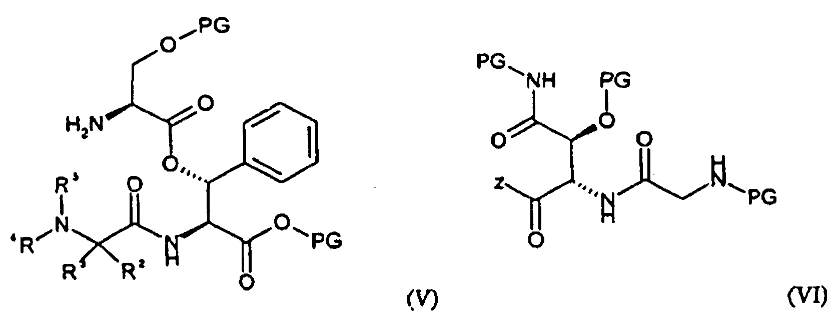
dans lesquelles R² à R⁵ sont définis comme dans la revendication 1,
dans lesquelles Z = OH, un ester actif, un pseudohalogène ou un halogène, et
dans lesquelles PG = H ou un groupe de protection approprié,
et le cas échéant la déprotection partielle ou totale du produit intermédiaire.

15. Composé de la formule suivante (VI) 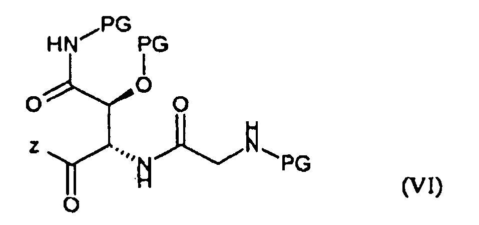
dans laquelle Z = OH, un ester actif, un pseudohalogène ou un halogène, et
dans laquelle PG = H ou un groupe de protection approprié.

16. Composé selon la revendication 15, avec la formule suivante (VIa)

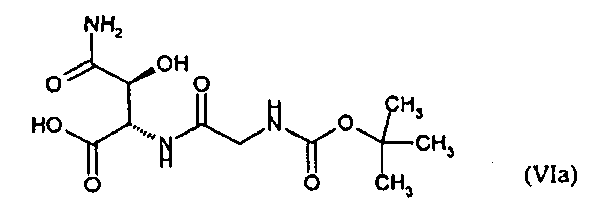

17. Procédé pour la préparation d'un composé selon la revendication 15, qui comporte l'accouplement d'un composé de la formule suivante (VII) avec un composé de la formule suivante (VIII) 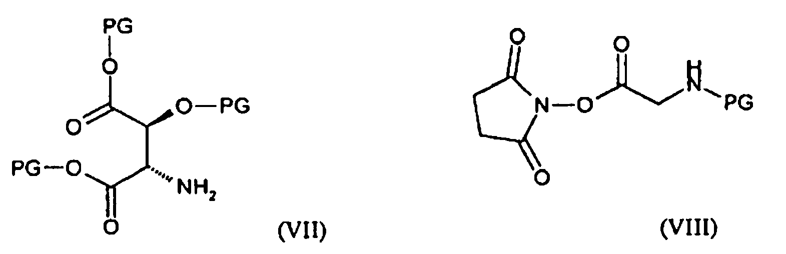
dans lesquelles PG = H ou un groupe de protection approprié
et, le cas échéant, la déprotection totale ou partielle du produit intermédiaire.

18. Procédé selon la revendication 17, **caractérisé en ce que** le composé de la formule (VII) est un composé de la formule suivante (VIIa).

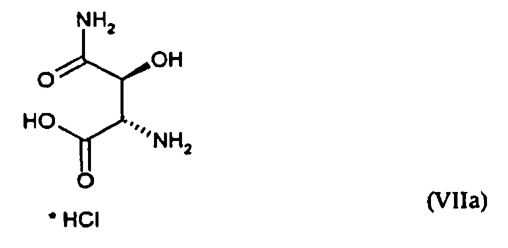

19. Procédé selon la revendication 17 ou 18, **caractérisé en ce que** le composé de la formule (VIII) est un composé de la formule suivante (VIIIa).
